(19) Europäisches Patentamt
European Patent Office
Office européen des brevets

(11) EP 3 143 135 B1

(12) **EUROPEAN PATENT SPECIFICATION**

(45) Date of publication and mention of the grant of the patent:
**10.04.2019 Bulletin 2019/15**

(21) Application number: **15724214.0**

(22) Date of filing: **15.05.2015**

(51) Int Cl.:
***C12N 9/16*** *(2006.01)* ***C11B 3/00*** *(2006.01)*
***C12P 7/64*** *(2006.01)*

(86) International application number:
**PCT/EP2015/060819**

(87) International publication number:
**WO 2015/173426 (19.11.2015 Gazette 2015/46)**

(54) **COMPOSITIONS COMPRISING POLYPEPTIDES HAVING PHOSPHOLIPASE C ACTIVITY AND USE THEREOF**

ZUSAMMENSETZUNGEN MIT POLYPEPTIDEN MIT PHOSPHOLIPASE C-AKTIVITÄT UND VERWENDUNG DAVON

COMPOSITIONS COMPRENANT DES POLYPEPTIDES AYANT UNE ACTIVITÉ DE PHOSPHOLIPASE C ET LEUR UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.05.2014 EP 14168437**

(43) Date of publication of application:
**22.03.2017 Bulletin 2017/12**

(73) Proprietor: **Novozymes A/S**
**2880 Bagsværd (DK)**

(72) Inventors:
- **DAMSTRUP, Marianne Linde**
  **DK-2880 Bagsvaerd (DK)**
- **BRASK, Jesper**
  **DK-2880 Bagsvaerd (DK)**
- **LILBAEK, Hanna Maria**
  **DK-2880 Bagsvaerd (DK)**

(74) Representative: **NZ EPO Representatives**
**Krogshoejvej 36**
**2880 Bagsvaerd (DK)**

(56) References cited:
**WO-A1-2008/094847**
**WO-A1-2012/062817**
**US-A1- 2012 210 467**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 143 135 B1

SEQUENCE LISTING

<110> Novozymes A/S

<120> COMPOSITIONS COMPRISING POLYPEPTIDES HAVING PHOSPHOLIPASE C ACTIVITY AND USE THEREOF

<130> 12870-WO-PCT

<160> 47

<170> PatentIn version 3.5

<210> 1
<211> 1932
<212> DNA
<213> Kinochaeta sp

<220>
<221> sig_peptide
<222> (1)..(54)

<400> 1

```
atgcgtgcct cctcgattct ttcgctggct ctgggcctct cggttgccca ggccgctgtg      60
aaccccgccg atgtcctgtc tgttgtggag aagcgagtcg acccggctag cggcctagag     120
gtgcgcagca tttgggacac catctggaac gacattaaat cggcggccga ctgtactgcc     180
tgcgaggccg tcttgactct gctcaagggc gtcgcggcct ttggcgataa tttttttcgta     240
gaggttttga ccgagatctg tgacctttcc ggggctgagg atgatgatgt gtgctccggt     300
gttcttagcc tcgagggccc aatcatagcc aacgatatcc gtaagatgag cattggctcc     360
aagacatcag agctcttctg catcaccttc ctgggactgt gctcgtaccc ggcggtggac     420
gctttcaccg tccccttccc gaccgcgaag tcagccgcca cccggcccgt gtcgtcgggc     480
aaagacccca tctacgtcgt gcactactct gacatccaca tcgatccctt ctatgtggca     540
ggatccgcca gcaactgcac caagcccatc tgctgccgag attacacttc ggcgtcgtcc     600
ccgggcaaca acaactcccc tgccggcccg tacggcgacc acaactgcga cgtcccgatt     660
agcctggagg acagcatgta tgctgccatc aagaagctgg tgcctgatgc cgccttcggc     720
atctttactg gcgatattgt cgaccacgcc gtctggaata cctcggagag tcagaacatc     780
atcgacatga atgacgccta cacgcgcatg aagaactcgg gcatgctgcc gaccatcttc     840
gccacggcgg gcaaccatga agcgtcgccc gtcaactcgt tcccgccgcc ggccatcggc     900
aacgagtcgc agtgggttta cgacacactg gccagcgact ggagccagtg gatcggcacg     960
tcgggcgcga gctcggtcga gtccatcggc gcttacagcg tgcagtacgg cagcaccaag    1020
ctgcgcgtca tctcgctcaa caccaacatg tactacatcg agaacttcta cctctatgag    1080
cccaccatgg agcaagatcc agccgggcag ttcgcctggc tcgtgtccga gctgagcgcc    1140
gccgaagccg ccggcgagcg cgtgtggatc atcggccaca tgccgctggg tctctcggac    1200
```

```
gccttccacg acccgagcaa ctactttgac cagatcgtca accgctacga ggccaccatc     1260

gccgccatgt tcttcggcca cacccacgag gaccatttcc agatctcgta ctcggactac     1320

aacgcccgca cggccgccaa cgcccgcgcc gtctcctaca tcatgccgtc gctgacgccg     1380

acctcgggcc acccgacctt ccgcgtctac acggtcgacc ccgagacctt cggcgtgctg     1440

gacgcgacga cctactacgc cgacatgtcg cagccgacct accagaccgc ggggccggcc     1500

tggtccgtct actacagcgc caaggccgcc tacggcgggc tcgtcgaccc gcccgtcgcc     1560

gccgacgacg ccgccgccga gctgacgccc gccttctggc acaacgtgac ggccgcgctg     1620

gccgccgacc cggccagctt cgacgcctac tacgcgcgca agacgcgcgg ctgggacgtg     1680

gccgcctgcg ccggcgcctg cgcggccgcc gaggtctgcg ccctgcgcgc cgcccgcgcc     1740

caggacaact gcgtcgtgcc cacgcccggc gtgcacttca gcaagcgcgc cgacgagggc     1800

accctggccc accaccgcga cgagtgcggc gtcagcgtcg cccgcaacag cctctccagc     1860

ctcgtcgtgc agcgcgaggc gctggagcac ctcgagggcc gcctgagcga gaagcggagg     1920

atggccgtgt ga                                                         1932
```

<210> 2
<211> 643
<212> PRT
<213> Kinochaeta sp

<400> 2

```
Met Arg Ala Ser Ser Ile Leu Ser Leu Ala Leu Gly Leu Ser Val Ala
1               5                   10                  15

Gln Ala Ala Val Asn Pro Ala Asp Val Leu Ser Val Val Glu Lys Arg
            20                  25                  30

Val Asp Pro Ala Ser Gly Leu Glu Val Arg Ser Ile Trp Asp Thr Ile
        35                  40                  45

Trp Asn Asp Ile Lys Ser Ala Ala Asp Cys Thr Ala Cys Glu Ala Val
    50                  55                  60

Leu Thr Leu Leu Lys Gly Val Ala Ala Phe Gly Asp Asn Phe Phe Val
65                  70                  75                  80

Glu Val Leu Thr Glu Ile Cys Asp Leu Ser Gly Ala Glu Asp Asp Asp
                85                  90                  95

Val Cys Ser Gly Val Leu Ser Leu Glu Gly Pro Ile Ile Ala Asn Asp
            100                 105                 110
```

```
Ile Arg Lys Met Ser Ile Gly Ser Lys Thr Ser Glu Leu Phe Cys Ile
        115                 120                 125

Thr Phe Leu Gly Leu Cys Ser Tyr Pro Ala Val Asp Ala Phe Thr Val
    130                 135                 140

Pro Phe Pro Thr Ala Lys Ser Ala Ala Thr Arg Pro Val Ser Ser Gly
145                 150                 155                 160

Lys Asp Pro Ile Tyr Val Val His Tyr Ser Asp Ile His Ile Asp Pro
                165                 170                 175

Phe Tyr Val Ala Gly Ser Ala Ser Asn Cys Thr Lys Pro Ile Cys Cys
            180                 185                 190

Arg Asp Tyr Thr Ser Ala Ser Ser Pro Gly Asn Asn Asn Ser Pro Ala
        195                 200                 205

Gly Pro Tyr Gly Asp His Asn Cys Asp Val Pro Ile Ser Leu Glu Asp
    210                 215                 220

Ser Met Tyr Ala Ala Ile Lys Lys Leu Val Pro Asp Ala Ala Phe Gly
225                 230                 235                 240

Ile Phe Thr Gly Asp Ile Val Asp His Ala Val Trp Asn Thr Ser Glu
                245                 250                 255

Ser Gln Asn Ile Ile Asp Met Asn Asp Ala Tyr Thr Arg Met Lys Asn
            260                 265                 270

Ser Gly Met Leu Pro Thr Ile Phe Ala Thr Ala Gly Asn His Glu Ala
        275                 280                 285

Ser Pro Val Asn Ser Phe Pro Pro Pro Ala Ile Gly Asn Glu Ser Gln
    290                 295                 300

Trp Val Tyr Asp Thr Leu Ala Ser Asp Trp Ser Gln Trp Ile Gly Thr
305                 310                 315                 320

Ser Gly Ala Ser Ser Val Glu Ser Ile Gly Ala Tyr Ser Val Gln Tyr
                325                 330                 335

Gly Ser Thr Lys Leu Arg Val Ile Ser Leu Asn Thr Asn Met Tyr Tyr
            340                 345                 350

Ile Glu Asn Phe Tyr Leu Tyr Glu Pro Thr Met Glu Gln Asp Pro Ala
        355                 360                 365
```

```
Gly Gln Phe Ala Trp Leu Val Ser Glu Leu Ser Ala Ala Glu Ala Ala
    370                 375                 380

Gly Glu Arg Val Trp Ile Ile Gly His Met Pro Leu Gly Leu Ser Asp
385                 390                 395                 400

Ala Phe His Asp Pro Ser Asn Tyr Phe Asp Gln Ile Val Asn Arg Tyr
                405                 410                 415

Glu Ala Thr Ile Ala Ala Met Phe Phe Gly His Thr His Glu Asp His
            420                 425                 430

Phe Gln Ile Ser Tyr Ser Asp Tyr Asn Ala Arg Thr Ala Ala Asn Ala
        435                 440                 445

Arg Ala Val Ser Tyr Ile Met Pro Ser Leu Thr Pro Thr Ser Gly His
    450                 455                 460

Pro Thr Phe Arg Val Tyr Thr Val Asp Pro Glu Thr Phe Gly Val Leu
465                 470                 475                 480

Asp Ala Thr Thr Tyr Tyr Ala Asp Met Ser Gln Pro Thr Tyr Gln Thr
                485                 490                 495

Ala Gly Pro Ala Trp Ser Val Tyr Tyr Ser Ala Lys Ala Ala Tyr Gly
            500                 505                 510

Gly Leu Val Asp Pro Pro Val Ala Ala Asp Asp Ala Ala Ala Glu Leu
        515                 520                 525

Thr Pro Ala Phe Trp His Asn Val Thr Ala Ala Leu Ala Ala Asp Pro
    530                 535                 540

Ala Ser Phe Asp Ala Tyr Tyr Ala Arg Lys Thr Arg Gly Trp Asp Val
545                 550                 555                 560

Ala Ala Cys Ala Gly Ala Cys Ala Ala Ala Glu Val Cys Ala Leu Arg
                565                 570                 575

Ala Ala Arg Ala Gln Asp Asn Cys Val Val Pro Thr Pro Gly Val His
            580                 585                 590

Phe Ser Lys Arg Ala Asp Glu Gly Thr Leu Ala His His Arg Asp Glu
        595                 600                 605

Cys Gly Val Ser Val Ala Arg Asn Ser Leu Ser Ser Leu Val Val Gln
    610                 615                 620
```

```
Arg Glu Ala Leu Glu His Leu Glu Gly Arg Leu Ser Glu Lys Arg Arg
625                 630                 635                 640

Met Ala Val
```

<210> 3
<211> 1833
<212> DNA
<213> Penicillium emersonii

<220>
<221> sig_peptide
<222> (1)..(48)

<400> 3

```
atgagagttc tcgccctcat cgctgctctg gccacggtgg ccaccgcaag tgccccctat      60
gacaagcgcg acttggccca ggagatttgg gacgacatca agaatgcggt ggattgcgct     120
ggctgccagg tcgttctgac tgccctgaag ggtgtggccg atctgggcac gactgcccctt    180
gtcgatgtgc tgaccgaagt gtgcaacatc agtggcaaag aagattccga tgtctgctcg     240
ggcatcatct cccgcgaggg tccggtgctg gattatgtcc tgcagcacct cgatatcggc     300
tcgcacacct cccaggtcat ctgtgccagc gcattcggcc tctgccagta tcctgaggtc     360
cggccctaca acctcacctt ccctaaaccc aagcccaaca cgactcgtcc agaacccagt     420
ggagagtcac caatccaggt cgtccacttc agcgatactc acgtggacct ctcctacgag     480
acggggtcca attacaactg tacaaagccc atctgctgtc gcccttacac ggccgaggat     540
gcaccgggaa acacgacgac tccgtgcggg ccatatggca acaccaaatg tgatgctccc     600
ttgagcctcg aggagagcat gttcgccgcg atcaaagcgc tcaaccccca gcccgccttt     660
tccatttata cgggcgacgt cgtcgcacac gacatctggc tggtggatca aaacgaggtc     720
attgaggacc tgaatgccac ctacgaccgc atggccgggc tggggctggt ctatgcggcc     780
attgggaatc acgacacggc gccggtcaac gatctgccga cgagcaacat ccccagcgag     840
tacagcgcga actggaccta cgaggccctc tcgtacgact ttacgatgct gacgcagtcg     900
gcctctgccc agaccgcggc gaattacggg tcttattcgg ccatctatcc cggcagctac     960
ggcacggatc tccgcgtcat ttcctacaac agcatcttct actacgtgga caatttctgg    1020
gcgtaccaag atcctatgga attcgacccg gatggacaac tggcctggct gatcaacgag    1080
ctccaggagg ccgagacggc ggggcagcgg gtctggatta ttgcgcatgt gccgacgggc    1140
acgtcggatc acttccacga ctattcgcac tactttgacc agatcgtgca gcgctacgag    1200
gccactattg cggcgctgtt ctacggccac actcacatcg accagttcca aatctcgtac    1260
```

```
tcgaactatt ccaaccgagc attcgacacg gcgaccgcca tcgggtatat catgccgtca      1320

ttgactccga cctcgggacc tcctaccttc cgggtctatg acgttgatcc caagacgttt      1380

gccgtgctgg acttcaccaa ctacattgcc aacatcagcg acccggcgtt ccagtcgggc      1440

ccgtcgtggc agaagtacta ctcggccaag gagacgtacg gctcgttgct gtctcctcca      1500

gtgacggacc cgacggcgga gctgacgccg gccttctggc acaacgtcac ggtggccttt      1560

gagcaggaca acgcgacctt ccaggagtac tgggcgcggc agacgcgggg gtacgacgtg      1620

tcgagctgca cggggtcctg catcactcag gccatctgcg gcctgcgcgc gggagacgcg      1680

cagtacaact gcgtgacgcc gacgccgggc ttcaactttg ccaaacggga tacctccaac      1740

cccaagcagg ctctatctca tgtcgagaaa tgcgagggct cgggattgct ggggctgctg      1800

cgcaggatgg tggctgacag taagtcttcc tag                                   1833
```

<210> 4
<211> 610
<212> PRT
<213> Penicillium emersonii

<400> 4

```
Met Arg Val Leu Ala Leu Ile Ala Ala Leu Ala Thr Val Ala Thr Ala
1               5                   10                  15

Ser Ala Pro Tyr Asp Lys Arg Asp Leu Ala Gln Glu Ile Trp Asp Asp
            20                  25                  30

Ile Lys Asn Ala Val Asp Cys Ala Gly Cys Gln Val Val Leu Thr Ala
        35                  40                  45

Leu Lys Gly Val Ala Asp Leu Gly Thr Thr Ala Leu Val Asp Val Leu
    50                  55                  60

Thr Glu Val Cys Asn Ile Ser Gly Lys Glu Asp Ser Asp Val Cys Ser
65                  70                  75                  80

Gly Ile Ile Ser Arg Glu Gly Pro Val Leu Asp Tyr Val Leu Gln His
                85                  90                  95

Leu Asp Ile Gly Ser His Thr Ser Gln Val Ile Cys Ala Ser Ala Phe
            100                 105                 110

Gly Leu Cys Gln Tyr Pro Glu Val Arg Pro Tyr Asn Leu Thr Phe Pro
        115                 120                 125

Lys Pro Lys Pro Asn Thr Thr Arg Pro Glu Pro Ser Gly Glu Ser Pro
    130                 135                 140
```

Ile Gln Val Val His Phe Ser Asp Thr His Val Asp Leu Ser Tyr Glu
145 150 155 160

Thr Gly Ser Asn Tyr Asn Cys Thr Lys Pro Ile Cys Cys Arg Pro Tyr
165 170 175

Thr Ala Glu Asp Ala Pro Gly Asn Thr Thr Thr Pro Cys Gly Pro Tyr
180 185 190

Gly Asn Thr Lys Cys Asp Ala Pro Leu Ser Leu Glu Glu Ser Met Phe
195 200 205

Ala Ala Ile Lys Ala Leu Asn Pro Gln Pro Ala Phe Ser Ile Tyr Thr
210 215 220

Gly Asp Val Val Ala His Asp Ile Trp Leu Val Asp Gln Asn Glu Val
225 230 235 240

Ile Glu Asp Leu Asn Ala Thr Tyr Asp Arg Met Ala Gly Leu Gly Leu
245 250 255

Val Tyr Ala Ala Ile Gly Asn His Asp Thr Ala Pro Val Asn Asp Leu
260 265 270

Pro Thr Ser Asn Ile Pro Ser Glu Tyr Ser Ala Asn Trp Thr Tyr Glu
275 280 285

Ala Leu Ser Tyr Asp Phe Thr Met Leu Thr Gln Ser Ala Ser Ala Gln
290 295 300

Thr Ala Ala Asn Tyr Gly Ser Tyr Ser Ala Ile Tyr Pro Gly Ser Tyr
305 310 315 320

Gly Thr Asp Leu Arg Val Ile Ser Tyr Asn Ser Ile Phe Tyr Tyr Val
325 330 335

Asp Asn Phe Trp Ala Tyr Gln Asp Pro Met Glu Phe Asp Pro Asp Gly
340 345 350

Gln Leu Ala Trp Leu Ile Asn Glu Leu Gln Glu Ala Glu Thr Ala Gly
355 360 365

Gln Arg Val Trp Ile Ile Ala His Val Pro Thr Gly Thr Ser Asp His
370 375 380

Phe His Asp Tyr Ser His Tyr Phe Asp Gln Ile Val Gln Arg Tyr Glu
385 390 395 400

```
Ala Thr Ile Ala Ala Leu Phe Tyr Gly His Thr His Ile Asp Gln Phe
                405                 410                 415

Gln Ile Ser Tyr Ser Asn Tyr Ser Asn Arg Ala Phe Asp Thr Ala Thr
            420                 425                 430

Ala Ile Gly Tyr Ile Met Pro Ser Leu Thr Pro Thr Ser Gly Pro Pro
        435                 440                 445

Thr Phe Arg Val Tyr Asp Val Asp Pro Lys Thr Phe Ala Val Leu Asp
    450                 455                 460

Phe Thr Asn Tyr Ile Ala Asn Ile Ser Asp Pro Ala Phe Gln Ser Gly
465                 470                 475                 480

Pro Ser Trp Gln Lys Tyr Tyr Ser Ala Lys Glu Thr Tyr Gly Ser Leu
                485                 490                 495

Leu Ser Pro Pro Val Thr Asp Pro Thr Ala Glu Leu Thr Pro Ala Phe
            500                 505                 510

Trp His Asn Val Thr Val Ala Phe Glu Gln Asp Asn Ala Thr Phe Gln
        515                 520                 525

Glu Tyr Trp Ala Arg Gln Thr Arg Gly Tyr Asp Val Ser Ser Cys Thr
    530                 535                 540

Gly Ser Cys Ile Thr Gln Ala Ile Cys Gly Leu Arg Ala Gly Asp Ala
545                 550                 555                 560

Gln Tyr Asn Cys Val Thr Pro Thr Pro Gly Phe Asn Phe Ala Lys Arg
                565                 570                 575

Asp Thr Ser Asn Pro Lys Gln Ala Leu Ser His Val Glu Lys Cys Glu
            580                 585                 590

Gly Ser Gly Leu Leu Gly Leu Leu Arg Arg Met Val Ala Asp Ser Lys
        595                 600                 605

Ser Ser
    610
```

<210> 5
<211> 2115
<212> DNA
<213> Nectria mariannaeae

<220>

<221> sig_peptide
<222> (1)..(57)

<220>
<221> Intron
<222> (169)..(222)

<220>
<221> Intron
<222> (310)..(360)

<220>
<221> Intron
<222> (666)..(727)

<220>
<221> Intron
<222> (937)..(985)

<400> 5

```
atgcaattac tctctatcct cgccgtcggg ctcggcctcg cgcagaacgc attctgccaa      60
gaggtcactc atgatcttgc cggtatcaag cgatccctag aatccagaga ttgggttgag     120
gatctttggg ataagttcga aagtgatgca acatgcgcgg gatgcgaggt tagccattca     180
tgttgatttg gtgttgagca tgacgagcta atgaaatatt agtcactcgt gttagtgctc     240
aagggcttgg ccgctataag tgatcaagcg tttattgacg tccttcagga gatttgcaag     300
atctccgggg tgagtacacc ttgactatgg tgcttctaag tggagttgac gatgcagtag     360
gctgaggatg acgatgtttg tgacggttca attcaacttg aaggccccgt tattgccagc     420
ggtcttcgat caatggctat cggctctcga acttccaaag aattctgtac tacattcctt     480
ggactttgcg cgtaccctgc ggtgcagcaa tggagtgttc ccttctcgtc ctccaagtct     540
tccaagactc gtccttcgtc cagtggaaag gaccctatca aggtggttca ttattctgat     600
attcatatcg atcctttata tgtcgggggc tcaaactcca actgcactaa gcccatatgt     660
tgtaggtaag agataacccc aaacacttgc cagcaaaact ggacatgttg ataactaaac     720
tacgaaggtc atacaccaag gctgaccagc ccgggaacaa caaatatcct gctggaccga     780
acggcgacca taactgcgat tctccagtca gtcttgagaa gagcatgtac aacgccatca     840
aggaaatcgt tccagatgca gccttcacca tcttcaccgg ggacattgtc gatcatgcag     900
tctggaatac tagccaatcc tataatacgg aacaaagtgt gttcagtata atccatccag     960
tggaataact tactaaatct cacagttacc aatgcctacg gcttgatgag tgataatctg    1020
ggcacaatct atgggactgc cggcaaccat gaagctcatc ccgctaatgc cttccaacct    1080
aactccgtcg gcaacgtgag ccaatgggta tacgatcttc tttcaggtct ctggtcacag    1140
tggattagca ccgaagccaa agctgactcg gagaagcttg gcgcttactc taccaagtat    1200
cctggcggca acttgcgcat tatctctctc aacaccaaca tgtactatcg agaaaactac    1260
```

tggctctacc gcaagacgat gattcaggac cctagcaatc agatttcctg gctcgtaaac 1320

gaactcgaag ccgctgagac tgcgggcgag cgcgtttata tcatcggcca catgccgctt 1380

ggagactcta acagttttca cgaccagtcc aactaccttg accaagtaat caaccgctac 1440

tccgcaacta tctctgccat gttctttggc cacacacacg acgaccagtt ccagataagt 1500

tactccaatt ggtcaaatcg caacttctcc aatgccctcg taacctccta cattggtccc 1560

tcactcactc ccaccgccgg tatgcccgcc tttcgcgttt acgatgtcga ccccgtgacg 1620

ttcggaatcc tcgactcgac cacatacatc gctgacatga ccgactctgc ctttcaaacc 1680

actggcccag tgtggaagaa gtattattct gccaaagaag tttacggctc tttattgagc 1740

ccggctgtga ctgacagcag cgccgagctc acggcagcct tctggcacaa cgtgacgacc 1800

ctgttcgagg cggacaacac cgcatttgag gcgtttcttt cccgcaagag ccgtgggtgg 1860

aagtcagaat cctgcacagg cacttgcaag gccaacgaga tctgtcaatt gcgcgcggct 1920

cgcagcgaga acaattgcta caccccgtcg ctaggaatta gcttcaacaa acgaagtctg 1980

aacccagttg aagagcggga cgagtgtggg atttcagtga ctagggctac ggttagtgct 2040

atgggcgtaa ggaaagacgt tttgcgtttg ttaaagaaga ggtttatcga gaaggcgggt 2100

gaggtccgtg gctga 2115

<210> 6
<211> 1899
<212> DNA
<213> Artificial sequence

<220>
<223> cDNA

<400> 6

```
atgcaattac tctctatcct cgccgtcggg ctcggcctcg cgcagaacgc attctgccaa      60
gaggtcactc atgatcttgc cggtatcaag cgatccctag aatccagaga ttgggttgag     120
gatctttggg ataagttcga aagtgatgca acatgcgcgg gatgcgagtc actcgtgtta     180
gtgctcaagg gcttggccgc tataagtgat caagcgttta ttgacgtcct tcaggagatt     240
tgcaagatct ccggggctga ggatgacgat gtttgtgacg gttcaattca acttgaaggc     300
cccgttattg ccagcggtct tcgatcaatg gctatcggct ctcgaacttc caaagaattc     360
tgtactacat tccttggact ttgcgcgtac cctgcggtgc agcaatggag tgttcccttc     420
tcgtcctcca agtcttccaa gactcgtcct tcgtccagtg gaaaggaccc tatcaaggtg     480
gttcattatt ctgatattca tatcgatcct ttatatgtcg gggctcaaa ctccaactgc      540
actaagccca tatgttgtag gtcatacacc aaggctgacc agcccgggaa caacaaatat     600
cctgctggac cgaacggcga ccataactgc gattctccag tcagtcttga gaagagcatg     660
```

tacaacgcca tcaaggaaat cgttccagat gcagccttca ccatcttcac cggggacatt 720

gtcgatcatg cagtctggaa tactagccaa tcctataata cggaacaaat taccaatgcc 780

tacggcttga tgagtgataa tctgggcaca atctatggga ctgccggcaa ccatgaagct 840

catcccgcta atgccttcca acctaactcc gtcggcaacg tgagccaatg ggtatacgat 900

cttctttcag gtctctggtc acagtggatt agcaccgaag ccaaagctga ctcggagaag 960

cttggcgctt actctaccaa gtatcctggc ggcaacttgc gcattatctc tctcaacacc 1020

aacatgtact atcgagaaaa ctactggctc taccgcaaga cgatgattca ggaccctagc 1080

aatcagattt cctggctcgt aaacgaactc gaagccgctg agactgcggg cgagcgcgtt 1140

tatatcatcg gccacatgcc gcttggagac tctaacagtt ttcacgacca gtccaactac 1200

cttgaccaag taatcaaccg ctactccgca actatctctg ccatgttctt tggccacaca 1260

cacgacgacc agttccagat aagttactcc aattggtcaa atcgcaactt ctccaatgcc 1320

ctcgtaacct cctacattgg tccctcactc actcccaccg ccggtatgcc cgcctttcgc 1380

gtttacgatg tcgaccccgt gacgttcgga atcctcgact cgaccacata catcgctgac 1440

atgaccgact ctgcctttca aaccactggc ccagtgtgga agaagtatta ttctgccaaa 1500

gaagtttacg gctctttatt gagcccggct gtgactgaca gcagcgccga gctcacggca 1560

gccttctggc acaacgtgac gaccctgttc gaggcggaca acaccgcatt tgaggcgttt 1620

ctttcccgca agagccgtgg gtggaagtca gaatcctgca caggcacttg caaggccaac 1680

gagatctgtc aattgcgcgc ggctcgcagc gagaacaatt gctacacccc gtcgctagga 1740

attagcttca acaaacgaag tctgaaccca gttgaagagc gggacgagtg tgggatttca 1800

gtgactaggg ctacggttag tgctatgggc gtaaggaaag acgttttgcg tttgttaaag 1860

aagaggttta tcgagaaggc gggtgaggtc cgtggctga 1899

<210> 7
<211> 632
<212> PRT
<213> Nectria mariannaeae

<220>
<221> SIGNAL
<222> (1)..(19)

<220>
<221> PROPEP
<222> (20)..(36)

<400> 7

Met Gln Leu Leu Ser Ile Leu Ala Val Gly Leu Gly Leu Ala Gln Asn
1 5 10 15

```
Ala Phe Cys Gln Glu Val Thr His Asp Leu Ala Gly Ile Lys Arg Ser
            20                  25                  30

Leu Glu Ser Arg Asp Trp Val Glu Asp Leu Trp Asp Lys Phe Glu Ser
        35                  40                  45

Asp Ala Thr Cys Ala Gly Cys Glu Ser Leu Val Leu Val Leu Lys Gly
    50                  55                  60

Leu Ala Ala Ile Ser Asp Gln Ala Phe Ile Asp Val Leu Gln Glu Ile
65                  70                  75                  80

Cys Lys Ile Ser Gly Ala Glu Asp Asp Asp Val Cys Asp Gly Ser Ile
                85                  90                  95

Gln Leu Glu Gly Pro Val Ile Ala Ser Gly Leu Arg Ser Met Ala Ile
            100                 105                 110

Gly Ser Arg Thr Ser Lys Glu Phe Cys Thr Thr Phe Leu Gly Leu Cys
        115                 120                 125

Ala Tyr Pro Ala Val Gln Gln Trp Ser Val Pro Phe Ser Ser Ser Lys
    130                 135                 140

Ser Ser Lys Thr Arg Pro Ser Ser Ser Gly Lys Asp Pro Ile Lys Val
145                 150                 155                 160

Val His Tyr Ser Asp Ile His Ile Asp Pro Leu Tyr Val Gly Gly Ser
                165                 170                 175

Asn Ser Asn Cys Thr Lys Pro Ile Cys Cys Arg Ser Tyr Thr Lys Ala
            180                 185                 190

Asp Gln Pro Gly Asn Asn Lys Tyr Pro Ala Gly Pro Asn Gly Asp His
        195                 200                 205

Asn Cys Asp Ser Pro Val Ser Leu Glu Lys Ser Met Tyr Asn Ala Ile
    210                 215                 220

Lys Glu Ile Val Pro Asp Ala Ala Phe Thr Ile Phe Thr Gly Asp Ile
225                 230                 235                 240

Val Asp His Ala Val Trp Asn Thr Ser Gln Ser Tyr Asn Thr Glu Gln
                245                 250                 255

Ile Thr Asn Ala Tyr Gly Leu Met Ser Asp Asn Leu Gly Thr Ile Tyr
            260                 265                 270
```

Gly Thr Ala Gly Asn His Glu Ala His Pro Ala Asn Ala Phe Gln Pro
275 280 285

Asn Ser Val Gly Asn Val Ser Gln Trp Val Tyr Asp Leu Leu Ser Gly
290 295 300

Leu Trp Ser Gln Trp Ile Ser Thr Glu Ala Lys Ala Asp Ser Glu Lys
305 310 315 320

Leu Gly Ala Tyr Ser Thr Lys Tyr Pro Gly Gly Asn Leu Arg Ile Ile
325 330 335

Ser Leu Asn Thr Asn Met Tyr Tyr Arg Glu Asn Tyr Trp Leu Tyr Arg
340 345 350

Lys Thr Met Ile Gln Asp Pro Ser Asn Gln Ile Ser Trp Leu Val Asn
355 360 365

Glu Leu Glu Ala Ala Glu Thr Ala Gly Glu Arg Val Tyr Ile Ile Gly
370 375 380

His Met Pro Leu Gly Asp Ser Asn Ser Phe His Asp Gln Ser Asn Tyr
385 390 395 400

Leu Asp Gln Val Ile Asn Arg Tyr Ser Ala Thr Ile Ser Ala Met Phe
405 410 415

Phe Gly His Thr His Asp Asp Gln Phe Gln Ile Ser Tyr Ser Asn Trp
420 425 430

Ser Asn Arg Asn Phe Ser Asn Ala Leu Val Thr Ser Tyr Ile Gly Pro
435 440 445

Ser Leu Thr Pro Thr Ala Gly Met Pro Ala Phe Arg Val Tyr Asp Val
450 455 460

Asp Pro Val Thr Phe Gly Ile Leu Asp Ser Thr Thr Tyr Ile Ala Asp
465 470 475 480

Met Thr Asp Ser Ala Phe Gln Thr Thr Gly Pro Val Trp Lys Lys Tyr
485 490 495

Tyr Ser Ala Lys Glu Val Tyr Gly Ser Leu Leu Ser Pro Ala Val Thr
500 505 510

Asp Ser Ser Ala Glu Leu Thr Ala Ala Phe Trp His Asn Val Thr Thr
515 520 525

```
Leu Phe Glu Ala Asp Asn Thr Ala Phe Glu Ala Phe Leu Ser Arg Lys
    530                 535                 540

Ser Arg Gly Trp Lys Ser Glu Ser Cys Thr Gly Thr Cys Lys Ala Asn
545                 550                 555                 560

Glu Ile Cys Gln Leu Arg Ala Ala Arg Ser Glu Asn Asn Cys Tyr Thr
                565                 570                 575

Pro Ser Leu Gly Ile Ser Phe Asn Lys Arg Ser Leu Asn Pro Val Glu
            580                 585                 590

Glu Arg Asp Glu Cys Gly Ile Ser Val Thr Arg Ala Thr Val Ser Ala
        595                 600                 605

Met Gly Val Arg Lys Asp Val Leu Arg Leu Leu Lys Lys Arg Phe Ile
    610                 615                 620

Glu Lys Ala Gly Glu Val Arg Gly
625                 630
```

<210> 8
<211> 969
<212> DNA
<213> pseudomonas sp

<220>
<221> sig_peptide
<222> (1)..(75)

<400> 8

```
atgactcatc tcattggttt cgcccctcgg ttgctggctt tttcggcgct gttgctcagc      60
cagacggcat tcagccagga aagcccggca ttcatcgatc ctgcgtcgtg gaacaccccg     120
ttcaatggca ttgcccaggt ggcttgtcat aactgctacg agaagcaata cgcgaacacc     180
ttcagcagtg tgcttgacag tgtacggacc ctggagctgg acttctggga ccagcgcgat     240
gcggtgagcg gcggttcgcc ccatcactgg ttcgtgcggc acaaccccgg caccttgttc     300
caatccggca acgacaataa ctgcaccggc gatggcaccg gcaagaacga cctcgaagcc     360
tgtctgaacg acgtcaagaa ctggagtgac aagcatccgg ggcacttccc catcacgctg     420
atcctggaca agaaacaggg ctggtcgaaa gaaagttcgg ggcgcacacc aaaggatttc     480
gacgaactgg tggcgcgggt gttccagggc aagctcttta ccccccagga tctggcgacg     540
cacattggca gtggcgcggg ggccttgcag ggcaacctca agggtaagtc ctggcccacc     600
gccaacgatc tgcagggcaa ggtgctgttg gtgctcaacc actcggaaaa ccagaagctc     660
```

tcgcagtacg ccgaggcccg cacctctaag gctaaggtgt tcatttcgcc agtaaccaac 720

ggccagaacg atatcagtgg caaggtcagc ggcatgtcca gccagtcatc cggctatgta 780

gccatgaaca acatgggcaa gggcgacaaa agttgggcaa agcaggcctt tgcctacagc 840

catatcggcc gcgtctgggg tgatgacgag gtgtcgttcg cccagcacat caaccagaag 900

atcaatctgt cggcgtacta caggttcgcc gcgcagagcg ctggcggcta ccgcatccgg 960

ccgttctga 969

<210> 9
<211> 322
<212> PRT
<213> pseudomonas sp

<220>
<221> SIGNAL
<222> (1)..(25)

<400> 9

```
Met Thr His Leu Ile Gly Phe Ala Pro Arg Leu Leu Ala Phe Ser Ala
1               5                   10                  15

Leu Leu Leu Ser Gln Thr Ala Phe Ser Gln Glu Ser Pro Ala Phe Ile
            20                  25                  30

Asp Pro Ala Ser Trp Asn Thr Pro Phe Asn Gly Ile Ala Gln Val Ala
        35                  40                  45

Cys His Asn Cys Tyr Glu Lys Gln Tyr Ala Asn Thr Phe Ser Ser Val
    50                  55                  60

Leu Asp Ser Val Arg Thr Leu Glu Leu Asp Phe Trp Asp Gln Arg Asp
65                  70                  75                  80

Ala Val Ser Gly Gly Ser Pro His His Trp Phe Val Arg His Asn Pro
                85                  90                  95

Gly Thr Leu Phe Gln Ser Gly Asn Asp Asn Asn Cys Thr Gly Asp Gly
            100                 105                 110

Thr Gly Lys Asn Asp Leu Glu Ala Cys Leu Asn Asp Val Lys Asn Trp
        115                 120                 125

Ser Asp Lys His Pro Gly His Phe Pro Ile Thr Leu Ile Leu Asp Lys
    130                 135                 140

Lys Gln Gly Trp Ser Lys Glu Ser Ser Gly Arg Thr Pro Lys Asp Phe
145                 150                 155                 160
```

Asp Glu Leu Val Ala Arg Val Phe Gln Gly Lys Leu Phe Thr Pro Gln
165 170 175

Asp Leu Ala Thr His Ile Gly Ser Gly Ala Gly Ala Leu Gln Gly Asn
180 185 190

Leu Lys Gly Lys Ser Trp Pro Thr Ala Asn Asp Leu Gln Gly Lys Val
195 200 205

Leu Leu Val Leu Asn His Ser Glu Asn Gln Lys Leu Ser Gln Tyr Ala
210 215 220

Glu Ala Arg Thr Ser Lys Ala Lys Val Phe Ile Ser Pro Val Thr Asn
225 230 235 240

Gly Gln Asn Asp Ile Ser Gly Lys Val Ser Gly Met Ser Ser Gln Ser
245 250 255

Ser Gly Tyr Val Ala Met Asn Asn Met Gly Lys Gly Asp Lys Ser Trp
260 265 270

Ala Lys Gln Ala Phe Ala Tyr Ser His Ile Gly Arg Val Trp Gly Asp
275 280 285

Asp Glu Val Ser Phe Ala Gln His Ile Asn Gln Lys Ile Asn Leu Ser
290 295 300

Ala Tyr Tyr Arg Phe Ala Ala Gln Ser Ala Gly Gly Tyr Arg Ile Arg
305 310 315 320

Pro Phe

<210> 10
<211> 298
<212> PRT
<213> Artificial sequence

<220>
<223> Variant

<400> 10

Ala Gln Glu Ser Pro Ala Phe Ile Asp Pro Ala Ser Trp Asn Thr Pro
1 5 10 15

Phe Asn Gly Ile Ala Gln Val Ala Cys His Asn Cys Tyr Glu Lys Gln
20 25 30

```
Tyr Ala Asn Thr Phe Ser Ser Val Leu Asp Ser Val Arg Thr Leu Glu
        35                  40                  45

Leu Asp Phe Trp Asp Gln Arg Asp Ala Val Ser Gly Gly Ser Pro His
    50                  55                  60

His Trp Phe Val Arg His Asn Pro Gly Thr Leu Phe Gln Ser Gly Asn
65                  70                  75                  80

Asp Asn Asn Cys Thr Gly Asp Gly Thr Gly Lys Asn Asp Leu Glu Ala
                85                  90                  95

Cys Leu Asn Asp Val Lys Asn Trp Ser Asp Lys His Pro Gly His Phe
            100                 105                 110

Pro Ile Thr Leu Ile Leu Asp Lys Lys Gln Gly Trp Ser Lys Glu Ser
        115                 120                 125

Ser Gly Arg Thr Pro Lys Asp Phe Asp Glu Leu Val Ala Arg Val Phe
    130                 135                 140

Gln Gly Lys Leu Phe Thr Pro Gln Asp Leu Ala Thr His Ile Gly Ser
145                 150                 155                 160

Gly Ala Gly Ala Leu Gln Gly Asn Leu Lys Gly Lys Ser Trp Pro Thr
                165                 170                 175

Ala Asn Asp Leu Gln Gly Lys Val Leu Leu Val Leu Asn His Ser Glu
            180                 185                 190

Asn Gln Lys Leu Ser Gln Tyr Ala Glu Ala Arg Thr Ser Lys Ala Lys
        195                 200                 205

Val Phe Ile Ser Pro Val Thr Asn Gly Gln Asn Asp Ile Ser Gly Lys
    210                 215                 220

Val Ser Gly Met Ser Ser Gln Ser Ser Gly Tyr Val Ala Met Asn Asn
225                 230                 235                 240

Met Gly Lys Gly Asp Lys Ser Trp Ala Lys Gln Ala Phe Ala Tyr Ser
                245                 250                 255

His Ile Gly Arg Val Trp Gly Asp Asp Glu Val Ser Phe Ala Gln His
            260                 265                 270

Ile Asn Gln Lys Ile Asn Leu Ser Ala Tyr Tyr Arg Phe Ala Ala Gln
        275                 280                 285
```

Ser Ala Gly Gly Tyr Arg Ile Arg Pro Phe
290 295

<210> 11
<211> 972
<212> DNA
<213> Pseudomonas chlororaphis

<220>
<221> sig_peptide
<222> (1)..(75)

<400> 11

atgtcccgtc tcactcgctt tgccctccgt accgccgtga tgcccctggt gctggccagc 60
cagatggcgt ccagccagga ggctgtggga ttcatttccc cggcttcctg gtacaccgcc 120
ttcaacgcta tcgcccaggt ggcgtgccat aactgctacg aaaaacagta cgccggcacc 180
ttcaccagcg tgctcgacag cgtgcgcacc ctggagctgg acttctggga ccaacgcgat 240
gcggtcaccg gaggttcgcc ccgccactgg ttcgtgcggc acaaccccgg cagcctgttc 300
cagtccggca atgacaacaa ttgcaccggc gacggcaaag gcaccaacga tcttgaggcc 360
tgcctcaatg acatcaagct ctggagcgac agccatcccg ggcacttccc gattaccctg 420
atcctcgaca agaagcaggg ctggtcgaag gaaagctccg ggcgtacgcc taaagacttc 480
gatgacctgg tcagccggat tttccagggc aagctctaca ccccgggcga cctggcccag 540
cacctgggtg tcagcagcag tgccttgcag ggctcgctca agggcaagtc ctggccgacc 600
gccagccaac tgcagggcaa ggtgctgttg gtgctcaacc actcggagaa ccagaagctc 660
tcgcaatacg ccgaggcccg caccaccagc gccaaggtgt tcatttcccc ggtcaccaac 720
ggccagaacg atgtcagtgg cgaggtcagc ggcatgtcca ggacgtcgtc cggctacgtg 780
gccatgaaca acatgggcaa gggcgacaag cagtgggccg cgcaagcctt tgcctacagc 840
cacatcggtc gggtgtgggg cgatgacggc gtgtcattca cccagcacat cgccgagaag 900
gtcaatctgt cggcgtatta caaattcgcc gaggccaagg atggcaacgg ctatcgcatc 960
cggccgttct ga 972

<210> 12
<211> 323
<212> PRT
<213> pseudomonas chlororaphis

<220>
<221> SIGNAL
<222> (1)..(25)

<400> 12

```
Met Ser Arg Leu Thr Arg Phe Ala Leu Arg Thr Ala Val Met Pro Leu
1               5                   10                  15

Val Leu Ala Ser Gln Met Ala Ser Ser Gln Glu Ala Val Gly Phe Ile
            20                  25                  30

Ser Pro Ala Ser Trp Tyr Thr Ala Phe Asn Ala Ile Ala Gln Val Ala
        35                  40                  45

Cys His Asn Cys Tyr Glu Lys Gln Tyr Ala Gly Thr Phe Thr Ser Val
    50                  55                  60

Leu Asp Ser Val Arg Thr Leu Glu Leu Asp Phe Trp Asp Gln Arg Asp
65                  70                  75                  80

Ala Val Thr Gly Gly Ser Pro Arg His Trp Phe Val Arg His Asn Pro
                85                  90                  95

Gly Ser Leu Phe Gln Ser Gly Asn Asp Asn Asn Cys Thr Gly Asp Gly
            100                 105                 110

Lys Gly Thr Asn Asp Leu Glu Ala Cys Leu Asn Asp Ile Lys Leu Trp
        115                 120                 125

Ser Asp Ser His Pro Gly His Phe Pro Ile Thr Leu Ile Leu Asp Lys
    130                 135                 140

Lys Gln Gly Trp Ser Lys Glu Ser Ser Gly Arg Thr Pro Lys Asp Phe
145                 150                 155                 160

Asp Asp Leu Val Ser Arg Ile Phe Gln Gly Lys Leu Tyr Thr Pro Gly
                165                 170                 175

Asp Leu Ala Gln His Leu Gly Val Ser Ser Ser Ala Leu Gln Gly Ser
            180                 185                 190

Leu Lys Gly Lys Ser Trp Pro Thr Ala Ser Gln Leu Gln Gly Lys Val
        195                 200                 205

Leu Leu Val Leu Asn His Ser Glu Asn Gln Lys Leu Ser Gln Tyr Ala
    210                 215                 220

Glu Ala Arg Thr Thr Ser Ala Lys Val Phe Ile Ser Pro Val Thr Asn
225                 230                 235                 240

Gly Gln Asn Asp Val Ser Gly Glu Val Ser Gly Met Ser Arg Thr Ser
                245                 250                 255
```

Ser Gly Tyr Val Ala Met Asn Asn Met Gly Lys Gly Asp Lys Gln Trp
260 265 270

Ala Ala Gln Ala Phe Ala Tyr Ser His Ile Gly Arg Val Trp Gly Asp
275 280 285

Asp Gly Val Ser Phe Thr Gln His Ile Ala Glu Lys Val Asn Leu Ser
290 295 300

Ala Tyr Tyr Lys Phe Ala Glu Ala Lys Asp Gly Asn Gly Tyr Arg Ile
305 310 315 320

Arg Pro Phe

<210> 13
<211> 299
<212> PRT
<213> Artificial sequence

<220>
<223> Variant

<400> 13

Ala Gln Glu Ala Val Gly Phe Ile Ser Pro Ala Ser Trp Tyr Thr Ala
1 5 10 15

Phe Asn Ala Ile Ala Gln Val Ala Cys His Asn Cys Tyr Glu Lys Gln
20 25 30

Tyr Ala Gly Thr Phe Thr Ser Val Leu Asp Ser Val Arg Thr Leu Glu
35 40 45

Leu Asp Phe Trp Asp Gln Arg Asp Ala Val Thr Gly Gly Ser Pro Arg
50 55 60

His Trp Phe Val Arg His Asn Pro Gly Ser Leu Phe Gln Ser Gly Asn
65 70 75 80

Asp Asn Asn Cys Thr Gly Asp Gly Lys Gly Thr Asn Asp Leu Glu Ala
85 90 95

Cys Leu Asn Asp Ile Lys Leu Trp Ser Asp Ser His Pro Gly His Phe
100 105 110

Pro Ile Thr Leu Ile Leu Asp Lys Lys Gln Gly Trp Ser Lys Glu Ser
115 120 125

```
Ser Gly Arg Thr Pro Lys Asp Phe Asp Asp Leu Val Ser Arg Ile Phe
    130                 135                 140

Gln Gly Lys Leu Tyr Thr Pro Gly Asp Leu Ala Gln His Leu Gly Val
145                 150                 155                 160

Ser Ser Ser Ala Leu Gln Gly Ser Leu Lys Gly Lys Ser Trp Pro Thr
                165                 170                 175

Ala Ser Gln Leu Gln Gly Lys Val Leu Leu Val Leu Asn His Ser Glu
            180                 185                 190

Asn Gln Lys Leu Ser Gln Tyr Ala Glu Ala Arg Thr Thr Ser Ala Lys
        195                 200                 205

Val Phe Ile Ser Pro Val Thr Asn Gly Gln Asn Asp Val Ser Gly Glu
    210                 215                 220

Val Ser Gly Met Ser Arg Thr Ser Ser Gly Tyr Val Ala Met Asn Asn
225                 230                 235                 240

Met Gly Lys Gly Asp Lys Gln Trp Ala Ala Gln Ala Phe Ala Tyr Ser
                245                 250                 255

His Ile Gly Arg Val Trp Gly Asp Asp Gly Val Ser Phe Thr Gln His
            260                 265                 270

Ile Ala Glu Lys Val Asn Leu Ser Ala Tyr Tyr Lys Phe Ala Glu Ala
        275                 280                 285

Lys Asp Gly Asn Gly Tyr Arg Ile Arg Pro Phe
    290                 295
```

<210> 14
<211> 972
<212> DNA
<213> pseudomonas sp

<220>
<221> sig_peptide
<222> (1)..(75)

<400> 14

```
atgtcccgtc tcactggttt tgccctccgt accgccgtgc tgcccctggc actggccagc      60
cagatggcgt ccagccagga ggccgtggga ttcatttccc cggcgtcctg gtacaccgcc     120
ttcaacgcta tcgcccaggt ggcatgccat aactgctacg aaaaacagta cgccggcacc     180
ttcaccagcg tgctcgatag cgtgcgcacc ctggagctgg acttctggga ccaacgcgat     240
gcggtcaccg gaggttcgcc ccgccactgg ttcgtgcggc acaaccccgg cagcctgttc     300
cagtccggca atgacaacaa ttgcaccggc gacggcaacg gcaccaacga tctcgaagcc     360
tgcctcaacg acatcaagct ctggagcgac agccatcccg ggcacttccc gattaccctg     420
atcctcgaca agaagcaggg ctggtcgaag gaaagctccg ggcgtacgcc gaaagacttc     480
gatgacctgg tcagccggat tttccagggc aagctctaca ccccgggcga cctggcccag     540
catctgggtg tcagcagcag tgccttgcag ggctcgctca agggcaagtc ctggccgacc     600
gccagccaac tgcagggcaa ggtgctgctg gtgctcaacc actcggagaa ccagaagctc     660
tcgcaatacg ccgaggcccg caccaccagc gccaaggtgt tcatttcccc ggtcaccaac     720
ggccagaacg atgtcagtgg cgaggtcagc ggcatgtcga ggacgtcgtc cggctacgtg     780
gccatgaaca acatgggcaa gggcgacaag cagtgggccg cgcaagcctt tgtctacagc     840
cacatcggtc gggtgtgggg tgatgacggc gtgtcattca cccagcacat cgccgagaag     900
gtcaatctgt cggcgtatta caaattcgcc gaggccaagg atggcaacgg ctatcgcatc     960
cgaccgttct ga                                                         972
```

<210> 15
<211> 323
<212> PRT
<213> pseudomonas sp

<220>
<221> SIGNAL
<222> (1)..(25)

<400> 15

```
Met Ser Arg Leu Thr Gly Phe Ala Leu Arg Thr Ala Val Leu Pro Leu
1               5                   10                  15

Ala Leu Ala Ser Gln Met Ala Ser Ser Gln Glu Ala Val Gly Phe Ile
            20                  25                  30

Ser Pro Ala Ser Trp Tyr Thr Ala Phe Asn Ala Ile Ala Gln Val Ala
        35                  40                  45

Cys His Asn Cys Tyr Glu Lys Gln Tyr Ala Gly Thr Phe Thr Ser Val
    50                  55                  60

Leu Asp Ser Val Arg Thr Leu Glu Leu Asp Phe Trp Asp Gln Arg Asp
65                  70                  75                  80

Ala Val Thr Gly Gly Ser Pro Arg His Trp Phe Val Arg His Asn Pro
                85                  90                  95
```

Gly Ser Leu Phe Gln Ser Gly Asn Asp Asn Asn Cys Thr Gly Asp Gly
100 105 110

Asn Gly Thr Asn Asp Leu Glu Ala Cys Leu Asn Asp Ile Lys Leu Trp
115 120 125

Ser Asp Ser His Pro Gly His Phe Pro Ile Thr Leu Ile Leu Asp Lys
130 135 140

Lys Gln Gly Trp Ser Lys Glu Ser Ser Gly Arg Thr Pro Lys Asp Phe
145 150 155 160

Asp Asp Leu Val Ser Arg Ile Phe Gln Gly Lys Leu Tyr Thr Pro Gly
165 170 175

Asp Leu Ala Gln His Leu Gly Val Ser Ser Ser Ala Leu Gln Gly Ser
180 185 190

Leu Lys Gly Lys Ser Trp Pro Thr Ala Ser Gln Leu Gln Gly Lys Val
195 200 205

Leu Leu Val Leu Asn His Ser Glu Asn Gln Lys Leu Ser Gln Tyr Ala
210 215 220

Glu Ala Arg Thr Thr Ser Ala Lys Val Phe Ile Ser Pro Val Thr Asn
225 230 235 240

Gly Gln Asn Asp Val Ser Gly Glu Val Ser Gly Met Ser Arg Thr Ser
245 250 255

Ser Gly Tyr Val Ala Met Asn Asn Met Gly Lys Gly Asp Lys Gln Trp
260 265 270

Ala Ala Gln Ala Phe Val Tyr Ser His Ile Gly Arg Val Trp Gly Asp
275 280 285

Asp Gly Val Ser Phe Thr Gln His Ile Ala Glu Lys Val Asn Leu Ser
290 295 300

Ala Tyr Tyr Lys Phe Ala Glu Ala Lys Asp Gly Asn Gly Tyr Arg Ile
305 310 315 320

Arg Pro Phe

<210> 16
<211> 299
<212> PRT

<213> Artificial sequence

<220>
<223> Variant

<400> 16

```
Ala Gln Glu Ala Val Gly Phe Ile Ser Pro Ala Ser Trp Tyr Thr Ala
1               5                   10                  15

Phe Asn Ala Ile Ala Gln Val Ala Cys His Asn Cys Tyr Glu Lys Gln
            20                  25                  30

Tyr Ala Gly Thr Phe Thr Ser Val Leu Asp Ser Val Arg Thr Leu Glu
        35                  40                  45

Leu Asp Phe Trp Asp Gln Arg Asp Ala Val Thr Gly Gly Ser Pro Arg
    50                  55                  60

His Trp Phe Val Arg His Asn Pro Gly Ser Leu Phe Gln Ser Gly Asn
65                  70                  75                  80

Asp Asn Asn Cys Thr Gly Asp Gly Asn Gly Thr Asn Asp Leu Glu Ala
                85                  90                  95

Cys Leu Asn Asp Ile Lys Leu Trp Ser Asp Ser His Pro Gly His Phe
            100                 105                 110

Pro Ile Thr Leu Ile Leu Asp Lys Lys Gln Gly Trp Ser Lys Glu Ser
        115                 120                 125

Ser Gly Arg Thr Pro Lys Asp Phe Asp Asp Leu Val Ser Arg Ile Phe
    130                 135                 140

Gln Gly Lys Leu Tyr Thr Pro Gly Asp Leu Ala Gln His Leu Gly Val
145                 150                 155                 160

Ser Ser Ser Ala Leu Gln Gly Ser Leu Lys Gly Lys Ser Trp Pro Thr
                165                 170                 175

Ala Ser Gln Leu Gln Gly Lys Val Leu Leu Val Leu Asn His Ser Glu
            180                 185                 190

Asn Gln Lys Leu Ser Gln Tyr Ala Glu Ala Arg Thr Thr Ser Ala Lys
        195                 200                 205

Val Phe Ile Ser Pro Val Thr Asn Gly Gln Asn Asp Val Ser Gly Glu
    210                 215                 220
```

```
Val Ser Gly Met Ser Arg Thr Ser Ser Gly Tyr Val Ala Met Asn Asn
225                 230                 235                 240

Met Gly Lys Gly Asp Lys Gln Trp Ala Ala Gln Ala Phe Val Tyr Ser
                245                 250                 255

His Ile Gly Arg Val Trp Gly Asp Asp Gly Val Ser Phe Thr Gln His
            260                 265                 270

Ile Ala Glu Lys Val Asn Leu Ser Ala Tyr Tyr Lys Phe Ala Glu Ala
        275                 280                 285

Lys Asp Gly Asn Gly Tyr Arg Ile Arg Pro Phe
    290                 295
```

<210> 17
<211> 972
<212> DNA
<213> Pseudomonas chlororaphis

<220>
<221> sig_peptide
<222> (1)..(84)

<400> 17

```
atgtcccgtc tcactcgttt tgccctccgt acagccgtac tgcccctggc gctggccagc      60
cagatggcgt ccagccagga ggccgcggga ttcatttctc cggcttcctg gtacagcgcc     120
ttcaacgcta tcgcccaggt ggcgtgccat aactgctacg aaaaacagta cgccagcacc     180
ttcaccagtg tgctcgacag cgtgcgtacc ctggagctgg atttctggga ccagcgcgat     240
gcggtcaccg gtggttcggc cggtcactgg ttcgtgcggc acaaccccgg cacgctgttc     300
cagtccggca atgacaacaa ctgcaccggc gacggcaaag gcaccaacga tctcgaggcc     360
tgcctcaacg acatcaggct ctggagcgac agccatcccg ggcacttccc gattaccctg     420
atcctcgaca agaagcaggg ctggtcgaag gaaagctccg ggcgtacgcc gaaagacttc     480
gatgatctgg tcagccggat tttccagggc aagctctata ccccgggcga cctggcccag     540
cacctgggtg tcagcagcgg tgccttgcag ggctcgctcg agggcaagtc ctggccgacc     600
gctagccaac tgcagggcaa ggtgctgctg gtgctcaacc actcggagaa ccagaagctc     660
tcgcaatacg ccgaagcccg gaccaccagc gccaaggtgt tcatttcacc ggtcaccaac     720
ggccagaacg atgtcagtgg cgaggtcagc ggcatgtcca ggacgtcgtc cggttacgtg     780
gccatgaaca acatgggcaa gggcgacaag cagtgggccg cacaagcctt tgcctacagc     840
cacatcggtc gggtgtgggg tgatgacggc gtgtcattca cccagcacat cgccgagaag     900
gtcaatctgt cggcgtatta caagttcgcc gaagccaagg atggcaacgg ctatcgcatc     960

cggccgttct aa                                                         972
```

<210> 18
<211> 323
<212> PRT
<213> Pseudomonas chlororaphis

<220>
<221> SIGNAL
<222> (1)..(28)

<400> 18

```
Met Ser Arg Leu Thr Arg Phe Ala Leu Arg Thr Ala Val Leu Pro Leu
1               5                   10                  15

Ala Leu Ala Ser Gln Met Ala Ser Ser Gln Glu Ala Ala Gly Phe Ile
            20                  25                  30

Ser Pro Ala Ser Trp Tyr Ser Ala Phe Asn Ala Ile Ala Gln Val Ala
        35                  40                  45

Cys His Asn Cys Tyr Glu Lys Gln Tyr Ala Ser Thr Phe Thr Ser Val
    50                  55                  60

Leu Asp Ser Val Arg Thr Leu Glu Leu Asp Phe Trp Asp Gln Arg Asp
65                  70                  75                  80

Ala Val Thr Gly Gly Ser Ala Gly His Trp Phe Val Arg His Asn Pro
                85                  90                  95

Gly Thr Leu Phe Gln Ser Gly Asn Asp Asn Asn Cys Thr Gly Asp Gly
            100                 105                 110

Lys Gly Thr Asn Asp Leu Glu Ala Cys Leu Asn Asp Ile Arg Leu Trp
        115                 120                 125

Ser Asp Ser His Pro Gly His Phe Pro Ile Thr Leu Ile Leu Asp Lys
    130                 135                 140

Lys Gln Gly Trp Ser Lys Glu Ser Ser Gly Arg Thr Pro Lys Asp Phe
145                 150                 155                 160

Asp Asp Leu Val Ser Arg Ile Phe Gln Gly Lys Leu Tyr Thr Pro Gly
                165                 170                 175

Asp Leu Ala Gln His Leu Gly Val Ser Ser Gly Ala Leu Gln Gly Ser
            180                 185                 190
```

Leu Glu Gly Lys Ser Trp Pro Thr Ala Ser Gln Leu Gln Gly Lys Val
195 200 205

Leu Leu Val Leu Asn His Ser Glu Asn Gln Lys Leu Ser Gln Tyr Ala
210 215 220

Glu Ala Arg Thr Thr Ser Ala Lys Val Phe Ile Ser Pro Val Thr Asn
225 230 235 240

Gly Gln Asn Asp Val Ser Gly Glu Val Ser Gly Met Ser Arg Thr Ser
245 250 255

Ser Gly Tyr Val Ala Met Asn Asn Met Gly Lys Gly Asp Lys Gln Trp
260 265 270

Ala Ala Gln Ala Phe Ala Tyr Ser His Ile Gly Arg Val Trp Gly Asp
275 280 285

Asp Gly Val Ser Phe Thr Gln His Ile Ala Glu Lys Val Asn Leu Ser
290 295 300

Ala Tyr Tyr Lys Phe Ala Glu Ala Lys Asp Gly Asn Gly Tyr Arg Ile
305 310 315 320

Arg Pro Phe

<210> 19
<211> 296
<212> PRT
<213> Artificial sequence

<220>
<223> Variant

<400> 19

Ala Ala Gly Phe Ile Ser Pro Ala Ser Trp Tyr Ser Ala Phe Asn Ala
1 5 10 15

Ile Ala Gln Val Ala Cys His Asn Cys Tyr Glu Lys Gln Tyr Ala Ser
20 25 30

Thr Phe Thr Ser Val Leu Asp Ser Val Arg Thr Leu Glu Leu Asp Phe
35 40 45

Trp Asp Gln Arg Asp Ala Val Thr Gly Gly Ser Ala Gly His Trp Phe
50 55 60

```
Val Arg His Asn Pro Gly Thr Leu Phe Gln Ser Gly Asn Asp Asn Asn
65                  70                  75                  80

Cys Thr Gly Asp Gly Lys Gly Thr Asn Asp Leu Glu Ala Cys Leu Asn
                85                  90                  95

Asp Ile Arg Leu Trp Ser Asp Ser His Pro Gly His Phe Pro Ile Thr
            100                 105                 110

Leu Ile Leu Asp Lys Lys Gln Gly Trp Ser Lys Glu Ser Ser Gly Arg
        115                 120                 125

Thr Pro Lys Asp Phe Asp Asp Leu Val Ser Arg Ile Phe Gln Gly Lys
    130                 135                 140

Leu Tyr Thr Pro Gly Asp Leu Ala Gln His Leu Gly Val Ser Ser Gly
145                 150                 155                 160

Ala Leu Gln Gly Ser Leu Glu Gly Lys Ser Trp Pro Thr Ala Ser Gln
                165                 170                 175

Leu Gln Gly Lys Val Leu Leu Val Leu Asn His Ser Glu Asn Gln Lys
            180                 185                 190

Leu Ser Gln Tyr Ala Glu Ala Arg Thr Thr Ser Ala Lys Val Phe Ile
        195                 200                 205

Ser Pro Val Thr Asn Gly Gln Asn Asp Val Ser Gly Glu Val Ser Gly
    210                 215                 220

Met Ser Arg Thr Ser Ser Gly Tyr Val Ala Met Asn Asn Met Gly Lys
225                 230                 235                 240

Gly Asp Lys Gln Trp Ala Ala Gln Ala Phe Ala Tyr Ser His Ile Gly
                245                 250                 255

Arg Val Trp Gly Asp Asp Gly Val Ser Phe Thr Gln His Ile Ala Glu
            260                 265                 270

Lys Val Asn Leu Ser Ala Tyr Tyr Lys Phe Ala Glu Ala Lys Asp Gly
        275                 280                 285

Asn Gly Tyr Arg Ile Arg Pro Phe
    290                 295
```

<210> 20
<211> 969

<212> DNA
<213> Pseudomonas protegens

<220>
<221> sig_peptide
<222> (1)..(75)

<400> 20

```
atgactcatc tttcccgttt cgtcccccgc gccctggcgc tgtcggcact cttgctcagc      60
ccggcggcgt tcagccagga aagcccggcg ttcatcgccc cggctacctg gaatacccg      120
ttcaacggca tcgcccaggt ggcctgtcac aactgctacg agaagcaata cgcaagcacc     180
ttcagcagcg tgctcgacag tgtgcgcacc ctggaactgg acttctggga ccagcgcgat     240
gcggtgaccg gcggctcgcc ccggcactgg ttcgtgcggc acaaccccgg caccctgttc     300
cagtccggca acgacaacaa ttgcacgggc gatggcaccg gcaagaacga tctcgaagcc     360
tgcctcaacg acgtcaagaa ctggagcgaa aaccaccccg ggcactttcc catcacggtg     420
atcctcgaca agaagcaggg ctggtcgaag gaaagctcgg ggcgcacgcc caaggatttc     480
gatgagctgg tgacccgggt cttccagggc aagctctata ccccccagga cctggccacg     540
cacatcggca gcagcgcggg cgccttgcag ggcaacctca agggcaagtc ctggcccacc     600
gccagccagc ttcagggcaa ggtcctgctg gtgctcaacc actcggaaaa ccagaagctc     660
tcgcagtacg ccgaggcccg gacttccagc gccaaggtgt tcatctcgcc ggtgaccaat     720
ggccagaacg atgtcagcgg caaggtcagt ggcatgtccg gccagtcgtc cggctacgtg     780
gccatgaaca acatgtccaa gggcgacaag aagtgggcgg cccaggcctt tgcctacagc     840
catgtgggcc gggtctgggg cgatgacggg gtgtcgttcg cccagcacat cagcgagaag     900
gtcaacctct cggcctacta caagttcgcc gagcagaact ccggcggcta tcgcatccgg     960
ccgttctga                                                             969
```

<210> 21
<211> 322
<212> PRT
<213> Pseudomonas protegens

<220>
<221> SIGNAL
<222> (1)..(25)

<400> 21

```
Met Thr His Leu Ser Arg Phe Val Pro Arg Ala Leu Ala Leu Ser Ala
1               5                   10                  15

Leu Leu Leu Ser Pro Ala Ala Phe Ser Gln Glu Ser Pro Ala Phe Ile
            20                  25                  30
```

```
Ala Pro Ala Thr Trp Asn Thr Pro Phe Asn Gly Ile Ala Gln Val Ala
        35                  40                  45

Cys His Asn Cys Tyr Glu Lys Gln Tyr Ala Ser Thr Phe Ser Ser Val
    50                  55                  60

Leu Asp Ser Val Arg Thr Leu Glu Leu Asp Phe Trp Asp Gln Arg Asp
65                  70                  75                  80

Ala Val Thr Gly Gly Ser Pro Arg His Trp Phe Val Arg His Asn Pro
                85                  90                  95

Gly Thr Leu Phe Gln Ser Gly Asn Asp Asn Asn Cys Thr Gly Asp Gly
            100                 105                 110

Thr Gly Lys Asn Asp Leu Glu Ala Cys Leu Asn Asp Val Lys Asn Trp
        115                 120                 125

Ser Glu Asn His Pro Gly His Phe Pro Ile Thr Val Ile Leu Asp Lys
    130                 135                 140

Lys Gln Gly Trp Ser Lys Glu Ser Ser Gly Arg Thr Pro Lys Asp Phe
145                 150                 155                 160

Asp Glu Leu Val Thr Arg Val Phe Gln Gly Lys Leu Tyr Thr Pro Gln
                165                 170                 175

Asp Leu Ala Thr His Ile Gly Ser Ser Ala Gly Ala Leu Gln Gly Asn
            180                 185                 190

Leu Lys Gly Lys Ser Trp Pro Thr Ala Ser Gln Leu Gln Gly Lys Val
        195                 200                 205

Leu Leu Val Leu Asn His Ser Glu Asn Gln Lys Leu Ser Gln Tyr Ala
    210                 215                 220

Glu Ala Arg Thr Ser Ser Ala Lys Val Phe Ile Ser Pro Val Thr Asn
225                 230                 235                 240

Gly Gln Asn Asp Val Ser Gly Lys Val Ser Gly Met Ser Gly Gln Ser
                245                 250                 255

Ser Gly Tyr Val Ala Met Asn Asn Met Ser Lys Gly Asp Lys Lys Trp
            260                 265                 270

Ala Ala Gln Ala Phe Ala Tyr Ser His Val Gly Arg Val Trp Gly Asp
        275                 280                 285
```

Asp Gly Val Ser Phe Ala Gln His Ile Ser Glu Lys Val Asn Leu Ser
    290                 295                 300

Ala Tyr Tyr Lys Phe Ala Glu Gln Asn Ser Gly Gly Tyr Arg Ile Arg
305                 310                 315                 320

Pro Phe

<210> 22
<211> 298
<212> PRT
<213> Artificial sequence

<220>
<223> Variant

<400> 22

Ala Gln Glu Ser Pro Ala Phe Ile Ala Pro Ala Thr Trp Asn Thr Pro
1               5                   10                  15

Phe Asn Gly Ile Ala Gln Val Ala Cys His Asn Cys Tyr Glu Lys Gln
            20                  25                  30

Tyr Ala Ser Thr Phe Ser Ser Val Leu Asp Ser Val Arg Thr Leu Glu
        35                  40                  45

Leu Asp Phe Trp Asp Gln Arg Asp Ala Val Thr Gly Gly Ser Pro Arg
    50                  55                  60

His Trp Phe Val Arg His Asn Pro Gly Thr Leu Phe Gln Ser Gly Asn
65                  70                  75                  80

Asp Asn Asn Cys Thr Gly Asp Gly Thr Gly Lys Asn Asp Leu Glu Ala
                85                  90                  95

Cys Leu Asn Asp Val Lys Asn Trp Ser Glu Asn His Pro Gly His Phe
            100                 105                 110

Pro Ile Thr Val Ile Leu Asp Lys Lys Gln Gly Trp Ser Lys Glu Ser
        115                 120                 125

Ser Gly Arg Thr Pro Lys Asp Phe Asp Glu Leu Val Thr Arg Val Phe
    130                 135                 140

Gln Gly Lys Leu Tyr Thr Pro Gln Asp Leu Ala Thr His Ile Gly Ser
145                 150                 155                 160

```
Ser Ala Gly Ala Leu Gln Gly Asn Leu Lys Gly Lys Ser Trp Pro Thr
                165                 170                 175

Ala Ser Gln Leu Gln Gly Lys Val Leu Leu Val Leu Asn His Ser Glu
            180                 185                 190

Asn Gln Lys Leu Ser Gln Tyr Ala Glu Ala Arg Thr Ser Ser Ala Lys
        195                 200                 205

Val Phe Ile Ser Pro Val Thr Asn Gly Gln Asn Asp Val Ser Gly Lys
    210                 215                 220

Val Ser Gly Met Ser Gly Gln Ser Ser Gly Tyr Val Ala Met Asn Asn
225                 230                 235                 240

Met Ser Lys Gly Asp Lys Lys Trp Ala Ala Gln Ala Phe Ala Tyr Ser
                245                 250                 255

His Val Gly Arg Val Trp Gly Asp Asp Gly Val Ser Phe Ala Gln His
            260                 265                 270

Ile Ser Glu Lys Val Asn Leu Ser Ala Tyr Tyr Lys Phe Ala Glu Gln
        275                 280                 285

Asn Ser Gly Gly Tyr Arg Ile Arg Pro Phe
    290                 295
```

<210> 23
<211> 969
<212> DNA
<213> Pseudomonas protegens

<220>
<221> sig_peptide
<222> (1)..(75)

<400> 23

```
atgactcatc tttcccgttt cgtcccccgc gccctggcgc tgtcggcact cctgctcagc      60
ccggcggcgt tcagccagga aagcccggcg ttcatcgccc cggctacctg gaataccccg     120
ttcaacggca tcgcccaggt ggcctgtcac aactgctacg agaagcagta cgcaagcacc     180
tttagcagcg tgctcgacag tgtgcgcacc ctggaactgg acttctggga ccagcgcgat     240
gcggtgaccg gcggctcgcc ccggcactgg ttcgtgcggc acaaccccgg caccctgttc     300
cagtccggca acgacaacaa ttgcacgggc gatggcaccg gcaagaacga tctcgaagcc     360
tgcctcaacg acgtgaagaa ctggagcgac aaccaccccg ggcactttcc cattacggtg     420
atcctcgaca agaagcaggg ctggtcgaag gaaagctcgg ggcgcacgcc caaggatttc     480

gatgagctgg tgacccgggt cttccagggc aagctctata ccccccagga cctggccacg     540
cacatcggca gcagcgcggg cgccttgcag ggcaacctca agggcaagtc ctggcccacc     600
gccagccagc ttcagggcaa ggtcctgctg gtgctcaacc actcggaaaa ccagaagctc     660
tcgcagtacg ccgaggcccg gacttccagc gccaaggtgt tcatctcgcc ggtgaccaat     720
ggccagaacg atgtcagtgg caaggtcagt ggcatgtccg gccagtcgtc cggctacgtg     780
gccatgaaca acatgtccaa gggcgacaag aagtgggcgg cccaggcctt tgcctacagc     840
catgtgggcc gggtctgggg cgatgacggg gtgtcgttcg cccagcacat cagcgagaag     900
gtcaacctct cggcctacta caagttcgcc gagcagaacg ccggcggcta tcgcatccgg     960
ccgttctga                                                             969
```

<210> 24
<211> 322
<212> PRT
<213> Pseudomonas protegens

<220>
<221> SIGNAL
<222> (1)..(25)

<400> 24

```
Met Thr His Leu Ser Arg Phe Val Pro Arg Ala Leu Ala Leu Ser Ala
1               5                   10                  15

Leu Leu Leu Ser Pro Ala Ala Phe Ser Gln Glu Ser Pro Ala Phe Ile
            20                  25                  30

Ala Pro Ala Thr Trp Asn Thr Pro Phe Asn Gly Ile Ala Gln Val Ala
        35                  40                  45

Cys His Asn Cys Tyr Glu Lys Gln Tyr Ala Ser Thr Phe Ser Ser Val
    50                  55                  60

Leu Asp Ser Val Arg Thr Leu Glu Leu Asp Phe Trp Asp Gln Arg Asp
65                  70                  75                  80

Ala Val Thr Gly Gly Ser Pro Arg His Trp Phe Val Arg His Asn Pro
                85                  90                  95

Gly Thr Leu Phe Gln Ser Gly Asn Asp Asn Asn Cys Thr Gly Asp Gly
            100                 105                 110

Thr Gly Lys Asn Asp Leu Glu Ala Cys Leu Asn Asp Val Lys Asn Trp
        115                 120                 125
```

Ser Asp Asn His Pro Gly His Phe Pro Ile Thr Val Ile Leu Asp Lys
130 135 140

Lys Gln Gly Trp Ser Lys Glu Ser Ser Gly Arg Thr Pro Lys Asp Phe
145 150 155 160

Asp Glu Leu Val Thr Arg Val Phe Gln Gly Lys Leu Tyr Thr Pro Gln
165 170 175

Asp Leu Ala Thr His Ile Gly Ser Ser Ala Gly Ala Leu Gln Gly Asn
180 185 190

Leu Lys Gly Lys Ser Trp Pro Thr Ala Ser Gln Leu Gln Gly Lys Val
195 200 205

Leu Leu Val Leu Asn His Ser Glu Asn Gln Lys Leu Ser Gln Tyr Ala
210 215 220

Glu Ala Arg Thr Ser Ser Ala Lys Val Phe Ile Ser Pro Val Thr Asn
225 230 235 240

Gly Gln Asn Asp Val Ser Gly Lys Val Ser Gly Met Ser Gly Gln Ser
245 250 255

Ser Gly Tyr Val Ala Met Asn Asn Met Ser Lys Gly Asp Lys Lys Trp
260 265 270

Ala Ala Gln Ala Phe Ala Tyr Ser His Val Gly Arg Val Trp Gly Asp
275 280 285

Asp Gly Val Ser Phe Ala Gln His Ile Ser Glu Lys Val Asn Leu Ser
290 295 300

Ala Tyr Tyr Lys Phe Ala Glu Gln Asn Ala Gly Gly Tyr Arg Ile Arg
305 310 315 320

Pro Phe

<210> 25
<211> 837
<212> DNA
<213> Bacillus sp

<220>
<221> sig_peptide
<222> (1)..(99)

<400> 25

atgaaacatc atcgttttcg aacgaattta ttatcggctc tttcagtaag ttcaattgtc 60

atcacctcaa tcatcggctc cactcaaacc acgtacgctt ggtcggccga tgctccgcat 120

gatcccaatc agagcacaca cctgtttatc gtgaacggtg ctgtcaatct ggttgccaac 180

aatacggacc cgcagatcaa caagcccacc gcgctgttgc aacagtggcg ttctcaatgg 240

gagcaagggt tgtacgatgc cgatcatctg aacccttact atgactccgg cacttttatg 300

tcccattttt acgatccgga cacgcaaacg aactacgcag ggttgtcgta cccgacagca 360

cgccaaacag gggcaaaata ttttacgatt gcctcgaatg actatcaagc aggggatatg 420

tcggatgcat tttacaattt aggcttgtcc ctgcactatt tcacagatgt cacgatgccg 480

cttcatgccg gaaatatttc caatctcgat cacgaagcac ccggctacca tgcgaaactc 540

gaagcgtatg ccgaatcgat tcaaaatcaa gtgacgcctc cgactgccgg actctacaat 600

tgggtctctc cgaatgatcc ggaactctgg attcatcaag cggctgtgca agcgaaatcc 660

gtcttgccgc aagtatggaa cagtgatatc acgagttggt tctgggaggc ggctttcagc 720

aattactact cgcaacaatg gcacaacgca gtaaccactc cggtcctgaa tcagttgtcg 780

caagcggagg cagagaccgc cggatatatt gatctgttct tccgtgtaaa cggttag 837

<210> 26
<211> 278
<212> PRT
<213> Bacillus sp

<220>
<221> SIGNAL
<222> (1)..(33)

<400> 26

```
Met Lys His His Arg Phe Arg Thr Asn Leu Leu Ser Ala Leu Ser Val
1               5                   10                  15

Ser Ser Ile Val Ile Thr Ser Ile Ile Gly Ser Thr Gln Thr Thr Tyr
            20                  25                  30

Ala Trp Ser Ala Asp Ala Pro His Asp Pro Asn Gln Ser Thr His Leu
        35                  40                  45

Phe Ile Val Asn Gly Ala Val Asn Leu Val Ala Asn Asn Thr Asp Pro
    50                  55                  60

Gln Ile Asn Lys Pro Thr Ala Leu Leu Gln Gln Trp Arg Ser Gln Trp
65                  70                  75                  80

Glu Gln Gly Leu Tyr Asp Ala Asp His Leu Asn Pro Tyr Tyr Asp Ser
                85                  90                  95
```

Gly Thr Phe Met Ser His Phe Tyr Asp Pro Asp Thr Gln Thr Asn Tyr
100 105 110

Ala Gly Leu Ser Tyr Pro Thr Ala Arg Gln Thr Gly Ala Lys Tyr Phe
115 120 125

Thr Ile Ala Ser Asn Asp Tyr Gln Ala Gly Asp Met Ser Asp Ala Phe
130 135 140

Tyr Asn Leu Gly Leu Ser Leu His Tyr Phe Thr Asp Val Thr Met Pro
145 150 155 160

Leu His Ala Gly Asn Ile Ser Asn Leu Asp His Glu Ala Pro Gly Tyr
165 170 175

His Ala Lys Leu Glu Ala Tyr Ala Glu Ser Ile Gln Asn Gln Val Thr
180 185 190

Pro Pro Thr Ala Gly Leu Tyr Asn Trp Val Ser Pro Asn Asp Pro Glu
195 200 205

Leu Trp Ile His Gln Ala Ala Val Gln Ala Lys Ser Val Leu Pro Gln
210 215 220

Val Trp Asn Ser Asp Ile Thr Ser Trp Phe Trp Glu Ala Ala Phe Ser
225 230 235 240

Asn Tyr Tyr Ser Gln Gln Trp His Asn Ala Val Thr Thr Pro Val Leu
245 250 255

Asn Gln Leu Ser Gln Ala Glu Ala Glu Thr Ala Gly Tyr Ile Asp Leu
260 265 270

Phe Phe Arg Val Asn Gly
275

<210> 27
<211> 246
<212> PRT
<213> Artificial sequence

<220>
<223> Variant

<400> 27

Ala Trp Ser Ala Asp Ala Pro His Asp Pro Asn Gln Ser Thr His Leu
1 5 10 15

```
Phe Ile Val Asn Gly Ala Val Asn Leu Val Ala Asn Asn Thr Asp Pro
            20                  25                  30

Gln Ile Asn Lys Pro Thr Ala Leu Leu Gln Gln Trp Arg Ser Gln Trp
        35                  40                  45

Glu Gln Gly Leu Tyr Asp Ala Asp His Leu Asn Pro Tyr Tyr Asp Ser
    50                  55                  60

Gly Thr Phe Met Ser His Phe Tyr Asp Pro Asp Thr Gln Thr Asn Tyr
65                  70                  75                  80

Ala Gly Leu Ser Tyr Pro Thr Ala Arg Gln Thr Gly Ala Lys Tyr Phe
                85                  90                  95

Thr Ile Ala Ser Asn Asp Tyr Gln Ala Gly Asp Met Ser Asp Ala Phe
            100                 105                 110

Tyr Asn Leu Gly Leu Ser Leu His Tyr Phe Thr Asp Val Thr Met Pro
        115                 120                 125

Leu His Ala Gly Asn Ile Ser Asn Leu Asp His Glu Ala Pro Gly Tyr
    130                 135                 140

His Ala Lys Leu Glu Ala Tyr Ala Glu Ser Ile Gln Asn Gln Val Thr
145                 150                 155                 160

Pro Pro Thr Ala Gly Leu Tyr Asn Trp Val Ser Pro Asn Asp Pro Glu
                165                 170                 175

Leu Trp Ile His Gln Ala Ala Val Gln Ala Lys Ser Val Leu Pro Gln
            180                 185                 190

Val Trp Asn Ser Asp Ile Thr Ser Trp Phe Trp Glu Ala Ala Phe Ser
        195                 200                 205

Asn Tyr Tyr Ser Gln Gln Trp His Asn Ala Val Thr Thr Pro Val Leu
    210                 215                 220

Asn Gln Leu Ser Gln Ala Glu Ala Glu Thr Ala Gly Tyr Ile Asp Leu
225                 230                 235                 240

Phe Phe Arg Val Asn Gly
                245
```

<210> 28
<211> 852

<212> DNA
<213> Bacillus thuringiensis

<220>
<221> sig_peptide
<222> (1)..(72)

<400> 28

```
atgaaaaaga aagtacttgc tttagcagca gctattacat tagtagctcc tttacaaagc      60
gttgcatttg ctcatgaaaa tgatgggggga agtaaaataa aaatagttca ccgctggtct     120
gctgaagata aacataaaga aggcgtaaat tctcatttat ggattgtaaa ccgtgcgatt     180
gatattatgt ctcgcaatac aacacttgta aaacaagatc gagttgcaca attaaatgaa     240
tggcgtacag agttagagaa cggtatttat gctgctgact atgaaaatcc ttattatgat     300
aatagcacat ttgcttcaca tttctatgat ccagacaatg gaaaaacata tattccattt     360
gcaaagcagg caaaagaaac tggagctaaa tattttaaat tagcgggtga atcatacaaa     420
aataaagata tgaaacaagc attcttctat ttaggattat ctcttcatta tttaggagat     480
gtaaatcaac cgatgcatgc ggcaaacttt acaaatcttt cgtatccaca aggattccat     540
tctaaatatg aaaactttgt agatacgata aaagataatt ataaagtaac ggatggaaat     600
ggatattgga attggaaagg tacaaatcca gaagattgga tccatggagc ggcagtagtg     660
gcgaaacaag attactctgg aattgtaaat gataatacga aagattggtt cgtaaaagca     720
gctgtgtcac aagaatatgc agataaatgg cgtgctgaag ttacaccgat gacaggtaag     780
cgattaatgg atgcacaacg tgttactgct ggatacattc agctttggtt tgatacgtac     840
ggagatcgtt aa                                                          852
```

<210> 29
<211> 283
<212> PRT
<213> Bacillus thuringiensis

<220>
<221> SIGNAL
<222> (1)..(24)

<220>
<221> PROPEP
<222> (25)..(38)

<400> 29

```
Met Lys Lys Lys Val Leu Ala Leu Ala Ala Ala Ile Thr Leu Val Ala
1               5                   10                  15

Pro Leu Gln Ser Val Ala Phe Ala His Glu Asn Asp Gly Gly Ser Lys
            20                  25                  30
```

Ile Lys Ile Val His Arg Trp Ser Ala Glu Asp Lys His Lys Glu Gly
35 40 45

Val Asn Ser His Leu Trp Ile Val Asn Arg Ala Ile Asp Ile Met Ser
50 55 60

Arg Asn Thr Thr Leu Val Lys Gln Asp Arg Val Ala Gln Leu Asn Glu
65 70 75 80

Trp Arg Thr Glu Leu Glu Asn Gly Ile Tyr Ala Ala Asp Tyr Glu Asn
85 90 95

Pro Tyr Tyr Asp Asn Ser Thr Phe Ala Ser His Phe Tyr Asp Pro Asp
100 105 110

Asn Gly Lys Thr Tyr Ile Pro Phe Ala Lys Gln Ala Lys Glu Thr Gly
115 120 125

Ala Lys Tyr Phe Lys Leu Ala Gly Glu Ser Tyr Lys Asn Lys Asp Met
130 135 140

Lys Gln Ala Phe Phe Tyr Leu Gly Leu Ser Leu His Tyr Leu Gly Asp
145 150 155 160

Val Asn Gln Pro Met His Ala Ala Asn Phe Thr Asn Leu Ser Tyr Pro
165 170 175

Gln Gly Phe His Ser Lys Tyr Glu Asn Phe Val Asp Thr Ile Lys Asp
180 185 190

Asn Tyr Lys Val Thr Asp Gly Asn Gly Tyr Trp Asn Trp Lys Gly Thr
195 200 205

Asn Pro Glu Asp Trp Ile His Gly Ala Ala Val Val Ala Lys Gln Asp
210 215 220

Tyr Ser Gly Ile Val Asn Asp Asn Thr Lys Asp Trp Phe Val Lys Ala
225 230 235 240

Ala Val Ser Gln Glu Tyr Ala Asp Lys Trp Arg Ala Glu Val Thr Pro
245 250 255

Met Thr Gly Lys Arg Leu Met Asp Ala Gln Arg Val Thr Ala Gly Tyr
260 265 270

Ile Gln Leu Trp Phe Asp Thr Tyr Gly Asp Arg
275 280

<210> 30
<211> 852
<212> DNA
<213> Bacillus pseudomycoides

<220>
<221> sig_peptide
<222> (1)..(72)

<400> 30

```
atgaaaaaga aagtattagc cttagcagca gctattacat tagtagcacc attgcaaagt      60
gtagcgtttg cccatgaaaa tgagggcgga aataaggtaa gagtaattca atattggtct     120
gctgaagata aacatgcaga aggtgtaaac tcccatttat ggattgtcaa tcgtgcaatt     180
gatattatgt ctcgtaatac aacggttgta aaacaagatc aagttgcagt attaaatgaa     240
tggcgtacag agttagagaa tggtatatat gctgctgatt atgaaaaccc ttactatgat     300
aacagtacat ttgcttctca tttctacgag cctgatacag ggaagacata tataccttt     360
gctaagcagg caaaagaaac tggggctaaa tattttaaac ttgctggtga agcttatcaa     420
aagcaagata tgaaacaagc attcttctat ttgggtttat cccttcatta cttaggcgat     480
gtcaatcaac cgatgcatgc agcaaacttt acaaatcttt cttatccaca aggtttccac     540
tccaaatatg aaaattttgt agatacaata aaaaataatt ataaagtggc tgatggaaat     600
ggatattgga attggaaagg agtaaatcct gaagactgga ttcatggagc ggctgtagct     660
gctaagcaag attatgctgg tattgtaaat ggcactacaa aagattggtt cgtaagagcg     720
gcagtttcac aagaatatgc agataaatgg cgtgcagaag ttacactaac aacaggaaaa     780
cgtttagtag aagcacagcg tgtcacagcg ggatatattc agctttggtt tgatacgtat     840
gtaaatcgct ag                                                         852
```

<210> 31
<211> 283
<212> PRT
<213> Bacillus pseudomycoides

<220>
<221> SIGNAL
<222> (1)..(24)

<220>
<221> PROPEP
<222> (25)..(38)

<400> 31

```
Met Lys Lys Lys Val Leu Ala Leu Ala Ala Ala Ile Thr Leu Val Ala
1               5                   10                  15
```

Pro Leu Gln Ser Val Ala Phe Ala His Glu Asn Glu Gly Gly Asn Lys
20 25 30

Val Arg Val Ile Gln Tyr Trp Ser Ala Glu Asp Lys His Ala Glu Gly
35 40 45

Val Asn Ser His Leu Trp Ile Val Asn Arg Ala Ile Asp Ile Met Ser
50 55 60

Arg Asn Thr Thr Val Val Lys Gln Asp Gln Val Ala Val Leu Asn Glu
65 70 75 80

Trp Arg Thr Glu Leu Glu Asn Gly Ile Tyr Ala Ala Asp Tyr Glu Asn
85 90 95

Pro Tyr Tyr Asp Asn Ser Thr Phe Ala Ser His Phe Tyr Glu Pro Asp
100 105 110

Thr Gly Lys Thr Tyr Ile Pro Phe Ala Lys Gln Ala Lys Glu Thr Gly
115 120 125

Ala Lys Tyr Phe Lys Leu Ala Gly Glu Ala Tyr Gln Lys Gln Asp Met
130 135 140

Lys Gln Ala Phe Phe Tyr Leu Gly Leu Ser Leu His Tyr Leu Gly Asp
145 150 155 160

Val Asn Gln Pro Met His Ala Ala Asn Phe Thr Asn Leu Ser Tyr Pro
165 170 175

Gln Gly Phe His Ser Lys Tyr Glu Asn Phe Val Asp Thr Ile Lys Asn
180 185 190

Asn Tyr Lys Val Ala Asp Gly Asn Gly Tyr Trp Asn Trp Lys Gly Val
195 200 205

Asn Pro Glu Asp Trp Ile His Gly Ala Ala Val Ala Ala Lys Gln Asp
210 215 220

Tyr Ala Gly Ile Val Asn Gly Thr Thr Lys Asp Trp Phe Val Arg Ala
225 230 235 240

Ala Val Ser Gln Glu Tyr Ala Asp Lys Trp Arg Ala Glu Val Thr Leu
245 250 255

Thr Thr Gly Lys Arg Leu Val Glu Ala Gln Arg Val Thr Ala Gly Tyr
260 265 270

```
Ile Gln Leu Trp Phe Asp Thr Tyr Val Asn Arg
            275                 280
```

<210> 32
<211> 260
<212> PRT
<213> Artificial sequence

<220>
<223> Variant

<400> 32

```
Ala His Glu Asn Glu Gly Gly Asn Lys Val Arg Val Ile Gln Tyr Trp
1               5                   10                  15

Ser Ala Glu Asp Lys His Ala Glu Gly Val Asn Ser His Leu Trp Ile
            20                  25                  30

Val Asn Arg Ala Ile Asp Ile Met Ser Arg Asn Thr Thr Val Val Lys
        35                  40                  45

Gln Asp Gln Val Ala Val Leu Asn Glu Trp Arg Thr Glu Leu Glu Asn
    50                  55                  60

Gly Ile Tyr Ala Ala Asp Tyr Glu Asn Pro Tyr Tyr Asp Asn Ser Thr
65                  70                  75                  80

Phe Ala Ser His Phe Tyr Glu Pro Asp Thr Gly Lys Thr Tyr Ile Pro
                85                  90                  95

Phe Ala Lys Gln Ala Lys Glu Thr Gly Ala Lys Tyr Phe Lys Leu Ala
            100                 105                 110

Gly Glu Ala Tyr Gln Lys Gln Asp Met Lys Gln Ala Phe Phe Tyr Leu
        115                 120                 125

Gly Leu Ser Leu His Tyr Leu Gly Asp Val Asn Gln Pro Met His Ala
    130                 135                 140

Ala Asn Phe Thr Asn Leu Ser Tyr Pro Gln Gly Phe His Ser Lys Tyr
145                 150                 155                 160

Glu Asn Phe Val Asp Thr Ile Lys Asn Asn Tyr Lys Val Ala Asp Gly
                165                 170                 175

Asn Gly Tyr Trp Asn Trp Lys Gly Val Asn Pro Glu Asp Trp Ile His
            180                 185                 190
```

```
Gly Ala Ala Val Ala Ala Lys Gln Asp Tyr Ala Gly Ile Val Asn Gly
        195                 200                 205

Thr Thr Lys Asp Trp Phe Val Arg Ala Ala Val Ser Gln Glu Tyr Ala
    210                 215                 220

Asp Lys Trp Arg Ala Glu Val Thr Leu Thr Thr Gly Lys Arg Leu Val
225                 230                 235                 240

Glu Ala Gln Arg Val Thr Ala Gly Tyr Ile Gln Leu Trp Phe Asp Thr
                245                 250                 255

Tyr Val Asn Arg
            260
```

<210> 33
<211> 852
<212> DNA
<213> Bacillus mycoides

<220>
<221> sig_peptide
<222> (1)..(72)

<400> 33

```
atgaaaaaga aagtattagc cttagcagca gctattacat tagtagcacc attgcaaagt      60
gtagcgtttg cccatgaaaa tgagggcgga aataaggtaa gagtaattca atattggtct     120
gctgaagata aacatgcaga aggtgtaaac tcccatttat ggattgtcaa tcgtgcaatt     180
gatattatgt ctcgtaatac aacggttgta aaacaagatc aagttgcatt attaaatgaa     240
tggcgtacag agttagagaa tggtatatat gctgctgatt atgaaaaccc ttattatgat     300
aacagtacat ttgcttctca tttctacgat cctgacacag ggaagacata tataccttt      360
gctaagcagg caaaagaaac tggggctaaa tattttaaac ttgctggtga agcctatcaa     420
aagcaagaaa taaaacaagc attcttctat ttaggcttat cgcttcatta cttaggagat     480
gtaaatcaac caatgcatgc agcaaacttt acaaatcttt cttatccaca aggtttccac     540
tccaaatatg aaaattttgt agatacaata aaaaataatt ataaagtggc tgatggaaat     600
ggatattgga attggaaagg agtaaatcct gaagactgga ttcatggagc ggctgtagct     660
gctaagcaag attatgctgg tattgtaaat ggcactacaa aagattggtt cgtaagagcg     720
gcagtttcac aagaatatgc agataaatgg cgtgcagaag ttacactaac aacaggaaaa     780
cgtttagtag aagcacagcg tgtcacagcg ggatatattc agctttggtt tgatacatat     840
gtaaatcgct ag                                                        852
```

<210> 34
<211> 283
<212> PRT
<213> Bacillus mycoides

<220>
<221> SIGNAL
<222> (1)..(24)

<400> 34

```
Met Lys Lys Lys Val Leu Ala Leu Ala Ala Ala Ile Thr Leu Val Ala
1               5                   10                  15

Pro Leu Gln Ser Val Ala Phe Ala His Glu Asn Glu Gly Gly Asn Lys
            20                  25                  30

Val Arg Val Ile Gln Tyr Trp Ser Ala Glu Asp Lys His Ala Glu Gly
        35                  40                  45

Val Asn Ser His Leu Trp Ile Val Asn Arg Ala Ile Asp Ile Met Ser
    50                  55                  60

Arg Asn Thr Thr Val Val Lys Gln Asp Gln Val Ala Leu Leu Asn Glu
65                  70                  75                  80

Trp Arg Thr Glu Leu Glu Asn Gly Ile Tyr Ala Ala Asp Tyr Glu Asn
                85                  90                  95

Pro Tyr Tyr Asp Asn Ser Thr Phe Ala Ser His Phe Tyr Asp Pro Asp
            100                 105                 110

Thr Gly Lys Thr Tyr Ile Pro Phe Ala Lys Gln Ala Lys Glu Thr Gly
        115                 120                 125

Ala Lys Tyr Phe Lys Leu Ala Gly Glu Ala Tyr Gln Lys Gln Glu Ile
    130                 135                 140

Lys Gln Ala Phe Phe Tyr Leu Gly Leu Ser Leu His Tyr Leu Gly Asp
145                 150                 155                 160

Val Asn Gln Pro Met His Ala Ala Asn Phe Thr Asn Leu Ser Tyr Pro
                165                 170                 175

Gln Gly Phe His Ser Lys Tyr Glu Asn Phe Val Asp Thr Ile Lys Asn
            180                 185                 190

Asn Tyr Lys Val Ala Asp Gly Asn Gly Tyr Trp Asn Trp Lys Gly Val
        195                 200                 205
```

```
Asn Pro Glu Asp Trp Ile His Gly Ala Ala Val Ala Ala Lys Gln Asp
    210                 215                 220

Tyr Ala Gly Ile Val Asn Gly Thr Thr Lys Asp Trp Phe Val Arg Ala
225                 230                 235                 240

Ala Val Ser Gln Glu Tyr Ala Asp Lys Trp Arg Ala Glu Val Thr Leu
                245                 250                 255

Thr Thr Gly Lys Arg Leu Val Glu Ala Gln Arg Val Thr Ala Gly Tyr
            260                 265                 270

Ile Gln Leu Trp Phe Asp Thr Tyr Val Asn Arg
        275                 280
```

<210> 35
<211> 870
<212> DNA
<213> Listeria innocua

<220>
<221> sig_peptide
<222> (1)..(81)

<400> 35

```
atgaaattca aaaaggtagt tctaggtatg tgtttgactg caagtgttct agtctttccg      60
gtaacgataa aagcaagtgc ctgttgcgat gaatatttac aagcacccgc agctccgcat     120
gatatcgaca gtaaattacc acataaactt agttggtccg cggataaccc gacaaatact     180
gacgtaaata cacactattg gctttttaaa caagctgaaa aaatactagc taaagatgta     240
aatcatatac gagctaattt aatgaatgag cttaaaaatt tcgataaaca aattgctcaa     300
ggtatatatg atgcggatca taaaaatcca tattatgata ctagtacatt tttatctcat     360
ttttataatc ctgatagaga taatacttat ttgccgggtt ttgctaatgc gaaaataact     420
ggagccaagt atttcaatca atcggtggct gattatcgag aaggtaaatt tgacacagca     480
ttttataaat taggtctagc aatccattat tatacggata ttagtcaacc tatgcacgcc     540
aataatttta ccgcaatatc atacccacca ggctaccact gcgcatatga aaattatgtg     600
gatactatta aacacaatta tcaagcaaca gaagacatgg tagtgaaaag attttgctca     660
gatgacgtga aagtctggct ctatgaaaat gcgaaaagag caaaagccga ctaccctaaa     720
atagtcaatg caaaaactaa aaaatcatat ttagtaggaa attccaaatg gaaaaaggat     780
acagtggaac ctactggagc tagactaaga gattcacagc aaactttggc aggcttttta     840
gaattttggt ccaaaaaaac aaatgaataa                                        870
```

<210> 36
<211> 289
<212> PRT
<213> Listeria innocua

<220>
<221> SIGNAL
<222> (1)..(27)

<400> 36

Met Lys Phe Lys Lys Val Val Leu Gly Met Cys Leu Thr Ala Ser Val
1               5                   10                  15

Leu Val Phe Pro Val Thr Ile Lys Ala Ser Ala Cys Cys Asp Glu Tyr
            20                  25                  30

Leu Gln Ala Pro Ala Ala Pro His Asp Ile Asp Ser Lys Leu Pro His
        35                  40                  45

Lys Leu Ser Trp Ser Ala Asp Asn Pro Thr Asn Thr Asp Val Asn Thr
    50                  55                  60

His Tyr Trp Leu Phe Lys Gln Ala Glu Lys Ile Leu Ala Lys Asp Val
65                  70                  75                  80

Asn His Ile Arg Ala Asn Leu Met Asn Glu Leu Lys Asn Phe Asp Lys
                85                  90                  95

Gln Ile Ala Gln Gly Ile Tyr Asp Ala Asp His Lys Asn Pro Tyr Tyr
            100                 105                 110

Asp Thr Ser Thr Phe Leu Ser His Phe Tyr Asn Pro Asp Arg Asp Asn
        115                 120                 125

Thr Tyr Leu Pro Gly Phe Ala Asn Ala Lys Ile Thr Gly Ala Lys Tyr
    130                 135                 140

Phe Asn Gln Ser Val Ala Asp Tyr Arg Glu Gly Lys Phe Asp Thr Ala
145                 150                 155                 160

Phe Tyr Lys Leu Gly Leu Ala Ile His Tyr Tyr Thr Asp Ile Ser Gln
                165                 170                 175

Pro Met His Ala Asn Asn Phe Thr Ala Ile Ser Tyr Pro Pro Gly Tyr
            180                 185                 190

His Cys Ala Tyr Glu Asn Tyr Val Asp Thr Ile Lys His Asn Tyr Gln
        195                 200                 205

Ala Thr Glu Asp Met Val Val Lys Arg Phe Cys Ser Asp Asp Val Lys
210 215 220

Val Trp Leu Tyr Glu Asn Ala Lys Arg Ala Lys Ala Asp Tyr Pro Lys
225 230 235 240

Ile Val Asn Ala Lys Thr Lys Lys Ser Tyr Leu Val Gly Asn Ser Lys
245 250 255

Trp Lys Lys Asp Thr Val Glu Pro Thr Gly Ala Arg Leu Arg Asp Ser
260 265 270

Gln Gln Thr Leu Ala Gly Phe Leu Glu Phe Trp Ser Lys Lys Thr Asn
275 280 285

Glu

<210> 37
<211> 263
<212> PRT
<213> Artificial sequence

<220>
<223> Variant

<400> 37

Ala Cys Cys Asp Glu Tyr Leu Gln Ala Pro Ala Ala Pro His Asp Ile
1 5 10 15

Asp Ser Lys Leu Pro His Lys Leu Ser Trp Ser Ala Asp Asn Pro Thr
20 25 30

Asn Thr Asp Val Asn Thr His Tyr Trp Leu Phe Lys Gln Ala Glu Lys
35 40 45

Ile Leu Ala Lys Asp Val Asn His Ile Arg Ala Asn Leu Met Asn Glu
50 55 60

Leu Lys Asn Phe Asp Lys Gln Ile Ala Gln Gly Ile Tyr Asp Ala Asp
65 70 75 80

His Lys Asn Pro Tyr Tyr Asp Thr Ser Thr Phe Leu Ser His Phe Tyr
85 90 95

Asn Pro Asp Arg Asp Asn Thr Tyr Leu Pro Gly Phe Ala Asn Ala Lys
100 105 110

```
Ile Thr Gly Ala Lys Tyr Phe Asn Gln Ser Val Ala Asp Tyr Arg Glu
        115                 120                 125

Gly Lys Phe Asp Thr Ala Phe Tyr Lys Leu Gly Leu Ala Ile His Tyr
    130                 135                 140

Tyr Thr Asp Ile Ser Gln Pro Met His Ala Asn Asn Phe Thr Ala Ile
145                 150                 155                 160

Ser Tyr Pro Pro Gly Tyr His Cys Ala Tyr Glu Asn Tyr Val Asp Thr
                165                 170                 175

Ile Lys His Asn Tyr Gln Ala Thr Glu Asp Met Val Val Lys Arg Phe
            180                 185                 190

Cys Ser Asp Asp Val Lys Val Trp Leu Tyr Glu Asn Ala Lys Arg Ala
        195                 200                 205

Lys Ala Asp Tyr Pro Lys Ile Val Asn Ala Lys Thr Lys Lys Ser Tyr
    210                 215                 220

Leu Val Gly Asn Ser Lys Trp Lys Lys Asp Thr Val Glu Pro Thr Gly
225                 230                 235                 240

Ala Arg Leu Arg Asp Ser Gln Gln Thr Leu Ala Gly Phe Leu Glu Phe
                245                 250                 255

Trp Ser Lys Lys Thr Asn Glu
            260
```

<210> 38
<211> 849
<212> DNA
<213> Artificial sequence

<220>
<223> Variant

<400> 38

```
atgaaaaaga aagtattagc actagcagct atggttgctt tagctgcgcc agttcaaagt      60
gtagtatttg cacaaacaaa taatagtgaa agtcctgcac cgattttaag atggtcagct     120
gaggataagc ataatgaggg gattaactct catttgtgga ttgtaaatcg tgcaattgac     180
atcatgtctc gtaatacaac gattgtgaat ccgaatgaaa ctgcattatt aaatgagtgg     240
cgtgctgatt tagaaaatgg tatttattct gctgattacg agaatcctta ttatgatgat     300
agtacatatg cttctcactt ttatgatccg gatactggaa caacatatat tcctttttgcg    360
aaacatgcaa aagaaacagg cgcaaaatat tttaaccttg ctggtcaagc ataccaaaat     420

caagatatgc agcaagcatt cttctactta ggattatcgc ttcattattt aggagatgtg     480
aatcagccaa tgcatgcagc atcttttacg gatctttctt atccaatggg tttccattct     540
aaatacgaaa attttgttga tacaataaaa aataactata ttgtttcaga tagcaatgga     600
tattggaatt ggaaaggagc aaacccagaa gattggattg aaggagcagc ggtagcagct     660
aaacaagatt atcctggcgt tgtgaacgat acgacaaaag attggtttgt aaaagcagcc     720
gtatctcaag aatatgcaga taaatggcgt gcggaagtaa caccggtgac aggaaagcgt     780
ttaatggaag cgcagcgcgt tacagctggt tatattcatt tgtggtttga tacgtatgta     840
aatcgctaa                                                             849
```

<210> 39
<211> 282
<212> PRT
<213> Artificial sequence

<220>
<223> Variant

<400> 39

```
Met Lys Lys Lys Val Leu Ala Leu Ala Ala Met Val Ala Leu Ala Ala
1               5                   10                  15

Pro Val Gln Ser Val Val Phe Ala Gln Thr Asn Asn Ser Glu Ser Pro
            20                  25                  30

Ala Pro Ile Leu Arg Trp Ser Ala Glu Asp Lys His Asn Glu Gly Ile
        35                  40                  45

Asn Ser His Leu Trp Ile Val Asn Arg Ala Ile Asp Ile Met Ser Arg
    50                  55                  60

Asn Thr Thr Ile Val Asn Pro Asn Glu Thr Ala Leu Leu Asn Glu Trp
65                  70                  75                  80

Arg Ala Asp Leu Glu Asn Gly Ile Tyr Ser Ala Asp Tyr Glu Asn Pro
                85                  90                  95

Tyr Tyr Asp Asp Ser Thr Tyr Ala Ser His Phe Tyr Asp Pro Asp Thr
            100                 105                 110

Gly Thr Thr Tyr Ile Pro Phe Ala Lys His Ala Lys Glu Thr Gly Ala
        115                 120                 125

Lys Tyr Phe Asn Leu Ala Gly Gln Ala Tyr Gln Asn Gln Asp Met Gln
    130                 135                 140
```

```
Gln Ala Phe Phe Tyr Leu Gly Leu Ser Leu His Tyr Leu Gly Asp Val
145                 150                 155                 160

Asn Gln Pro Met His Ala Ala Ser Phe Thr Asp Leu Ser Tyr Pro Met
                165                 170                 175

Gly Phe His Ser Lys Tyr Glu Asn Phe Val Asp Thr Ile Lys Asn Asn
            180                 185                 190

Tyr Ile Val Ser Asp Ser Asn Gly Tyr Trp Asn Trp Lys Gly Ala Asn
        195                 200                 205

Pro Glu Asp Trp Ile Glu Gly Ala Ala Val Ala Ala Lys Gln Asp Tyr
    210                 215                 220

Pro Gly Val Val Asn Asp Thr Thr Lys Asp Trp Phe Val Lys Ala Ala
225                 230                 235                 240

Val Ser Gln Glu Tyr Ala Asp Lys Trp Arg Ala Glu Val Thr Pro Val
                245                 250                 255

Thr Gly Lys Arg Leu Met Glu Ala Gln Arg Val Thr Ala Gly Tyr Ile
            260                 265                 270

His Leu Trp Phe Asp Thr Tyr Val Asn Arg
        275                 280
```

<210> 40
<211> 987
<212> DNA
<213> Unknown

<220>
<223> DNA obtained from an environmental sample

<400> 40

```
atgaacaata agaagtttat tttgaagtta ttcatatgta gtatggtact tagcgccttt      60
gtatttgctt tcaatgataa gaaaaccgtt gcagctagct ctattaatgt gcttgaaaat     120
tggtctagat ggatgaaacc tataaatgat gacataccgt tagcacgaat ttcaattcca     180
ggaacacatg atagtggaac gttcaagttg caaaatccga taaagcaagt gtggggaatg     240
acgcaagaat atgattttcg ttatcaaatg gatcatggag ctagaatttt tgatataaga     300
gggcgtttaa cagatgataa tacgatagtt cttcatcatg ggccattata tctttatgta     360
acactgcacg aatttataaa cgaagcgaaa caatttttaa aagataatcc aagtgaaacg     420
attattatgt ctttaaaaaa agagtatgag gatatgaaag gggcggaaag ctcatttagt     480
agtacgtttg agaaaaatta ttttcgtgat ccaatctttt taaaaacaga agggaatata     540

aagcttggag atgctcgtgg gaaaattgta ttactaaaaa gatatagtgg tagtaatgaa     600
tctgggggat ataataattt ctattggcca gacaatgaga cgtttacctc aactataaat     660
caaaatgtaa atgtaacagt acaagataaa tataaagtga gttatgatga gaaaataaac     720
gctattaaag atacattaaa tgaaacgatt aacaatagtg aagatgttaa tcatctatat     780
attaatttta caagcttgtc ttctggtggt acagcatgga atagtccata ttattatgcg     840
tcctacataa atcctgaaat tgcaaattat atgaagcaaa agaatcctac gagagtgggc     900
tggataatac aagattatat aaatgaaaaa tggtcaccat tactttatca agaagttata     960
agagcgaata agtcacttgt aaaatag                                         987
```

<210> 41
<211> 328
<212> PRT
<213> Unknown

<220>
<223> protein obtained from an environmental sample

<220>
<221> SIGNAL
<222> (1)..(23)

<220>
<221> DOMAIN
<222> (56)..(195)
<223> Phosphatidylinositol-specific phospholipase C, X domain

<400> 41

```
Met Asn Asn Lys Lys Phe Ile Leu Lys Leu Phe Ile Cys Ser Met Val
1               5                   10                  15

Leu Ser Ala Phe Val Phe Ala Phe Asn Asp Lys Lys Thr Val Ala Ala
            20                  25                  30

Ser Ser Ile Asn Val Leu Glu Asn Trp Ser Arg Trp Met Lys Pro Ile
        35                  40                  45

Asn Asp Asp Ile Pro Leu Ala Arg Ile Ser Ile Pro Gly Thr His Asp
    50                  55                  60

Ser Gly Thr Phe Lys Leu Gln Asn Pro Ile Lys Gln Val Trp Gly Met
65                  70                  75                  80

Thr Gln Glu Tyr Asp Phe Arg Tyr Gln Met Asp His Gly Ala Arg Ile
                85                  90                  95

Phe Asp Ile Arg Gly Arg Leu Thr Asp Asp Asn Thr Ile Val Leu His
            100                 105                 110
```

His Gly Pro Leu Tyr Leu Tyr Val Thr Leu His Glu Phe Ile Asn Glu
115 120 125

Ala Lys Gln Phe Leu Lys Asp Asn Pro Ser Glu Thr Ile Ile Met Ser
130 135 140

Leu Lys Lys Glu Tyr Glu Asp Met Lys Gly Ala Glu Ser Ser Phe Ser
145 150 155 160

Ser Thr Phe Glu Lys Asn Tyr Phe Arg Asp Pro Ile Phe Leu Lys Thr
165 170 175

Glu Gly Asn Ile Lys Leu Gly Asp Ala Arg Gly Lys Ile Val Leu Leu
180 185 190

Lys Arg Tyr Ser Gly Ser Asn Glu Ser Gly Gly Tyr Asn Asn Phe Tyr
195 200 205

Trp Pro Asp Asn Glu Thr Phe Thr Ser Thr Ile Asn Gln Asn Val Asn
210 215 220

Val Thr Val Gln Asp Lys Tyr Lys Val Ser Tyr Asp Glu Lys Ile Asn
225 230 235 240

Ala Ile Lys Asp Thr Leu Asn Glu Thr Ile Asn Asn Ser Glu Asp Val
245 250 255

Asn His Leu Tyr Ile Asn Phe Thr Ser Leu Ser Ser Gly Gly Thr Ala
260 265 270

Trp Asn Ser Pro Tyr Tyr Tyr Ala Ser Tyr Ile Asn Pro Glu Ile Ala
275 280 285

Asn Tyr Met Lys Gln Lys Asn Pro Thr Arg Val Gly Trp Ile Ile Gln
290 295 300

Asp Tyr Ile Asn Glu Lys Trp Ser Pro Leu Leu Tyr Gln Glu Val Ile
305 310 315 320

Arg Ala Asn Lys Ser Leu Val Lys
325

<210> 42
<211> 1020
<212> DNA
<213> Amycolatopsis azurea

<400> 42

```
atgaaactcg tccccgcggt atccctcgtc ggcgtgatct tggcggcttc gctgtccgga        60
acggtcgcga acgccgacgc cgccaaggtc tcccacgtga caacggtcgg cgtccacaac       120
acctacgaga ccggtgccta cgactacctg gcgcgctcgc tggacgccgg tacctcgctg       180
atcgaactcg acgtctggcc gaacatcatc actcgcgagt ggaaagtgag tcactcgaac       240
ccgttgggga acaacaacaa ctgcgtcgcg gcgagtacgc cgtcgcagtt gtactccggt       300
gggcggaaca agaacctcga acactgcctc gatgacatcc gcgtctggct gggcgcgcat       360
ccggacagca aaccggtcac gctcaaactg gagatgaaga ccgggttcgc cgacaaccgc       420
ggcctcggcc cggacgaact cgacgcgtcc atccgggcac atctgggcag tacggtgttc       480
cggccggcgg acctgctcgg cgggtacgcg acgctcgacg acgcggccaa agcggacaac       540
tggccgtccc tggacgcgct gcggggcaag gtgatcatcg agatcatccc cggcacggtc       600
gaagagggaa atccgacgga cacgctcaag accgacgtcg agtacgggcg atatctgcgg       660
tcgctcaagg acgcgggccg cgtcggcgag gtgcagatct tcccgacggt gcacggcgcg       720
gcacccggcg acccgcgcac gaagtacgcc gacgccgggc tgcggccgtg gttcgtggtc       780
ttcgacggcg acgccaccgc gttcctgacg cagaccgggc ccggctggta cgacgacaac       840
cactactacg tggtgatgac cgacgcgcac aacgtcgcgc cggccatcga ctcccgtgcc       900
cccacggtgg accaggcgag cgcgcgagcg gccctgctgg cgaagaacca cgcttcggtg       960
ctgacctcgg attggacggg gctgaccacc gtgctgccgc aggtacttcc gcgcggctag      1020
```

<210> 43
<211> 339
<212> PRT
<213> Amycolatopsis azurea

<220>
<221> SIGNAL
<222> (1)..(27)

<400> 43

```
Met Lys Leu Val Pro Ala Val Ser Leu Val Gly Val Ile Leu Ala Ala
1               5                   10                  15

Ser Leu Ser Gly Thr Val Ala Asn Ala Asp Ala Ala Lys Val Ser His
            20                  25                  30

Val Thr Thr Val Gly Val His Asn Thr Tyr Glu Thr Gly Ala Tyr Asp
        35                  40                  45

Tyr Leu Ala Arg Ser Leu Asp Ala Gly Thr Ser Leu Ile Glu Leu Asp
    50                  55                  60
```

Val Trp Pro Asn Ile Ile Thr Arg Glu Trp Lys Val Ser His Ser Asn
65 70 75 80

Pro Leu Gly Asn Asn Asn Asn Cys Val Ala Ala Ser Thr Pro Ser Gln
85 90 95

Leu Tyr Ser Gly Gly Arg Asn Lys Asn Leu Glu His Cys Leu Asp Asp
100 105 110

Ile Arg Val Trp Leu Gly Ala His Pro Asp Ser Lys Pro Val Thr Leu
115 120 125

Lys Leu Glu Met Lys Thr Gly Phe Ala Asp Asn Arg Gly Leu Gly Pro
130 135 140

Asp Glu Leu Asp Ala Ser Ile Arg Ala His Leu Gly Ser Thr Val Phe
145 150 155 160

Arg Pro Ala Asp Leu Leu Gly Gly Tyr Ala Thr Leu Asp Asp Ala Ala
165 170 175

Lys Ala Asp Asn Trp Pro Ser Leu Asp Ala Leu Arg Gly Lys Val Ile
180 185 190

Ile Glu Ile Ile Pro Gly Thr Val Glu Glu Gly Asn Pro Thr Asp Thr
195 200 205

Leu Lys Thr Asp Val Glu Tyr Gly Arg Tyr Leu Arg Ser Leu Lys Asp
210 215 220

Ala Gly Arg Val Gly Glu Val Gln Ile Phe Pro Thr Val His Gly Ala
225 230 235 240

Ala Pro Gly Asp Pro Arg Thr Lys Tyr Ala Asp Ala Gly Leu Arg Pro
245 250 255

Trp Phe Val Val Phe Asp Gly Asp Ala Thr Ala Phe Leu Thr Gln Thr
260 265 270

Gly Pro Gly Trp Tyr Asp Asp Asn His Tyr Tyr Val Val Met Thr Asp
275 280 285

Ala His Asn Val Ala Pro Ala Ile Asp Ser Arg Ala Pro Thr Val Asp
290 295 300

Gln Ala Ser Ala Arg Ala Ala Leu Leu Ala Lys Asn His Ala Ser Val
305 310 315 320

Leu Thr Ser Asp Trp Thr Gly Leu Thr Thr Val Leu Pro Gln Val Leu
325 330 335

Pro Arg Gly

<210> 44
<211> 771
<212> DNA
<213> Artificial sequence

<220>
<223> Synthetic gene

<400> 44

gaagatgacg cacaaccgcc tattacagca aaatggtcag cggaagatcc gcatcatgaa 60

gatacaaata cacatctgtg gattgttcgc catgccatgg agattatggc gaataacaaa 120

gatgttgtca aacctggcga agtcgaacaa ctgaaacaat ggcaatcaga tctggaacag 180

ggcatttatg atgcagatca tgcaaacccg tattatgata atgcaacatt tgcgagccat 240

ttttatgatc cggatacagg caaatcatat attccgctgg cagcacatgc aaaaacaaca 300

agcgttaaat actttaaaag agcaggcgaa gcgtatcaga aaggcgatca taaacaagcg 360

ttttacaatc tgggcctggc gctgcattat attggcgatc tgaatcaacc gatgcatgca 420

gcgaatttta caaatctgtc atatccgcaa ggctttcata gcaaatatga aaactatgtc 480

gatagcttta aagaagatta cgcggtcaaa gatggcgaag gctattggca ttggaaaggc 540

acaaatccgg aagattggct gcatggcaca gcagttgcag caaaaaaaga ttatccggat 600

atcgtcaacg atacaacgaa agcatggttt gttaaagcag cagtctcaaa tagctatgca 660

gcaaaatggc gtgcagcagt tgttccggca acaggcaaaa gactgacaga agcacaaaga 720

attctggcag gctatatgca actgtggttt gatacatatg tcaacaaata a 771

<210> 45
<211> 280
<212> PRT
<213> Bacillus macauensis

<220>
<221> SIGNAL
<222> (1)..(24)

<220>
<221> PROPEP
<222> (25)..(35)

<400> 45

Met Lys Lys Thr Phe Val Ala Val Ala Thr Ala Ala Leu Leu Val Thr

```
1               5                   10                  15

Gly Phe Gln Gly Asn Ala Ser Ala Glu Asp Asp Ala Gln Pro Pro Ile
            20                  25                  30

Thr Ala Lys Trp Ser Ala Glu Asp Pro His His Glu Asp Thr Asn Thr
        35                  40                  45

His Leu Trp Ile Val Arg His Ala Met Glu Ile Met Ala Asn Asn Lys
    50                  55                  60

Asp Val Val Lys Pro Gly Glu Val Glu Gln Leu Lys Gln Trp Gln Ser
65                  70                  75                  80

Asp Leu Glu Gln Gly Ile Tyr Asp Ala Asp His Ala Asn Pro Tyr Tyr
                85                  90                  95

Asp Asn Ala Thr Phe Ala Ser His Phe Tyr Asp Pro Asp Thr Gly Lys
            100                 105                 110

Ser Tyr Ile Pro Leu Ala Ala His Ala Lys Thr Thr Ser Val Lys Tyr
        115                 120                 125

Phe Lys Arg Ala Gly Glu Ala Tyr Gln Lys Gly Asp His Lys Gln Ala
    130                 135                 140

Phe Tyr Asn Leu Gly Leu Ala Leu His Tyr Ile Gly Asp Leu Asn Gln
145                 150                 155                 160

Pro Met His Ala Ala Asn Phe Thr Asn Leu Ser Tyr Pro Gln Gly Phe
                165                 170                 175

His Ser Lys Tyr Glu Asn Tyr Val Asp Ser Phe Lys Glu Asp Tyr Ala
            180                 185                 190

Val Lys Asp Gly Glu Gly Tyr Trp His Trp Lys Gly Thr Asn Pro Glu
        195                 200                 205

Asp Trp Leu His Gly Thr Ala Val Ala Ala Lys Lys Asp Tyr Pro Asp
    210                 215                 220

Ile Val Asn Asp Thr Thr Lys Ala Trp Phe Val Lys Ala Ala Val Ser
225                 230                 235                 240

Asn Ser Tyr Ala Ala Lys Trp Arg Ala Ala Val Val Pro Ala Thr Gly
                245                 250                 255
```

Lys Arg Leu Thr Glu Ala Gln Arg Ile Leu Ala Gly Tyr Met Gln Leu
260 265 270

Trp Phe Asp Thr Tyr Val Asn Lys
275 280

<210> 46
<211> 277
<212> PRT
<213> Talaromyces leycettanus

<400> 46

Ala Pro Ala Pro Val Leu Arg Arg Asp Val Ser Ser Ser Val Leu Ser
1 5 10 15

Glu Leu Asp Leu Phe Ala Gln Tyr Ser Ala Ala Ala Tyr Cys Ser Ser
20 25 30

Asn Ile Gly Ser Pro Gly Thr Lys Leu Thr Cys Ser Val Gly Asn Cys
35 40 45

Pro Arg Val Glu Ala Ala Asp Thr Glu Thr Leu Ile Glu Phe Asn Glu
50 55 60

Ser Ser Ser Phe Gly Asp Val Thr Gly Tyr Ile Ala Val Asp Arg Thr
65 70 75 80

Asn Ser Leu Leu Val Leu Ala Phe Arg Gly Ser Ser Thr Val Ser Asn
85 90 95

Trp Glu Ala Asp Leu Asp Phe Pro Leu Thr Asp Ala Ser Ser Leu Cys
100 105 110

Ser Gly Cys Glu Ile His Ser Gly Phe Trp Ala Ala Trp Gln Thr Val
115 120 125

Gln Ala Ser Ile Thr Ser Thr Leu Glu Ser Ala Ile Ala Ser Tyr Pro
130 135 140

Gly Tyr Thr Leu Val Phe Thr Gly His Ser Tyr Gly Ala Ala Leu Ala
145 150 155 160

Ala Ile Ala Ala Thr Thr Leu Arg Asn Ala Gly Tyr Thr Ile Gln Leu
165 170 175

Tyr Asp Tyr Gly Gln Pro Arg Leu Gly Asn Leu Ala Leu Ala Gln Tyr
180 185 190

Ile Thr Ala Gln Thr Gln Gly Ala Asn Tyr Arg Val Thr His Thr Asp
195 200 205

Asp Ile Val Pro Lys Leu Pro Pro Glu Leu Phe Gly Tyr His His Phe
210 215 220

Ser Pro Glu Tyr Trp Ile Thr Ser Gly Asp Asn Val Thr Val Thr Thr
225 230 235 240

Ser Asp Val Gln Val Val Thr Gly Ile Asp Ser Thr Ala Gly Asn Asp
245 250 255

Gly Thr Leu Leu Asp Ser Thr Ser Ala His Asp Trp Tyr Ile Val Tyr
260 265 270

Ile Asp Gly Cys Asp
275

<210> 47
<211> 277
<212> PRT
<213> Talaromyces leycettanus

<400> 47

Ala Pro Ala Pro Val Leu Arg Arg Asp Val Ser Ser Ser Val Leu Ser
1 5 10 15

Glu Leu Asp Leu Phe Ala Gln Tyr Ser Ala Ala Ala Tyr Cys Ser Ser
20 25 30

Asn Ile Gly Ser Pro Gly Thr Lys Leu Thr Cys Ser Val Gly Asn Cys
35 40 45

Pro Arg Val Glu Ala Ala Asp Thr Glu Thr Leu Ile Glu Phe Asn Glu
50 55 60

Ser Ser Ser Phe Gly Asp Val Thr Gly Tyr Ile Ala Val Asp Arg Thr
65 70 75 80

Asn Ser Leu Leu Val Leu Ala Phe Arg Gly Ser Ser Thr Val Ser Asn
85 90 95

Trp Glu Ala Asp Leu Asp Phe Pro Leu Thr Asp Ala Ser Ser Leu Cys
100 105 110

Ser Gly Cys Glu Ile His Ser Gly Phe Trp Ala Ala Trp Gln Thr Val
115 120 125

```
Gln Ala Ser Ile Thr Ser Thr Leu Glu Ser Ala Ile Ala Ser Tyr Pro
    130                 135                 140

Gly Tyr Thr Leu Val Phe Thr Gly His Ser Tyr Gly Ala Ala Leu Ala
145                 150                 155                 160

Ala Ile Ala Ala Thr Thr Leu Arg Asn Ala Gly Tyr Thr Ile Gln Leu
                165                 170                 175

Tyr Asp Tyr Gly Gln Pro Arg Leu Gly Asn Leu Ala Leu Ala Gln Tyr
            180                 185                 190

Ile Thr Ala Gln Thr Gln Gly Ala Asn Tyr Arg Val Thr His Thr Asp
        195                 200                 205

Asp Ile Val Pro Lys Leu Pro Pro Glu Leu Phe Gly Tyr His His Phe
    210                 215                 220

Ser Pro Glu Tyr Trp Ile Thr Ser Gly Asp Asn Val Thr Val Thr Thr
225                 230                 235                 240

Ser Asp Val Gln Val Val Thr Gly Ile Asp Ser Thr Ala Gly Asn Asp
                245                 250                 255

Gly Thr Leu Leu Asp Ser Thr Ser Ala His Asp Trp Tyr Ile Val Tyr
            260                 265                 270

Ile Asp Gly Cys Asp
        275
```

## Description

### Reference to a Sequence Listing

This application contains a Sequence Listing in computer readable form.

### Background of the Invention

### Field of the Invention

The present invention relates to compositions comprising a plurality of polypeptides having phospholipase C activity, wherein at least one of said polypeptides having phospholipase C activity is a polypeptide derived from a fungus, and at least one of said polypeptides having phospholipase C activity is a polypeptide derived from a bacterium.

The invention also relates to such compositions, for use in processes such as for degumming of an aqueous carbohydrate solution or slurry, and for degumming of oil.

The invention further relates to a process for hydrolysing a phospholipid or lysophospholipid, comprising contacting said phospholipid or lysophospholipid with a composition as defined above.

### Description of the Related Art

Several types of phospholipases are known which differ in their specificity according to the position of the bond attacked in the phospholipid molecule. Phospholipase A1 (PLA1) removes the 1-position fatty acid to produce free fatty acid and 1-lyso-2-acylphospholipid. Phospholipase A2 (PLA2) removes the 2-position fatty acid to produce free fatty acid and 1-acyl-2-lysophospholipid. The term phospholipase B (PLB) is used for phospholipases having both A1 and A2 activity. Phospholipase C (PLC) removes the phosphate moiety to produce 1,2 diacylglycerol and phosphate ester. Phospholipase D (PLD) produces 1,2-diacylglycero-phosphate and base group (See Figure 1).

Before consumption vegetable oils are degummed to provide refined storage stable vegetable oils of neutral taste and light color. The degumming process comprises removing the phospholipid components (the gum) from the triglyceride rich oil fraction. The most commonly used processes in the industry are water degumming, chemical/caustic refining and physical refining including acid assisted degumming and/or enzyme assisted degumming. Due to the emulsifying properties of the phospholipid components, the degumming procedure has resulted in a loss of oil i.e. of triglycerides.

Enzymatic degumming reduces the oils loss due to an efficient hydrolysis of the phospholipids which decrease the gum formation and reduce the oil entrained in the gum phase. For a review on enzymatic degumming see Dijkstra 2010 Eur. J. Lipid Sci. Technol. 112, 1178. The use of Phospholipase A and/or phospholipase C in degumming is for example described in Clausen 2001 Eur J Lipid Sci Techno 103 333-340, WO 2003/089620 and WO 2008/094847. Phospholipase A solutions generate lysophospholipid and free fatty acids resulting in oil loss. Phospholipase C on the other hand has the advantage that it produces diglyceride (Figure 2) which will remain in the oil and therefore will reduce losses. There are four major phospholipids in vegetable oil phosphatidylcholine (PC), phosphatidylethanolamine (PE), phosphatidic acid (PA) and phosphatidyl inositol (PI). Phospholipase C enzymes have different specificity towards these phospholipids. The only known commercially available phospholipase C is (f) PURIFINE® PLC of Verenium/DSM (Dijkstra, 101st AOCS Annual Meeting 10. May 2010) which has specificity towards PC and PE. WO07/059927 describes a thermostable *Bacillus* PLC for degumming. WO 2012/062817 describes a fungal PLC with specificity towards all four phospholipids. A PI-specific phospholipase C has been described in WO 2011/046815. Despite the availability of such phospholipases, there is a need to improve the efficacy of the enzymatic hydrolysis of phospholipids by decreasing the reaction time of the conversion into diacylglycerol and phosphate ester, while providing broad substrate specificity.

### Summary of the Invention

The present invention relates to a composition comprising a plurality of polypeptides having phospholipase C activity, wherein

- at least one of said polypeptides having phospholipase C activity is a polypeptide derived from a fungus; and
- at least one of said polypeptides having phospholipase C activity is a polypeptide derived from a bacterium.

The invention further provides such a composition, for use in

i) degumming of an aqueous carbohydrate solution or slurry, in degumming of oil, e.g. vegetable oil, or an edible vegetable oil, or in a process comprising hydrolysis of phospholipids in the gum fraction from water degumming to release entrapped triglyceride oil,

ii) a process comprising hydrolysis of phospholipids to obtain improved phospholipid emulsifiers, in particular wherein said phospholipid is lecithin,

iii) a process for improving the filterability of an aqueous solution or slurry of carbohydrate origin which contains phospholipid,

iv) a process for the extraction of oil,

v) a process for the production of an animal feed product,

vi) a process for the production of a biofuel, e.g. a biodiesel,

vii) in a process for the production of a detergent product, and/or

viii) in a process for making a baked product, comprising adding the phospholipase to a dough, and baking the dough to make the baked product.

Finally, the invention provides a process for hydrolysing a phospholipid or lysophospholipid, comprising contacting said phospholipid or lysophospholipid with a plurality of polypeptides having phospholipase C activity and/or a composition according to the invention.

## Brief Description of the Figures

Figure 1 illustrates where different phospholipases cleave a phospholipid as well as the four major functional groups on phospholipids.

Figure 2 illustrates the reaction of a phospholipid with a phospholipase C to form diglyceride and a phosphate ester or phosphoric acid.

## Definitions

**Phospholipase C activity:** The term "phospholipase C activity" or "PLC activity" relates to an enzymatic activity that removes the phosphate ester moiety from a phospholipid to produce a 1,2 diacylglycerol (see Figure 2). Most PLC enzymes belong to the family of hydrolases and phosphodiesterases and are generally classified as EC 3.1.4.3. Some PLC enzymes are classified in other EC classes, for example PI-specific PLC's. Phospholipase C activity may be determined according to the procedure described in Example 1 or by one of the assays described in the "Assay for phospholipase activity" section.

**Phospholipase A activity:** In the context of the present invention the term "phospholipase A activity" comprises enzymes having phospholipase A1 and/or phospholipase A2 activity (A1 or A2, EC 3.1.1.32 or EC 3.1.1.4), *i.e.,* hydrolytic activity towards one or both carboxylic ester bonds in phospholipids such as lecithin. A phospholipases having both A1 and A2 activity is also referred to as a phospholipase B.

Phospholipase A activity may be determined by one of the assays described in the "Assay for phospholipase activity" section. For purposes of the present invention, phospholipase A activity may also be determined using a LEU assay, in which the phospholipase A activity is determined from the ability to hydrolyze lecithin at pH 8.0, 40°C. The hydrolysis reaction can be followed by titration with NaOH for a reaction time of 2 minutes. The phospholipase from *Fusarium oxysporum* (LIPOPAN F) disclosed in WO 1998/26057 has an activity of 1540 LEU/mg enzyme protein and may be used as a standard.

In one aspect, the polypeptides of the present invention have at least 20%, e.g., at least 40%, at least 50%, at least 60%, at least 70%, at least 80%, at least 90%, at least 95%, or at least 100% of the phospholipase A activity of the mature polypeptide of SEQ ID NO: 46 and/or of the polypeptide of SEQ ID NO: 47.

In addition to having phospholipase A activity, the polypeptides of the present invention may also have lipase activity.

**Assays for phospholipase activity:** Routine protocols for determining the activity phospholipase A, C, are well known in the art.

Exemplary activity assays include turbidity assays, methylumbelliferyl phosphocholine (fluorescent) assays, Amplex red (fluorescent) phospholipase assays, thin layer chromatography assays (TLC), cytolytic assays and p-nitrophenylphosphorylcholineassays. Using these assays polypeptides, peptides or antibodies can be quickly screened for a phospholipase activity.

Plate assays with a substrate containing agar can be used to determine phospholipase activity. Useful substrates are lecithin or specific phospholipids. The assay can be conducted as follows. Plates are casted by mixing of 5 ml 2% Agarose (Litex HSA 1000) prepared by mixing and cooking in buffers (100 mM HEPES and 100 mM Citrate with pH adjusted from pH 3.0 to pH 7.0) for 5 minutes followed by cooling to approximately 60°C and 5ml substrate (L-alfa Phosohatidylcholine, 95% from Soy (Avanti 441601) or L-$\alpha$-phosphatidylinositol from Soy (Avanti 840044P) for PI-specificity or L-$\alpha$-phosphatidylethanolamine from Soy (Avanti 840024P) or lecithin) dispersed in water (MilliQ) at 60°C for 1 minute with Ultra Turrax for PC-specificity) gently mixed into petri dishes with diameter of 7 cm and cooled to room temperature before holes with a diameter of approximately 3 mm were punched by vacuum. Ten microliters of purified enzyme diluted to 0.4 mg/ml is added into each well before plates were sealed by parafilm and placed in an incubator at 55°C for 48 hours. Plates were taken out for photography at regular intervals.

Turbidity assays to determine phospholipase activity are described, e.g., in Kauffmann (2001) "Conversion of Bacillus thermocatenulatus lipase into an efficient phospholipase with increased activity towards long-chain fatty acyl substrates by directed evolution and rational design," Protein Engineering 14:919-928; Ibrahim (1995) "Evidence implicating phospholipase as a virulence factor of Candida albicans," Infect. Immun. 63:1993-1998.

Methylumbelliferyl (fluorescent) phosphocholine assays to determine phospholipase activity are described, e.g., in Goode (1997) "Evidence for cell surface internal phospholipase activity in ascidian eggs," Develop. Growth Differ. 39:655-660; Diaz (1999) "Direct fluorescence-based lipase activity assay," BioTechniques 27:696-700.

Amplex Red (fluorescent) Phospholipase Assays to determine phospholipase activity are available as kits, e.g., the detection of phosphatidylcholine-specific phospholipase using an Amplex Red phosphatidylcholine-specific phospholipase assay kit from Molecular Probes Inc. (Eugene, OR), according to manufacturer's instructions.

Fluorescence is measured in a fluorescence microplate reader using excitation at 560 $\pm$ 10nm and fluorescence detection at 590 $\pm$ 10 nm. The assay is sensitive at very low enzyme concentrations.

Thin layer chromatography assays (TLC) to determine phospholipase activity are described, e.g., in Reynolds (1991) Methods in Enzymol. 197:3-13; Taguchi (1975) "Phospholipase from Clostridium novyi type A.I," Biochim. Biophys. Acta 409:75-85. Thin layer chromatography (TLC) is a widely used technique for detection of phospholipase activity. Various modifications of this method have been used to extract the phospholipids from the aqueous assay mixtures. In some PLC assays the hydrolysis is stopped by addition of chloroform/methanol (2: 1) to the reaction mixture. The unreacted starting material and the diacylglycerol are extracted into the organic phase and may be fractionated by TLC, while the head group product remains in the aqueous phase. For more precise measurement of the phospholipid digestion, radio labeled substrates can be used (see, e.g., Reynolds (1991) Methods in Enzymol. 197:3-13). The ratios of products and reactants can be used to calculate the actual number of moles of substrate hydrolyzed per unit time. If all the components are extracted equally, any losses in the extraction will affect all components equally. Separation of phospholipid digestion products can be achieved by silica gel TLC with chloroform/methanol/water (65:25:4) used as a solvent system (see, e.g., Taguchi (1975) Biochim. Biophys. Acta 409:75-85).

p-Nitrophenylphosphorylcholine assays to determine phospholipase activity are described, e.g., in Korbsrisate (1999) J. Clin. Microbiol. 37:3742-3745; Berka (1981) Infect. Immun. 34:1071-1074. This assay is based on enzymatic hydrolysis of the substrate analog p-nitrophenylphosphorylcholine to liberate a yellow chromogenic compound p-nitrophenol, detectable at 405 nm. This substrate is convenient for high throughput screening. Similar assays using substrates towards the other phospholipid groups can also be applied e.g. using p-nitrophenylphosphorylinositol or p-nitrophenylphosphorylethanolamine.

A cytolytic assay can detect phospholipases with cytolytic activity based on lysis of erythrocytes. Toxic phospholipases can interact with eukaryotic cell membranes and hydrolyze phosphatidylcholine and sphingomyelin, leading to cell lysis. See, e.g., Titball (1993) Microbiol. Rev. 57:347-366.

Further assays like $^{31}$P-NMR and Liquid Chromatography coupled to triple quadrupole mass spectrometer (LC/MS/MS) are described in the example section of this application.

**PC and PE-specific phospholipase C:** The terms "PC and PE-specific phospholipase C" and "PC, PE-specific phospholipase C" and "polypeptide having activity towards phosphatidylcholine (PC) and phosphatidylethanolamine (PE)" are used interchangeably. They relate to a polypeptide having activity towards phosphatidylcholine (PC), phosphatidylethanolamine (PE). In addition to the PC and PE specificity it may also have some activity towards phosphatidic acid (PA) and phosphatidyl inositol (PI). Preferably a PC and PE specific phospholipase C removes at least 30% PC and at least 30% PE from an oil or fat with at least 100 ppm PC and 100 ppm PE when using the P-NMR assay of Example 1 at the optimal pH of the enzyme and an enzyme dosage of 10 mg/kg. More preferably it removes 40%, 50%, 60%, 70% or 80%, even more preferred it removes 90% and most preferred it removes between 90% and 100% of the PC in the oil or fat and 40%, 50%, 60%, 70% or 80%, even more preferred it removes 90% and most preferred it removes between 90% and 100% of the PE in the oil or fat.

**PI-Specific Phospholipase C:** The terms "PI-specific phospholipase C", "Phosphatidylinositol phospholipase C" and and "polypeptide having activity towards phosphatidylinositol (PI)" are used interchangeably. They relate to a polypeptide having activity towards phosphatidyl inositol (PI), meaning that its activity towards phosphatidylcholine (PC), phosphatidylethanolamine (PE), phosphatidic acid (PA) is low compared to the PI activity. PI-specific phospholipase C enzymes can either belong to the family of hydrolases and phosphodiesterases classified as EC 3.1.4.11 or to the family of lyases classified as EC 4.6.1.13. PI-specific phospholipase C activity may be determined according to the procedure described in Example 5. Preferably a PI-specific phospholipase C removes at least 30% PI from an oil or fat with at least 50 ppm PI when using the P-NMR assay of Example 1 at the optimal pH of the enzyme and an enzyme dosage of 10 mg/kg. More preferably it removes 40%, 50%, 60%, 70% or 80%, even more preferred it removes 90% and most preferred it removes between 90% and 100% of the PI in the oil or fat.

Preferably a PI-specific Phospholipase C removes at least 20% more PI when compared to the amount of PC, PE or PA it can remove, more preferred at least 30%, 40%, even more preferred at least 50% and most preferred at least 60% more PI when compared to the amount of PC, PE or PA it can remove.

**PC-, PE-, PA- and PI-Specific Phospholipase C:** The terms "PC-, PE-, PA,- and PI-specific phospholipase C", and "polypeptide having activity towards phosphatidylcholine (PC), phosphatidylethanoamine (PE), phosphatidic acid (PA) and phosphatidylinositol (PI)" are used interchangeably. They relate to a polypeptide having activity towards phosphatidylcholine (PC), phosphatidylethanoamine (PE), phosphatidic acid (PA), and phosphatidyl inositol (PI). Preferably a PC-, PE-, PA,- and PI-specific phospholipase C removes at least 30% of each of the four phospholipid species from an oil or fat with at least 100 ppm PC, 75 ppm PE, 5ppm PA and 50ppm PI when using the P-NMR assay of Example 1 at the optimal pH of the enzyme and an enzyme dosage of 10 mg/kg. More preferably it removes 40%, 50%, 60%, 70% or 80%, even more preferred it removes 90% and most preferred it removes between 90% and 100% of the PC in the oil or fat and 40%, 50%, 60%, 70% or 80%, even more preferred it removes 90% and most preferred it removes between 90% and 100% of the PE in the oil or fat.

**Bacterial Phospholipase C/Phospholipase C derived from a bacterium:** For purposes of the present invention, the term "derived from" as used herein in connection with a given source shall mean that the polypeptide is encoded by a polynucleotide, which in its native form is present in that source, and that the polypeptide is produced by the source or by a strain ("host cell") in which the polynucleotide from the source has been inserted. The term is also used in connection with polypeptides which are encoded by a modified form of a polynucleotide from the source, wherein the polynucleotides have been modified, such as by substitution, deletion, insertion or addition of one or more nucleic acid residues. Hence, the terms "bacterial Phospholipase C", "Phospholipase C derived from a bacterium" and "polypeptide having phospholipase C activity and being derived from a bacterium" are used interchangeably to refer to a polypeptide having Phospholipase C activity, which is encoded by a polynucleotide, which in its native form is present in bacterium, or is encoded by a modified form of that polynucleotide. In one aspect, the polypeptide obtained from a given source or host cell is secreted extracellularly.

Also comprised by this definition is a polypeptide, which has phospholipase C activity, and is selected from the group consisting of:

(a) a polypeptide having at least 60%, such as at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the amino acid sequence of a polypeptide having phospholipase C activity, and being encoded by a polynucleotide, which in its native form is present in a bacterium;

(b) a fragment of the polypeptide defined in (a);

including such polypeptides having a length of 280-320 amino acid residues, such as a length of 280-310 amino acid residues, 280-305 amino acid residues, 280-300 amino acid residues, 280-298 amino acid residues 280-297 amino acid residues, 280-296 amino acid residues, 285-320 amino acid residues, 285-315 amino acid residues, 285-310 amino acid residues, 285-305 amino acid residues, 285-300 amino acid residues, 285-298 amino acid residues, 285-297 amino acid residues, 285-296 amino acid residues, 290-320 amino acid residues, 290-315 amino acid residues, 290-310 amino acid residues, 290-305 amino acid residues, 290-300 amino acid residues, 290-298 amino acid residues, 290-297 amino acid residues, 290-296 amino acid residues, 295-320 amino acid residues, 295-315 amino acid residues, 295-310 amino acid residues, 295-305 amino acid residues, 295-300 amino acid residues, 295-298 amino acid residues, 255-297 amino acid residues, or a length of 295-296 amino acid residues.

**Fungal Phospholipase C/Phospholipase C derived from a fungus:** In accordance with the above, the terms "fungal Phospholipase C" "Phospholipase C derived from a fungus", and "polypeptide having phospholipase C activity and being derived from a fungus" are used interchangeably to refer to a polypeptide having Phospholipase C activity, which is encoded by a polynucleotide, which in its native form is present in a fungus, or is encoded by a modified form of that polynucleotide.

Also comprised by this definition is a polypeptide, which has phospholipase C activity, and is selected from the group consisting of:

(a) a polypeptide having at least 60%, such as at at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the amino acid sequence of a polypeptide having phospholipase C activity, and being encoded by a polynucleotide, which in its native form is present in a fungus;

(b) a fragment of the polypeptide defined in (a);

including such polypeptides having a length of 540-640 amino acid residues, such as a length of, 550-640 amino acid residues, 560-640 amino acid residues, 570-640 amino acid residues, 540-630 amino acid residues, such as a length of, 550-630 amino acid residues, 560-630 amino acid residues, 570-630 amino acid residues, 570-620 amino acid residues, 570-610 amino acid residues, 570-600 amino acid residues 570-597 amino acid residues, 570-596 amino acid residues, 580-640 amino acid residues, 540-630 amino acid residues, such as a length of, 540-620 amino acid residues, 540-610 amino acid residues, 540-600 amino acid residues, 540-590 amino acid residues, 540-580 amino acid residues, 540-570 amino acid residues, 540-560 amino acid residues, 540-560 amino acid residues, 550-630 amino acid residues, such as a length of, 550-620 amino acid residues, 550-610 amino acid residues, 550-600 amino acid residues, 550-590 amino acid residues, 550-580 amino acid residues, 550-570 amino acid residues, 550-560 amino acid residues, 550-560 amino acid residues, 560-630 amino acid residues, such as a length of, 560-620 amino acid residues, 560-610 amino acid residues, 560-600 amino acid residues, 560-590 amino acid residues, 560-580 amino acid residues, 560-570 amino acid residues, 560-560 amino acid residues, 560-560 amino acid residues, 570-630 amino acid residues, such as a length of, 570-620 amino acid residues, 570-610 amino acid residues, 570-600 amino acid residues, 570-590 amino acid residues, 570-580 amino acid residues, 570-570 amino acid residues, 570-560 amino acid residues, 570-560 amino acid residues, 580-630 amino acid residues, 580-620 amino acid residues, 580-610 amino acid residues, 580-600 amino acid residues, 580-598 amino acid residues, 580-597 amino acid residues, 580-596 amino acid residues, 590-640 amino acid residues, 590-630 amino acid residues, 590-620 amino acid residues, 590-610 amino acid residues, 590-600 amino acid residues, 590-698 amino acid residues, 590-597 amino acid residues, 590-596 amino acid residues, 595-640 amino acid residues, 595-630 amino acid residues, 595-620 amino acid residues, 595-610 amino acid residues, 595-600 amino acid residues, 595-598 amino acid residues, 595-597 amino acid residues, or a length of 595-596 amino acid residues.

**Reaction time:** For the purpose of the present invention the "reaction time" of a phospholipase C is the time required to convert 50% of the content in crude soybean oil of the phospholipid towards which the phospholipase C has activity, when determined in a degumming assay comprising: Adding Ortho Phosphoric acid (85% solution) to a sample of said oil in amounts equal to 0.05% (100% pure Ortho Phosphoric acid) based on oil amount and mixing the sample in ultrasonic bath for 5 minutes, incubating the sample in rotator for 15 minutes followed by base neutralization with 4 M NaOH applied in equivalents to pure Ortho Phosphoric acid in ultrasonic bath for 5 minutes, adding said phospholipase C in amounts corresponding to 10 mg enzyme protein/kg oil in low aqueous system (3% water total based on oil amount), subjecting the sample to ultrasonic treatment and incubation at a temperature 50-60°C with stirring, and subjecting the oil to centrifugation at 700g at 85°C for 15 minutes. An example of the degumming assay and data obtained thereby is provided in Example 1

**Catalytic domain:** The term "catalytic domain" means the region of an enzyme containing the catalytic machinery of the enzyme.

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

**Fragment:** The term "fragment" means a polypeptide or a catalytic or phospholipid binding domain having one or more (e.g., several) amino acids absent from the amino and/or carboxyl terminus of a mature polypeptide or domain; wherein the fragment has phospholipase C activity.

**Host cell:** The term "host cell" means any cell type that is susceptible to transformation, transfection, transduction, or the like with a nucleic acid construct or expression vector comprising a polynucleotide of the present invention.

The term "host cell" encompasses any progeny of a parent cell that is not identical to the parent cell due to mutations that occur during replication.

**Isolated:** The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (e.g., recombinant production in a host cell; multiple copies of a gene encoding the substance; and use of a stronger promoter than the promoter naturally associated with the gene encoding the substance).

**Low stringency conditions:** The term "low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 50°C.

**Medium stringency conditions:** The term "medium stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 55°C.

**Medium-high stringency conditions:** The term "medium-high stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 60°C.

**High stringency conditions:** The term "high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 65°C.

**Very high stringency conditions:** The term "very high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 70°C.

**Mature polypeptide:** The term "mature polypeptide" means a polypeptide in its final form following translation and any post-translational modifications, such as N-terminal processing, C-terminal truncation, glycosylation, phosphorylation, etc. In one aspect, the mature polypeptide may be predicted, such as by using the software SignalP (Nielsen et al., 1997, Protein Engineering 10: 1-6)]

**Mature polypeptide coding sequence:** The term "mature polypeptide coding sequence" means a polynucleotide that encodes a mature polypeptide having phospholipase C activity. A mature polypeptide sequence may be predicted using the software SignalP (Nielsen *et al.,* 1997, *supra*).

**Sequence identity:** The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment)

For purposes of the present invention, the sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, supra), preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment – Total Number of Gaps in Alignment)

**Subsequence:** When used in connection with nucleic acid sequences, the term "subsequence" means a polynucleotide having one or more (e.g., several) nucleotides absent from the 5' and/or 3' end of a mature polypeptide coding sequence; wherein the subsequence encodes a fragment having phospholipase C activity.

**Variant:** The term "variant" means a polypeptide having phospholipase C activity comprising one or more alterations, *i.e.*, one or more substitutions, insertions, and/or deletions, at one or more (e.g., several) positions. A substitution means replacement of the amino acid residue occupying a position with a different amino acid residue; a deletion means removal of the amino acid residue occupying a position; and an insertion means adding one or more amino acid residues adjacent to and immediately following the amino acid residue occupying a position.

**Crude oil:** The term "crude oil" refers to (also called a non-degummed oil) a pressed or extracted oil or a mixture thereof from, e.g. vegetable sources, including but not limited to acai oil, almond oil, babassu oil, blackcurrent seed oil, borage seed oil, canola oil, cashew oil, castor oil, coconut oil, coriander oil, corn oil, cottonseed oil, crambe oil, flax seed oil, grape seed oil, hazelnut oil, hempseed oil, jatropha oil, jojoba oil, linseed oil, macadamia nut oil, mango kernel oil, meadowfoam oil, mustard oil, neat's foot oil, olive oil, palm oil, palm kernel oil, palm olein, peanut oil, pecan oil, pine nut oil, pistachio oil, poppy seed oil, rapeseed oil, rice bran oil, safflower oil, sasanqua oil, sesame oil, shea butter, soybean oil, sunflower seed oil, tall oil, tsubaki oil walnut oil, varieties of "natural" oils having altered fatty acid compositions via Genetically Modified Organisms (GMO) or traditional "breading" such as high oleic, low linolenic, or low saturated oils (high oleic canola oil, low linolenic soybean oil or high stearic sunflower oils).

**Degummed oil:** The term "degummed oil" refers to an oil obtained after removal of non-hydratable phospholipids, hydratable phospholipids, and lecithins (known collectively as "gums") from the oil to produce a degummed oil or fat product that can be used for food production and/or non-food applications, e.g. biodiesel. In certain embodiments, the degummed oil has the phospholipids content of less than 200 ppm phosphorous, such as less than 150 ppm phosphorous, less than 100 ppm phosphorous, less than (or less than about) 50 ppm phosphorous, less than (or less than about) 40 ppm phosphorous, less than (or less than about) 30 ppm phosphorous, less than (or less than about) 20 ppm phosphorous, less than (or less than about) 15 ppm phosphorous, less than (or less than about) 10 ppm phosphorous, less than (or less than about) 7 ppm phosphorous, less than (or less than about) 5 ppm phosphorous, less than (or less than about) 3 ppm phosphorous or less than (or less than about) 1 ppm phosphorous

**Detailed Description of the Invention**

The present inventors have observed that very efficient hydrolysis of phospholipids in processes such as degumming of oils may be obtained by combining fungal phospholipase C, characterized by broad substrate specificity with bacterial phospholipase C characterized by faster, but more specific activity of bacterial phospholipase.

**Enzyme compositions**

Hence, in a first aspect the present invention provides a composition comprising a plurality of polypeptides having phospholipase C activity, wherein

- at least one of said polypeptides having phospholipase C activity is a polypeptide derived from a fungus; and
- at least one of said polypeptides having phospholipase C activity is a polypeptide derived from a bacterium.

In particular embodiments of the invention, the composition comprises 2 or more polypeptides, such as 2, 3, 4, 5, or 6 polypeptides having phospholipase C activity, each of which is derived from a fungus.

According to other embodiments of the invention the composition comprises 2 or more polypeptides, such as 1, 2, 3, 4, 5, or 6 polypeptides having phospholipase C activity, each of which is derived from a bacterium.

In particular, the invention provides compositions in which one or more of said polypeptides derived from a fungus has/have broad substrate acceptance, such as activity towards two, three or four phospholipids selected from the group consisting of phosphatidylcholine (PC), phosphatidylethanoamine (PE), phosphatidic acid (PA), and phosphatidyl inositol (PI). According to some embodiments, one or more of said polypeptides derived from a fungus has/have activity towards phosphatidylcholine (PC), phosphatidylethanoamine (PE), phosphatidic acid (PA), and phosphatidyl inositol (PI).In general, the polypeptides having phospholipase C activity which are derived from bacteria have relatively fast activity and short reaction time. Hence, according to some embodiments at least one of said polypeptides derived from a bacterium, such as at least 2, at least 3, at least 4, at least 5 or at least 6 of said polypeptides derived from a bacterium has/have a reaction time of 1-5 hours, such as 1-4 hours, 1-3 hours, 1.5-3.5 hours, 1.5-2.5 hours, 2-4 hours, such as 1 hour, 2 hours, 3 hours, 4 hours or 5 hours.

According to some embodiments, the invention provides compositions in which one or more of said polypeptides derived from a bacterium has/have a narrower substrate acceptance, such as activity towards one, two or three phospholipids selected from the group consisting of phosphatidylcholine (PC), phosphatidylethanoamine (PE), and phosphatidyl inositol (PI). In particular embodiments, the invention provides compositions, wherein one or more of said polypeptides derived from a bacterium has activity towards phosphatidyl inositol (PI) and/or is a PI-specific phospholipase C. According to additional embodiments, one or more of said polypeptides derived from a bacterium has/have activity towards phosphatidylcholine (PC) and phosphatidylethanoamine (PE) and/or is a PC- and PE-specific phospholipase C.

In general, the polypeptides having phospholipase C activity which are derived from fungi need long reaction time to convert all phospholipid species (PI; PA; PE; PC). Accordingly, in some embodiments, at least one of said polypeptides derived from a fungus, such as at least 2, at least 3, at least 4, at least 5 or at least 6 of said polypeptides derived from a fungus has/have a has a reaction time of 3-36 hours, such as 3- 30 hours, or such as 4-24 hours.

The fungus (kingdom Fungi) could be a dicaryotic fungus, more specifically from the fungal phylum *Ascomycota*, more specifically from a filamentous ascomycete from the subphylum *Pezizomycotina*, more specifically from the fungal classes *Sordariomycetes* or *Eurotiomycetes*; more specifically from the fungal orders *Sordariales, Hypocreales* and *Eurotiales*, more specifically from the fungal families *Nectriaceae* and *Aspergillaceae*; more specifically from the fungal genera *Kionochaeta*, *Nectria*, and *Penicillium.*

While several fungal sources are available, some embodiments of the Invention pertain to compositions in which one or more of said polypeptides derived from a fungus, such as 2, 3, 4, 5 or 6 of said polypeptides derived from a fungus is/are selected from the group consisting of a polypeptide derived from *Kionochaeta sp.*, a polypeptide derived from *Penicillium emersonii* and a polypeptide derived from *Nectria marianneae.* Likewise, the skilled person will be aware of numerous sources of bacterial polypeptides having Phospholipase C activity. The bacterial source (superkingdom Bacteria) could be members of the phyla *Firmicutes* or Proteobacteria, more specifically from the classes *Bacilli* or Gammaproteobacteria, more specifically from the bacterial orders *Bacillales* or Pseudomonades, more specifically from the bacterial families *Bacillaceae*, *Listeriaceae*, or *Pseudomonadaceae*; more specifically from the bacterial genera *Bacillus*, *Listeria*, or *Pseudomonas*. Hence, in specific embodiments of the invention one or more of said polypeptides derived from a bacterium is/are selected from the group consisting of a polypeptide derived from a *Bacillus* species, a polypeptide derived from a *Listeria* species, a polypeptide derived from a *pseudomonas* species. In particular, the said Bacillus species may be selected from the group consisting of *Bacillus alkalophilus*, *Bacillus amyloliquefaciens*, *Bacillus brevis, Bacillus cereus, Bacillus circulans, Bacillus clausii, Bacillus coagulans, Bacillus firmus, Bacillus lautus, Bacillus lentus, Bacillus licheniformis*, *Bacillus megaterium, Bacillus pseudomycoides, Bacillus mycoides, Bacillus pumilus, Bacillus stearothermophilus, Bacillus subtilis and Bacillus thuringiensis.*

In specific embodiments, the said *Listeria* species is *Listeria innocua.*

In other specific embodiments the said Pseudomonas species is selected from the group consisting of *Pseudomonas chlororaphis* and *Pseudomonas protegens.*

Strains of the various species mentioned above are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

In particular embodiments of the invention, the at least one polypeptide having phospholipase C activity and being derived from a fungus has a size/molecular weight which is at least 38kDa, such as within the range of 40-65 kDa, the molecular weight being calculated from the amino acid sequence of the polypeptide, excluding the amino acid sequence of any signal peptide and/or propeptide.

The at least one polypeptide having phospholipase C activity and being derived from a fungus may further be characterized by having a catalytic site defined by amino acid residues D171, H173, D245, H249, N285, H286, H393, H427 and H429, using the amino acid numbering of SEQ ID NO: 2. Preferably, amino acid residues D171, H173, D245, N285, H393, H427 and H429 are zink-binding residues, whereas H249 and H286 are not.

In the at least one polypeptide having phospholipase C activity and being derived from a fungus the length of the catalytic segment of the amino acid sequence; i.e. the sequence from first (N-terminal) to last (C-terminal) catalytic amino acid residue, preferably is within the range of 250-265 amino acid residues, such as 255-261 amino acid residues or such as 256-260 amino acid residues.

Also, in the at least one polypeptide having phospholipase C activity and being derived from a fungus the length of the amino acid sequence before the first catalytic site; i.e. from the N-terminus of the mature polypeptide to the first (N-terminal) catalytic amino acid residue, is from 100-200 amino acid residues, such as from 100-180 amino acid residues, from 100-170, from 100-160, from 100-150, from 110-180, from 120-180, from 130-180, from 140-180, from 150-180, from 110-170, from 120-170, or from 130-170 amino acid residues.

Further, in the at least one polypeptide having phospholipase C activity and being derived from a fungus the length of the amino acid sequence after the last catalytic site; i.e. from the last (C-terminal) catalytic amino acid residue to the C-terminus of the mature polypeptide, is from 100-200 amino acid residues, such as from 100-190 amino acid residues, from 100-180, from 100-175, from 110-200, from 110-190 from 110-180, from 110-175, from 120-200, from 120-190, from 120-180, from 120-175, from 130-200, from 130-190, from 130-180, from 130-175, from 140-200, from 140-190, from 140-180, from 140-170, from 150-200, from 150-190, from 150-180, or such as from 150-175 amino acid residues.

According to some embodiments, the at least one polypeptide having phospholipase C activity and being derived from a fungus comprises a calcineurin-like phosphoesterase domain as defined by CATH ID 3.60.21.10. Hence, the at least one polypeptide having phospholipase C activity and being derived from a fungus may be selected from polypeptides, which are identifiable (through standard alignment methods/tools such as those provided in the definition of "sequence identity") as having calcineurin-like phosphoesterase domain as defined by CATH ID 3.60.21.10, starting at position 130-170in the mature polypeptide, such as at position 130-150 and aligning to the rest of the sequence (allowing for insertions in the domain), In some embodiments of the invention, the said polypeptide(s) having activity towards phosphatidyl inositol (PI)/said PI-specific phospholipase C, and said polypeptides having activity towards phosphatidylcholine (PC) and phosphatidylethanolamine (PE)/said PC- and PE-specific phospholipase C is/are capable of reducing the content of the respective phospholipids present in a crude oil, such as crude soybean oil, by at least 30% (w/w), such as 35% (w/w), 40% (w/w), 45% (w/w), 50% (w/w), 55% (w/w), 60% (w/w), 65% (w/w), 70% (w/w), 75% (w/w), 80% (w/w), 85% (w/w), 90% (w/w), 95% (w/w), 98% (w/w), 99% (w/w) or such as 100% (w/w), when applied in an amount of 10 mg polypeptide/kg oil at the optimal pH, such as a pH within the range of 4.5 - 8.5, such as in the range of 5.0 - 8.0 or such as in the range of 6.5 - 7.5 and incubated for 2-4 hours at a temperature of 50-60°C.

According to some embodiments the polypeptide(s) having activity towards phosphatidylinositol/said PI-specific phospholipase C used according to the invention may be able to reduce the phosphatidylinositol content of crude soy bean oil by 50% or more, 50%, such as by 55%, by 60%, by 65%, by 70%, by 75%, by 80%, by 85%, by 90% or by 95% or more, the reduction in phosphatidylinositol content being determined by $^{31}$P-NMR after addition of 100 mg enzyme protein(EP)/kg oil and incubation of the oil and enzyme at 50°C for 2 hours at pH 5.5.

Preferably, the polypeptide(s) having activity towards phosphatidylinositol/the PI-specific phospholipase C according to the invention is/are able to reduce the phosphorous content of crude soy bean oil to 20 mg/kg oil or less as determined by Inductively coupled plasma optical emission spectrometry (ICP-OES) after incubation of 4 mg enzyme protein/kg oil in a low aqueous system comprising 3% water based on oil amount at 50-60°C for 5 hours.

In further embodiments the polypeptide having activity towards PC and PE/the PC- and PE-specific phospholipase C is able to reduce the phosphatidyl ethanolamine and/or phosphatidyl choline content of crude soy bean oil by 50% or more, 50%, such as by 55%, by 60%, by 65%, by 70%, by 75%, by 80%, by 85%, by 90% or by 95% or more, the reduction in phosphatidyl ethanolamine and/or phosphatidyl choline content being determined by $^{31}$P-NMR after addition of 100 mg enzyme protein(EP)/kg oil and incubation of the oil and enzyme at 50°C for 2 hours at pH 5.5.

In still further embodiments, the polypeptide having polypeptide having activity towards PC and PE/the PC- and PE-specific phospholipase C is/are able to reduce the phosphorous content of crude soy bean oil to 20 mg/kg oil or less as determined by Inductively coupled plasma optical emission spectrometry (ICP-OES) after incubation of 4 mg enzyme protein/kg oil in a low aqueous system comprising 3% water based on oil amount at 50-60°C for 5 hours.

The ability of the polypeptides to reduce phosphorous content may in particular be determined using a crude soy oil which comprises 80-140 ppm phosphorous present as phosphatidic acid (PA), 140-200 ppm phosphorous present as phosphatidyl ethanolamine (PE), 70-110 ppm phosphorous present as phosphatidic acid (PI) and 130-200 ppm phosphorous present as phosphatidyl choline; the phosphorous content being measured by $^{31}$P-NMR.

Further, said reduction of phosphorous content may be obtained in an oil degumming process comprising the steps of:

i) Optionally treating crude soy bean oil with acid/base by adding an 85% solution of Ortho Phosphoric acid in amounts corresponding to 0.05% (100% pure Ortho Phosphoric acid) based on oil amount, mixing in ultrasonic bath for 5 minutes, followed by incubation in rotator for 15 minutes and base neutralization with 4 M NaOH applied in equivalents (from 0.5 to 0.15) to pure Ortho Phosphoric acid in ultrasonic bath for 5 minutes;

ii) Adding the polypeptide to the oil in amounts of 4 mg enzyme protein/kg oil in a low aqueous system comprising 3% water based on oil amount and subjecting the oil and the polypeptide to ultrasonic treatment for 5 minutes;

iii) Incubating the polypeptide and oil at 50-60°C for 5 hours with stirring at 20 rpm;

iv) Centrifuging the oil and the polypeptide at 700 g at 85°C for 15 minutes.

Likewise, in some embodiments the composition according to the invention said polypeptide(s) having activity towards phosphatidylcholine (PC), phosphatidylethanolamine (PE), phosphatidic acid (PA), and phosphatidyl inositol (PI) is/are capable of reducing the content of the respective phospholipids present in a crude oil, such as crude soybean oil, by at least 30% (w/w), such as 35% (w/w), 40% (w/w), 45% (w/w), 50% (w/w), 55% (w/w), 60% (w/w), 65% (w/w), 70% (w/w), 75% (w/w), 80% (w/w), 85% (w/w), 90% (w/w), 95% (w/w), 98% (w/w), 99% (w/w) or such as 100% (w/w), when applied in an amount of 100mg polypeptide/kg oil at the optimal pH, such as a pH within the range of 4.5 - 8.5, such as in the range of 5.0 - 8.0 or such as in the range of 6.5 - 7.5 and incubated for 2-4 hours at a temperature of 50-60°C.

The reduction of PA, PC, PE and PI content may in particular be determined by $^{31}$P-NMR after addition of 100 mg enzyme protein(EP)/kg oil and incubation of the oil and enzyme at 50°C for 2 hours at pH 5.5.

The ability of the polypeptide to reduce phosphorous content and increase dicylglyceride content may in particular be determined using a crude soy oil which comprises 80-140 ppm phosphorous present as phosphatidic acid (PA), 140-200 ppm phosphorous present as phosphatidyl ethanolamine (PE), 70-110 ppm phosphorous present as phosphatidic acid (PI) and 130-200 ppm phosphorous present as phosphatidyl choline; the phosphorous content being measured by $^{31}$P-NMR and the diacylglycerol content being measured by HPLC-Evaporative Light Scattering Detection (HPLC-ELSD).

In particular, the polypeptide of the present invention is capable of increasing the amount of diacylglyceride by at least 0.1 %w/w when applied in amounts of 8.5 mg EP/kg oil to crude soy bean oil and incubated for 3 hours. Preferably, the oil has been acid/base treated by addition of 85% solution of Ortho Phosphoric acid in amounts corresponding to 0.05% (100% pure Ortho Phosphoric acid) based on oil amount, and base neutralization with 4 M NaOH applied in equivalents of 0.5 to pure Ortho Phosphoric acid.

Preferably, the polypeptide of the present invention is capable of increasing the amount of diacylglyceride by at least 0.3 %w/w when applied in amounts of 8.5 mg EP/kg to crude soybean oil and incubated with the oil for 3 hours at 50°C, when the crude oil has been acid/base treated with 0.05% Ortho Phosphoric acid and 1 eqv. NaOH.

The reduction of PA, PC, PE and PI content and/or production of diacylglyceride may in particular be obtained in an oil degumming process comprising the steps of:

i) Optionally treating crude soy bean oil with acid/base by adding an 85% solution of Ortho Phosphoric acid in amounts corresponding to 0.05% (100% pure Ortho Phosphoric acid) based on oil amount, mixing in ultrasonic bath for 5 minutes, followed by incubation in rotator for 15 minutes and base neutralization with 4 M NaOH applied in equivalents (from 0.5 to 0.15) to pure Ortho Phosphoric acid in ultrasonic bath for 5 minutes;

ii) Adding the polypeptide to the oil in a low aqueous system comprising 3% water based on oil amount and subjecting the oil and the polypeptide to ultrasonic treatment for 5 minutes;

iii) Incubating the polypeptide and oil at 50-60°C with stirring at 20 rpm;

iv) Centrifuging the oil and the polypeptide at 700 g at 85°C for 15 minutes.

The composition may in particular comprise a polypeptide, which has phospholipase C activity is derived from a fungus, and is selected from the group consisting of:

(a) a polypeptide having at least 60% sequence identity to the mature polypeptide of any one of SEQ ID NO: 2, SEQ ID NO: 4, and SEQ ID NO: 7;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 1, SEQ ID NO: 3 and SEQ ID NO: 5 (ii) the genomic DNA sequence comprising the mature polypeptide coding sequence of any one of SEQ ID NO: 1, SEQ ID NO: 3 and SEQ ID NO: 5, (iii) the cDNA sequence of SEQ ID NO: 5 (SEQ ID NO: 6) or (iv) the full-length complementary strand of (i), (ii) or (iii);

(c) a polypeptide encoded by a polynucleotide having at least 60% sequence identity to the mature polypeptide coding sequence of any one of SEQ ID NO: 1, SEQ ID NO: 3 and SEQ ID NO: 5;

(d) a variant of the mature polypeptide of any one of SEQ ID NO: 2, SEQ ID NO: 4, and SEQ ID NO: 7 comprising a substitution, deletion, and/or insertion at one or more positions; and

(e) a fragment of the polypeptide of (a), (b), (c), or (d).

In specific embodiments, the composition according to the invention comprises a polypeptide, which has phospholipase C activity and is selected from the group consisting of:

(a) a polypeptide having at least 60% sequence identity to the mature polypeptide of SEQ ID NO: 2,

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 1, (ii) the genomic DNA sequence comprising the mature polypeptide coding sequence of SEQ ID NO: 1, or (iii) the full-length complementary strand of (i) or (ii));

(c) a polypeptide encoded by a polynucleotide having at least 60% sequence identity to the mature polypeptide coding sequence of SEQ ID NO: 1;

(d) a variant of the mature polypeptide of SEQ ID NO: 2, comprising a substitution, deletion, and/or insertion at one or more positions; and

(e) a fragment of the polypeptide of (a), (b), (c), or (d).

In other specific embodiments, the composition comprises a polypeptide, which has phospholipase C activity and is selected from the group consisting of:

(a) a polypeptide having at least 60% sequence identity to the mature polypeptide of SEQ ID NO: 4;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 3 (ii) the genomic DNA sequence comprising the mature polypeptide coding sequence of SEQ ID NO: 3, or (iii) the full-length complementary strand of (i) or (ii);

(c) a polypeptide encoded by a polynucleotide having at least 60% sequence identity to the mature polypeptide coding sequence of SEQ ID NO: 3;

(d) a variant of the mature polypeptide of SEQ ID NO: 4, comprising a substitution, deletion, and/or insertion at one or more positions; and

(e) a fragment of the polypeptide of (a), (b), (c), or (d).

In still other embodiments, the composition comprises a polypeptide, which has phospholipase C activity and is selected from the group consisting of:

(a) a polypeptide having at least 60% sequence identity to the mature polypeptide of SEQ ID NO: 7;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 5 (ii) the genomic DNA sequence comprising the mature polypeptide coding sequence of SEQ ID NO: 5, (iii) the cDNA sequence of SEQ ID NO: 5 (SEQ ID NO: 6) or (iv) the full-length complementary strand of (i), (ii) or (iii);

(c) a polypeptide encoded by a polynucleotide having at least 60% sequence identity to the mature polypeptide coding sequence of SEQ ID NO: 5;

(d) a variant of the mature polypeptide of SEQ ID NO: 7 comprising a substitution, deletion, and/or insertion at one or more positions; and

(e) a fragment of the polypeptide of (a), (b), (c), or (d).

The said mature polypeptide may in particular consist of, essentially consist of or comprise an amino acid sequence selected from the group consisting of amino acid residues 19-643 of SEQ ID NO.: 2, amino acid residues 17-610 of SEQ ID NO.: 4, amino acid residues 20-626 of SEQ ID NO.: 7, and amino acid residues 37-626 of SEQ ID NO.: 7.

In other embodiments, the mature polypeptide of SEQ ID NO: 2 comprises, consists of or consists essentially of amino acids 43-618 of SEQ ID NO: 2. In further embodiments, the mature polypeptide of SEQ ID NO: 4 comprises, consists of or consists essentially of amino acids 20-576 of SEQ ID NO: 4. In still further embodiments of the invention, the mature polypeptide of SEQ ID NO: 7 comprises, consists of or consists essentially of amino acids 37-618 of SEQ ID NO: 7.

In further embodiments, the composition comprises a polypeptide, which has phospholipase C activity, is derived from a bacterium and is selected from the group consisting of:

(a) a polypeptide having at least 60% sequence identity to the mature polypeptide of any one of SEQ ID NO: 9, SEQ ID NO: 12, SEQ ID NO: 15, SEQ ID NO: 18, SEQ ID NO: 21, SEQ ID NO: 24, SEQ ID NO: 26, SEQ ID NO: 29, SEQ ID NO: 31, SEQ ID NO: 34, SEQ ID NO: 36, SEQ ID NO: 39, SEQ ID NO: 41, SEQ ID NO: 43 and SEQ ID NO: 45;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 8, SEQ ID NO: 11, SEQ ID NO: 14, SEQ ID NO: 17, SEQ ID NO: 20, SEQ ID NO: 23 SEQ ID NO: 25, SEQ ID NO: 28, SEQ ID NO: 30, SEQ ID NO: 33, SEQ ID NO: 35, SEQ ID NO: 38, SEQ ID NO: 40, SEQ ID NO: 42, and SEQ ID NO: 44 (ii) the genomic DNA sequence comprising the mature polypeptide coding sequence of any one of SEQ ID NO: 8, SEQ ID NO: 11, SEQ ID NO: 14, SEQ ID NO: 17, SEQ ID NO: 20, SEQ ID NO: 23, SEQ ID NO: 25, SEQ ID NO: 28, SEQ ID NO: 30, SEQ ID NO: 33, SEQ ID NO: 35, SEQ ID NO: 38, SEQ ID NO: 40, SEQ ID NO: 42, and SEQ ID NO: 44 or (iii) the full-length complementary strand of (i) or (ii);

(c) a polypeptide encoded by a polynucleotide having at least 60% sequence identity to the mature polypeptide coding sequence of any one of SEQ ID NO: 8, SEQ ID NO: 11, SEQ ID NO: 14, SEQ ID NO: 17, SEQ ID NO: 20, SEQ ID NO: 23, SEQ ID NO: 25, SEQ ID NO: 28, SEQ ID NO: 30, SEQ ID NO: 33, SEQ ID NO: 35, SEQ ID NO: 38, SEQ ID NO: 40 SEQ ID NO: 42 and SEQ ID NO: 44;

(d) a variant of the mature polypeptide of any one of SEQ ID NO: 9, SEQ ID NO: 12, SEQ ID NO: 15, SEQ ID NO: 18, SEQ ID NO: 21, SEQ ID NO: 24, SEQ ID NO: 26, SEQ ID NO: 29, SEQ ID NO: 31, SEQ ID NO: 34, SEQ ID NO: 36, SEQ ID NO: 39, SEQ ID NO: 41, SEQ ID NO: 43 and SEQ ID NO: 45comprising a substitution, deletion, and/or insertion at one or more positions; and

(e) a fragment of the polypeptide of (a), (b), (c), or (d).

In still further embodiments, the composition according to the invention comprises a polypeptide, which has phospholipase C activity and is selected from the group consisting of:

(a) a polypeptide having at least 60% sequence identity to the mature polypeptide of any one of SEQ ID NO: 9, SEQ ID NO: 12, SEQ ID NO: 15, SEQ ID NO: 18, SEQ ID NO: 21, SEQ ID NO: 24, and SEQ ID NO: 43;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42 (ii) the genomic DNA sequence comprising the mature polypeptide coding sequence of any one of SEQ ID NO: 8, SEQ ID NO: 11, SEQ ID NO: 14, SEQ ID NO: 17, SEQ ID NO: 20, SEQ ID NO: 23, and SEQ ID NO: 42 or (iii) the full-length complementary strand of (i) or (ii);

(c) a polypeptide encoded by a polynucleotide having at least 60% sequence identity to the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42;

(d) a variant of the mature polypeptide of any one of SEQ ID NO: 9, SEQ ID NO: 12, SEQ ID NO: 15, SEQ ID NO: 18, SEQ ID NO: 21, SEQ ID NO: 24, and SEQ ID NO: 43 comprising a substitution, deletion, and/or insertion at one or more positions; and

(e) a fragment of the polypeptide of (a), (b), (c), or (d).

The composition according to the invention may, according to some embodiments comprise a polypeptide, which has phospholipase C activity and is selected from the group consisting of:

(a) a polypeptide having at least 60% sequence identity to the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 25, SEQ ID NO: 28, SEQ ID NO: 30, SEQ ID NO: 33, SEQ ID NO: 35, SEQ ID NO: 38, and SEQ ID NO: 44 (ii) the genomic DNA sequence comprising the mature polypeptide coding sequence of any one of SEQ ID NO: 25, SEQ ID NO: 28, SEQ ID NO: 30, SEQ ID NO: 33, SEQ ID NO: 35, SEQ ID NO: 38, and SEQ ID NO: 44 or (iii) the full-length complementary strand of (i) or (ii);

(c) a polypeptide encoded by a polynucleotide having at least 60% sequence identity to the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44;

(d) a variant of the mature polypeptide of any one of SEQ ID NO: 26, SEQ ID NO: 29, SEQ ID NO: 31, SEQ ID NO: 34, SEQ ID NO: 36 SEQ ID NO: 39, and SEQ ID NO: 45 comprising a substitution, deletion, and/or insertion at one or more positions; and

(e) a fragment of the polypeptide of (a), (b), (c), or (d).

In other embodiments, the composition according to the invention comprises a polypeptide, which has phospholipase C activity and is selected from the group consisting of:

(a) a polypeptide having at least 60% sequence identity to the mature polypeptide of SEQ ID NO: 41;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 40 (ii) the genomic DNA sequence comprising the mature polypeptide coding sequence of SEQ ID NO: 40, or (iii) the full-length complementary strand of (i) or (ii);

(c) a polypeptide encoded by a polynucleotide having at least 60% sequence identity to the mature polypeptide coding sequence of SEQ ID NO: 40;

(d) a variant of the mature polypeptide of SEQ ID NO: 41, comprising a substitution, deletion, and/or insertion at one or more positions; and

(e) a fragment of the polypeptide of (a), (b), (c), or (d).

According to the embodiments set forth above, the said mature polypeptide consists of or comprises an amino acid sequence selected from the group consisting of amino acid residues 23-322 of SEQ ID NO: 9, amino acid residues 24-322 of SEQ ID NO: 9, amino acid residues 25-322 of SEQ ID NO: 9, amino acid residues 26-322 of SEQ ID NO: 9, amino acid residues 27-322 of SEQ ID NO: 9, amino acid residues 28-322 of SEQ ID NO: 9, the amino acid sequence of SEQ ID NO: 10, amino acid residues 26-323 of SEQ ID NO: 12, the amino acid sequence of SEQ ID NO: 13, amino acid residues 26-323 of SEQ ID NO: 15, the amino acid sequence of SEQ ID NO: 16, amino acid residues 29-323 of SEQ ID NO: 18, the amino acid sequence of SEQ ID NO: 19, amino acid residues 26-322 of SEQ ID NO: 21, the amino acid sequence of SEQ ID NO: 22, amino acid residues 26-322 of SEQ ID NO: 24, amino acid residues 34-278 of SEQ ID NO: 26, the amino acid sequence of SEQ ID NO: 27, amino acid residues 29-283 of SEQ ID NO: 29, amino acid residues 25-283 of SEQ ID NO: 31, the amino acid sequence of SEQ ID NO: 32, amino acid residues 25-283 of SEQ ID NO: 34, amino acid residues 28-289 of SEQ ID NO: 36, the amino acid sequence of SEQ ID NO: 37, the amino acid sequence of SEQ ID NO: 39, amino acid residues 24-328 of SEQ ID NO: 41, amino acids 28-339 of SEQ ID NO: 43 and amino acids 25-280 of SEQ ID NO: 45.

Further, the composition according to the invention may be one, wherein said plurality of polypeptides having phospholipase C activity is selected from the group consisting of:

- one polypeptide, which is as defined above and is derived from a fungus and one polypeptide, which is as defined above and is derived from a bacterium;
- one polypeptide, which is as defined above and is derived from a fungus and two polypeptides, which are as defined above and are each derived from a bacterium;
- one polypeptide, which is as defined above and is derived from a fungus and three polypeptides, which are as defined above and are each derived from a bacterium;
- two polypeptides, which are as defined above and are each derived from a fungus and one polypeptide, which is as defined above and is derived from a bacterium;
- two polypeptides, which are as defined above and are each derived from a fungus and two polypeptides, which are as defined above and are each derived from a bacterium;
- two polypeptides, which are as defined above and are each derived from a fungus and three polypeptides, which are as defined above and are each derived from a bacterium;
- three polypeptides, which are as defined above and are each derived from a fungus and one polypeptide, which is as defined above and is derived from a bacterium;
- three polypeptides, which are as defined above and are each derived from a fungus and two polypeptides, which are as defined above and are each derived from a bacterium;
- three polypeptides, which are as defined above and are each derived from a fungus and three polypeptides, which are as defined above and are each derived from a bacterium.

In embodiments relating to specific combinations of polypeptides, the composition comprises a polypeptide, which has phospholipase C activity, is derived from a fungus and is selected from the group consisting of:

(a) a polypeptide having at least 60% sequence identity to the mature polypeptide of SEQ ID NO: 2,

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 1, (ii) the genomic DNA sequence comprising the mature polypeptide coding sequence of any one of SEQ ID NO: 1, or (iii) the full-length complementary strand of (i) or (ii);

(c) a polypeptide encoded by a polynucleotide having at least 60% sequence identity to the mature polypeptide coding sequence of SEQ ID NO: 1;

(d) a variant of the mature polypeptide of SEQ ID NO: 2, comprising a substitution, deletion, and/or insertion at one or more positions; and

(e) a fragment of the polypeptide of (a), (b), (c), or (d);
and

a polypeptide, which has phospholipase C activity, is derived from a bacterium and is selected from the group consisting of:

(f) a polypeptide having at least 60% sequence identity to the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44 (ii) the genomic DNA sequence comprising the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44, or (iii) the full-length complementary strand of (i) or (ii);

(h) a polypeptide encoded by a polynucleotide having at least 60% sequence identity to the mature polypeptide coding sequence of any one of SEQ ID NO: 25, SEQ ID NO: 28, SEQ ID NO: 30, SEQ ID NO: 33, SEQ ID NO: 35, SEQ ID NO: 38, SEQ ID NO: 40 and SEQ ID NO: 44;

(i) a variant of the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45, comprising a substitution, deletion, and/or insertion at one or more positions; and

(j) a fragment of the polypeptide of (f), (g), (h), or (i).

In further embodiments relating to specific combinations of polypeptides, the composition comprises a polypeptide, which has phospholipase C activity, is derived from a fungus and is selected from the group consisting of:

(a) a polypeptide having at least 60% sequence identity to the mature polypeptide of SEQ ID NO: 2,

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 1, (ii) the genomic DNA sequence comprising the mature polypeptide coding sequence of any one of SEQ ID NO: 1, or (iii) the full-length complementary strand of (i) or (ii);

(c) a polypeptide encoded by a polynucleotide having at least 60% sequence identity to the mature polypeptide coding sequence of SEQ ID NO: 1;

(d) a variant of the mature polypeptide of SEQ ID NO: 2, comprising a substitution, deletion, and/or insertion at one or more positions; and

(e) a fragment of the polypeptide of (a), (b), (c), or (d);
and

a polypeptide, which has phospholipase C activity, is derived from a bacterium and is selected from the group consisting of:

(f) a polypeptide having at least 60% sequence identity to the mature polypeptide of SEQ ID NO: 39;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 38 (ii) the genomic DNA sequence comprising the mature polypeptide coding sequence of SEQ ID NO: 38, or (iii) the full-length complementary strand of (i) or (ii);

(h) a polypeptide encoded by a polynucleotide having at least 60% sequence identity to the mature polypeptide coding sequence of SEQ ID NO: 38;

(i) a variant of the mature polypeptide of SEQ ID NO: 39, comprising a substitution, deletion, and/or insertion at one or more positions; and

(j) a fragment of the polypeptide of (f), (g), (h), or (i).

In other embodiments relating to specific combinations of polypeptides, the composition comprises a polypeptide, which has phospholipase C activity, is derived from a fungus and is selected from the group consisting of:

(a) a polypeptide having at least 60% sequence identity to the mature polypeptide of SEQ ID NO: 4,

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 3, (ii) the genomic DNA sequence comprising the mature polypeptide coding sequence of any one of SEQ ID NO: 3, or (iii) the full-length complementary strand of (i) or (ii);

(c) a polypeptide encoded by a polynucleotide having at least 60% sequence identity to the mature polypeptide coding sequence of SEQ ID NO: 3;

(d) a variant of the mature polypeptide of SEQ ID NO: 4, comprising a substitution, deletion, and/or insertion at one or more positions; and

(e) a fragment of the polypeptide of (a), (b), (c), or (d);
and

a polypeptide, which has phospholipase C activity, is derived from a bacterium and is selected from the group consisting of:

(f) a polypeptide having at least 60% sequence identity to the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44 (ii) the genomic DNA sequence comprising the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44, or (iii) the full-length complementary strand of (i) or (ii);

(h) a polypeptide encoded by a polynucleotide having at least 60% sequence identity to the mature polypeptide coding sequence of any one of SEQ ID NO: 25, SEQ ID NO: 28, SEQ ID NO: 30, SEQ ID NO: 33, SEQ ID NO: 35, SEQ ID NO: 38, SEQ ID NO: 40 and SEQ ID NO: 44;

(i) a variant of the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45, comprising a substitution, deletion, and/or insertion at one or more positions; and

(j) a fragment of the polypeptide of (f), (g), (h), or (i).

In other embodiments relating to specific combinations of polypeptides, the composition comprises a polypeptide, which has phospholipase C activity, is derived from a fungus and is selected from the group consisting of:

(a) a polypeptide having at least 60% sequence identity to the mature polypeptide of SEQ ID NO: 4,

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 3, (ii) the genomic DNA sequence comprising the mature polypeptide coding sequence of any one of SEQ ID NO: 3, or (iii) the full-length complementary strand of (i) or (ii);

(c) a polypeptide encoded by a polynucleotide having at least 60% sequence identity to the mature polypeptide coding sequence of SEQ ID NO: 3;

(d) a variant of the mature polypeptide of SEQ ID NO: 4, comprising a substitution, deletion, and/or insertion at one or more positions; and

(e) a fragment of the polypeptide of (a), (b), (c), or (d);
and

a polypeptide, which has phospholipase C activity, is derived from a bacterium and is selected from the group consisting of:

(f) a polypeptide having at least 60% sequence identity to the mature polypeptide of SEQ ID NO: 39;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 38 (ii) the genomic DNA sequence comprising the mature polypeptide coding sequence of SEQ ID NO: 38, or (iii) the full-length complementary strand of (i) or (ii);

(h) a polypeptide encoded by a polynucleotide having at least 60% sequence identity to the mature polypeptide coding sequence of SEQ ID NO: 38;

(i) a variant of the mature polypeptide of SEQ ID NO: 39, comprising a substitution, deletion, and/or insertion at one or more positions; and

(j) a fragment of the polypeptide of (f), (g), (h), or (i).

In other embodiments relating to specific combinations of polypeptides, the compositions comprise a polypeptide, which has phospholipase C activity, is derived from a fungus, and is selected from the group consisting of:

(a) a polypeptide having at least 60% sequence identity to the mature polypeptide of SEQ ID NO: 7;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 5 (ii) the genomic DNA sequence comprising the mature polypeptide coding sequence of SEQ ID NO: 5, (iii) the cDNA sequence of SEQ ID NO: 5 (SEQ ID NO: 6) or (iv) the full-length complementary strand of (i), (ii) or (iii);

(c) a polypeptide encoded by a polynucleotide having at least 60% sequence identity to the mature polypeptide coding sequence of anyone of SEQ ID NO: 5;

(d) a variant of the mature polypeptide of SEQ ID NO: 7 comprising a substitution, deletion, and/or insertion at one or more positions; and

(e) a fragment of the polypeptide of (a), (b), (c), or (d);
and

a polypeptide, which has phospholipase C activity, is derived from a bacterium and is selected from the group consisting of:

(f) a polypeptide having at least 60% sequence identity to the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45;

(g) a polypeptide encoded by a polynucleotide that hybridizes under medium high stringency conditions with (i) the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44 (ii) the genomic DNA sequence comprising the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44, or (iii) the full-length complementary strand of (i) or (ii);

(h) a polypeptide encoded by a polynucleotide having at least 60% sequence identity to the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44;

(i) a variant of the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45, comprising a substitution, deletion, and/or insertion at one or more positions; and

(j) a fragment of the polypeptide of (f), (g), (h), or (i).

In other embodiments relating to specific combinations of polypeptides, the compositions comprise a polypeptide, which has phospholipase C activity, is derived from a fungus, and is selected from the group consisting of:

(a) a polypeptide having at least 60% sequence identity to the mature polypeptide of SEQ ID NO: 7;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 5 (ii) the genomic DNA sequence comprising the mature polypeptide coding sequence of SEQ ID NO: 5, (iii) the cDNA sequence of SEQ ID NO: 5 (SEQ ID NO: 6) or (iv) the full-length complementary strand of (i), (ii) or (iii);

(c) a polypeptide encoded by a polynucleotide having at least 60% sequence identity to the mature polypeptide coding sequence of anyone of SEQ ID NO: 5;

(d) a variant of the mature polypeptide of SEQ ID NO: 7 comprising a substitution, deletion, and/or insertion at one or more positions; and

(e) a fragment of the polypeptide of (a), (b), (c), or (d);
and

a polypeptide, which has phospholipase C activity, is derived from a bacterium and is selected from the group consisting of:

(f) a polypeptide having at least 60% sequence identity to the mature polypeptide of SEQ ID NO: 39;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 38 (ii) the genomic DNA sequence comprising the mature polypeptide coding sequence of SEQ ID NO: 38, or (iii) the full-length complementary strand of (i) or (ii);

(h) a polypeptide encoded by a polynucleotide having at least 60% sequence identity to the mature polypeptide coding sequence of SEQ ID NO: 38;

(i) a variant of the mature polypeptide of SEQ ID NO: 39, comprising a substitution, deletion, and/or insertion at one or more positions; and

(j) a fragment of the polypeptide of (f), (g), (h), or (i).

In still other embodiments relating to specific combinations of polypeptides, the composition comprises a polypeptide which has phospholipase C activity, is derived from a fungus and is selected from the group consisting of:

(a) a polypeptide having at least 60% sequence identity to the mature polypeptide of SEQ ID NO: 7;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 5 (ii) the genomic DNA sequence comprising the mature polypeptide coding sequence of SEQ ID NO: 5, (iii) the cDNA sequence of SEQ ID NO: 5 (SEQ ID NO: 6) or (iv) the full-length complementary strand of (i), (ii) or (iii);

(c) a polypeptide encoded by a polynucleotide having at least 60% sequence identity to the mature polypeptide coding sequence of anyone of SEQ ID NO: 5;

(d) a variant of the mature polypeptide of SEQ ID NO: 7 comprising a substitution, deletion, and/or insertion at one or more positions; and

(e) a fragment of the polypeptide of (a), (b), (c), or (d);
and

a polypeptide, which has phospholipase C activity, is derived from a bacterium and is selected from the group consisting of:

(f) a polypeptide having at least 60% sequence identity to the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42, (ii) the genomic DNA sequence comprising the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42, or (iii) the full-length complementary strand of (i) or (ii);

(h) a polypeptide encoded by a polynucleotide having at least 60% sequence identity to the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42;

(i) a variant of the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43, comprising a substitution, deletion, and/or insertion at one or more positions; and

(j) a fragment of the polypeptide of (f), (g), (h), or (i);
and

a polypeptide, which has phospholipase C activity, is derived from a bacterium and is selected from the group consisting of:

(k) a polypeptide having at least 60% sequence identity to the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45;

(l) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44 (ii) the genomic DNA sequence comprising the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44, or (iii) the full-length complementary strand of (i) or (ii);

(m) a polypeptide encoded by a polynucleotide having at least 60% sequence identity to the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44;

(n) a variant of the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45, comprising a substitution, deletion, and/or insertion at one or more positions; and

(o) a fragment of the polypeptide of (k), (l), (m), or (n).

In still other embodiments relating to specific combinations of polypeptides, the composition comprises a polypeptide which has phospholipase C activity, is derived from a fungus and is selected from the group consisting of:

(a) a polypeptide having at least 60% sequence identity to the mature polypeptide of SEQ ID NO: 7;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 5 (ii) the genomic DNA sequence comprising the mature polypeptide coding sequence of SEQ ID NO: 5, (iii) the cDNA sequence of SEQ ID NO: 5 (SEQ ID NO: 6) or (iv) the full-length complementary strand of (i), (ii) or (iii);

(c) a polypeptide encoded by a polynucleotide having at least 60% sequence identity to the mature polypeptide coding sequence of anyone of SEQ ID NO: 5;

(d) a variant of the mature polypeptide of SEQ ID NO: 7 comprising a substitution, deletion, and/or insertion at one or more positions; and

(e) a fragment of the polypeptide of (a), (b), (c), or (d);
and

a polypeptide, which has phospholipase C activity, is derived from a bacterium and is selected from the group consisting of:

(f) a polypeptide having at least 60% sequence identity to the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42, (ii) the genomic DNA sequence comprising the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42, or (iii) the full-length complementary strand of (i) or (ii);

(h) a polypeptide encoded by a polynucleotide having at least 60% sequence identity to the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42;

(i) a variant of the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43, comprising a substitution, deletion, and/or insertion at one or more positions; and

(j) a fragment of the polypeptide of (f), (g), (h), or (i);
and

a polypeptide, which has phospholipase C activity, is derived from a bacterium and is selected from the group consisting of:

(k) a polypeptide having at least 60% sequence identity to the mature polypeptide of SEQ ID NO: 39;

(l) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 38 (ii) the genomic DNA sequence comprising the mature polypeptide coding sequence of SEQ ID NO: 38, or (iii) the full-length complementary strand of (i) or (ii);

(m) a polypeptide encoded by a polynucleotide having at least 60% sequence identity to the mature polypeptide coding sequence of SEQ ID NO: 38;

(n) a variant of the mature polypeptide of SEQ ID NO: 39, comprising a substitution, deletion, and/or insertion at one or more positions; and

(o) a fragment of the polypeptide of (k), (l), (m), or (n).

In even further embodiments relating to specific combinations of polypeptides, the composition comprises a polypeptide, which has phospholipase C activity and is derived from a fungus, said polypeptide being selected from the group consisting of:

(a) a polypeptide having at least 60% sequence identity to the mature polypeptide of any one of SEQ ID NO: 2,

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 1, (ii) the genomic DNA sequence comprising the mature polypeptide coding sequence of any one of SEQ ID NO: 1, or (iii) the full-length complementary strand of (i) or (ii);

(c) a polypeptide encoded by a polynucleotide having at least 60% sequence identity to the mature polypeptide coding sequence of SEQ ID NO: 1;

(d) a variant of the mature polypeptide of SEQ ID NO: 2, comprising a substitution, deletion, and/or insertion at one or more positions; and

(e) a fragment of the polypeptide of (a), (b), (c), or (d); and

a polypeptide, which has phospholipase C activity, is derived from a bacterium, and is selected from the group consisting of:

(f) a polypeptide having at least 60% sequence identity to the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42, (ii) the genomic DNA sequence comprising the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42, or (iii) the full-length complementary strand of (i) or (ii);

(h) a polypeptide encoded by a polynucleotide having at least 60% sequence identity to the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42;

(i) a variant of the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43, comprising a substitution, deletion, and/or insertion at one or more positions; and

(j) a fragment of the polypeptide of (f), (g), (h), or (i); and

a polypeptide, which has phospholipase C activity, is derived from a bacterium is selected from the group consisting of:

(k) a polypeptide having at least 60% sequence identity to the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45;

(l) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44 (ii) the genomic DNA sequence comprising the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44, or (iii) the full-length complementary strand of (i) or (ii);

(m) a polypeptide encoded by a polynucleotide having at least 60% sequence identity to the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44;

(n) a variant of the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45, comprising a substitution, deletion, and/or insertion at one or more positions; and

(o) a fragment of the polypeptide of (k), (l), (m), or (n).

Particular embodiments of the invention provides a composition, which comprises at least one polypeptide, which has phospholipase C activity and is derived from a fungus; and at least one polypeptide selected from the group consisting of:

(a) a polypeptide having at least 60% sequence identity to the mature polypeptide of any one of SEQ ID NO: 41;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 40 (ii) the genomic DNA sequence comprising the mature polypeptide coding sequence of any one of SEQ ID NO: 40, (iii) the cDNA sequence of SEQ ID NO: 40 or (iv) the full-length complementary strand of (i), (ii) or (iii);

(c) a polypeptide encoded by a polynucleotide having at least 60% sequence identity to the mature polypeptide coding sequence of SEQ ID NO: 40;

(d) a variant of the mature polypeptide of SEQ ID NO: 41 comprising a substitution, deletion, and/or insertion at one or more positions; and

(e) a fragment of the polypeptide of (a), (b), (c), or (d).

In even further embodiments relating to specific combinations of polypeptides, the composition comprises a polypeptide, which has phospholipase C activity and is derived from a fungus, said polypeptide being selected from the group consisting of:

(a) a polypeptide having at least 60% sequence identity to the mature polypeptide of any one of SEQ ID NO: 2,

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 1, (ii) the genomic DNA sequence comprising the mature polypeptide coding sequence of any one of SEQ ID NO: 1, or (iii) the full-length complementary strand of (i) or (ii);

(c) a polypeptide encoded by a polynucleotide having at least 60% sequence identity to the mature polypeptide coding sequence of SEQ ID NO: 1;

(d) a variant of the mature polypeptide of SEQ ID NO: 2, comprising a substitution, deletion, and/or insertion at one or more positions; and

(e) a fragment of the polypeptide of (a), (b), (c), or (d);
and

a polypeptide, which has phospholipase C activity, is derived from a bacterium, and is selected from the group consisting of:

(f) a polypeptide having at least 60% sequence identity to the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42, (ii) the genomic DNA sequence comprising the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42, or (iii) the full-length complementary strand of (i) or (ii);

(h) a polypeptide encoded by a polynucleotide having at least 60% sequence identity to the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42;

(i) a variant of the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43, comprising a substitution, deletion, and/or insertion at one or more positions; and

(j) a fragment of the polypeptide of (f), (g), (h), or (i);

and

a polypeptide, which has phospholipase C activity, is derived from a bacterium is selected from the group consisting of:

(k) a polypeptide having at least 60% sequence identity to the mature polypeptide of SEQ ID NO: 39;

(l) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 38 (ii) the genomic DNA sequence comprising the mature polypeptide coding sequence of SEQ ID NO: 38, or (iii) the full-length complementary strand of (i) or (ii);

(m) a polypeptide encoded by a polynucleotide having at least 60% sequence identity to the mature polypeptide coding sequence of SEQ ID NO: 38;

(n) a variant of the mature polypeptide of SEQ ID NO: 39, comprising a substitution, deletion, and/or insertion at one or more positions; and

(o) a fragment of the polypeptide of (k), (l), (m), or (n).

It will be understood that this composition may have any of the characteristics and features set forth in relation to the other compositions disclosed above.

Also, the said mature polypeptide may comprise or consists of amino acid residues 24-328 of SEQ ID NO: 41 and/or the said variant in (d) may comprise amino acid residues 56-195 of SEQ ID NO: 41.

The said fragment in item (e) above may in some embodiments comprise amino acid residues 56-195 of SEQ ID NO: 41.

It is further contemplated that the composition according to the invention comprises one or more polypeptides selected from the group consisting of a phospholipase A1, a phospholipase A2, a phospholipase B, a phospholipase D and a protease, an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, invertase, laccase, lipase, mannosidase, oxidase, pectinolytic enzyme, peptidoglutaminase, peroxidase, phytase, polyphenoloxidase, proteolytic enzyme, ribonuclease, transglutaminase, or xylanase

In relation to items (a), (f) and/or (k) as defined in each of the aspects and embodiments set forth above, the a polypeptide having at least 65% sequence identity to the mature polypeptide defined by any one of the sequence identifiers provided in item (a) of the particular embodiment, e.g., at least 70%, at least 75%, at least 80%, at least 85%, at least 90%, at least 91%, at least 92%, at least 93%, at least 94%, at least 95%, at least 96%, at least 97%, at least 98%, at least 99%, or 100%. Further, in relation to each of the above embodiments, the polypeptide may have been isolated.

According to each of the aspects and embodiments of the invention, the variants of each mature polypeptide may comprise a substitution, deletion, and/or insertion at one or more (e.g., several) positions. In an embodiment, the number of amino acid substitutions, deletions and/or insertions introduced into the mature polypeptide of SEQ ID NO: 2 is up to 10, e.g., 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

As the skilled person will know, essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for Phospholipase C activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

According to each aspect and embodiment of the invention, the polypeptide may be a hybrid polypeptide in which a region of one polypeptide is fused at the N-terminus or the C-terminus of a region of another polypeptide.

The polypeptide may be a fusion polypeptide or cleavable fusion polypeptide in which another polypeptide is fused at the N-terminus or the C-terminus of the polypeptide of the present invention. A fusion polypeptide is produced by fusing a polynucleotide encoding another polypeptide to a polynucleotide of the present invention. Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

A fusion polypeptide can further comprise a cleavage site between the two polypeptides. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

In the embodiments of the invention provided above, the polypeptide having at least 60% sequence identity to the mature polypeptide of any one of SEQ ID NO: 2, SEQ ID NO: 4, and SEQ ID NO: 7 preferably has a length of 540-640 amino acid residues, such as a length of, 550-640 amino acid residues, 560-640 amino acid residues, 570-640 amino acid residues, 540-630 amino acid residues, such as a length of, 550-630 amino acid residues, 560-630 amino acid residues, 570-630 amino acid residues, 570-620 amino acid residues, 570-610 amino acid residues, 570-600 amino acid residues 570-597 amino acid residues, 570-596 amino acid residues, 580-640 amino acid residues, 540-630 amino acid residues, such as a length of, 540-620 amino acid residues, 540-610 amino acid residues, 540-600 amino acid residues, 540-590 amino acid residues, 540-580 amino acid residues, 540-570 amino acid residues, 540-560 amino acid residues, 540-560 amino acid residues, 550-630 amino acid residues, such as a length of, 550-620 amino acid residues, 550-610 amino acid residues, 550-600 amino acid residues, 550-590 amino acid residues, 550-580 amino acid residues, 550-570 amino acid residues, 550-560 amino acid residues, 550-560 amino acid residues, 560-630 amino acid residues, such as a length of, 560-620 amino acid residues, 560-610 amino acid residues, 560-600 amino acid residues, 560-590 amino acid residues, 560-580 amino acid residues, 560-570 amino acid residues, 560-560 amino acid residues, 560-560 amino acid residues, 570-630 amino acid residues, such as a length of, 570-620 amino acid residues, 570-610 amino acid residues, 570-600 amino acid residues, 570-590 amino acid residues, 570-580 amino acid residues, 570-570 amino acid residues, 570-560 amino acid residues, 570-560 amino acid residues, 580-630 amino acid residues, 580-620 amino acid residues, 580-610 amino acid residues, 580-600 amino acid residues, 580-598 amino acid residues, 580-597 amino acid residues, 580-596 amino acid residues, 590-640 amino acid residues, 590-630 amino acid residues, 590-620 amino acid residues, 590-610 amino acid residues, 590-600 amino acid residues, 590-698 amino acid residues, 590-597 amino acid residues, 590-596 amino acid residues, 595-640 amino acid residues, 595-630 amino acid residues, 595-620 amino acid residues, 595-610 amino acid residues, 595-600 amino acid residues, 595-598 amino acid residues, 595-597 amino acid residues, or a length of 595-596 amino acid residues.

Also, in the embodiments of the invention provided above, the polypeptide having at least 60% sequence identity to the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43 preferably has a length of 280-320 amino acid residues, such as a length of 280-310 amino acid residues, 280-305 amino acid residues, 280-300 amino acid residues, 280-298 amino acid residues 280-297 amino acid residues, 280-296 amino acid residues, 285-320 amino acid residues, 285-315 amino acid residues, 285-310 amino acid residues, 285-305 amino acid residues, 285-300 amino acid residues, 285-298 amino acid residues, 285-297 amino acid residues, 285-296 amino acid residues, 290-320 amino acid residues, 290-315 amino acid residues, 290-310 amino acid residues, 290-305 amino acid residues, 290-300 amino acid residues, 290-298 amino acid residues, 290-297 amino acid residues, 290-296 amino acid residues, 295-320 amino acid residues, 295-315 amino acid residues, 295-310 amino acid residues, 295-305 amino acid residues, 295-300 amino acid residues, 295-298 amino acid residues, 255-297 amino acid residues, or a length of 295-296 amino acid residues.

Similarly, the polypeptide having at least 60% sequence identity to the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45 preferably has a length of 220-280 amino acid residues, such as a length of 220-270 amino acid residues, 220-260 amino acid residues, 220-250 amino acid residues, 220-248 amino acid residues 220-246 amino acid residues, 220-244 amino acid residues, 225-280 amino acid residues, 225-270 amino acid residues, 225-260 amino acid residues, 225-250 amino acid residues, 225-248 amino acid residues 225-246 amino acid residues, 225-244 amino acid residues, 230-280 amino acid residues, 230-270 amino acid residues, 230-260 amino acid residues, 230-250 amino acid residues, 230-248 amino acid residues, 230-246 amino acid residues, 230-244 amino acid residues, 235-280 amino acid residues, 235-270 amino acid residues, 235-260 amino acid residues, 235-250 amino acid residues, 235-248 amino acid residues 235-246 amino acid residues, 235-244 amino acid residues, 240-280 amino acid residues, 240-270 amino acid residues, 240-260 amino acid residues, 240-250 amino acid residues, 240-248 amino acid residues 240-246 amino acid residues, 240-244 amino acid residues, 242-280 amino acid residues, 242-270 amino acid residues, 242-260 amino acid residues, 242-250 amino acid residues, 242-248 amino acid residues 242-246 amino acid residues, 242-244 amino acid residues, 243-280 amino acid residues, 243-270 amino acid residues, 243-260 amino acid residues, 243-250 amino acid residues, 243-248 amino acid residues 243-246 amino acid residues, 243-244 amino acid residues,

In further embodiments of the invention the composition comprises one or more enzyme activities in addition to the said plurality of polypeptides having phospholipase C activity. In particular, the composition may comprise one or more polypeptides having phopolipase A activity. The one or more polypeptides having phospholipase A activity may in particular be an isolated polypeptide having phospholipase A activity, selected from the group consisting of:

(a) a polypeptide having at least 80% sequence identity, such as at least 85%, at least 90%, at least 95% or at least 98% sequence identity, to the mature polypeptide of SEQ ID NO: 46 or to the polypeptide of SEQ ID NO: 47;

(b) a fragment of the polypeptide of (a) that has phospholipase A activity.

The compositions according to the invention may be prepared in accordance with methods known in the art and may be in the form of a liquid or a dry composition. The compositions may be stabilized in accordance with methods known in the art.

**Uses**

The phospholipases or compositions of the invention may be applied in a process for removing phospholipids from oil, e.g. a vegetable oil, animal oil or fat, tallow, or grease; *i.e.*, in a process to reduce the phospholipid content in the oil. The degumming process is applicable to the purification of any edible oil which contains phospholipid, e.g., vegetable oil such as soybean oil, rape seed oil, or sunflower oil or any other oil mentioned under the definition of crude oils.

Applications in which the phospholipase of the invention can be used comprise i) degumming of oil, e.g. vegetable oil, or an edible vegetable oil, or in a process comprising hydrolysis of phospholipids in the gum fraction from water degumming to release entrapped triglyceride oil, ii) in a process comprising hydrolysis of phospholipids to obtain improved phospholipid emulsifiers, in particular wherein said phospholipid is lecithin, iii) in a process for improving the filterability of an aqueous solution or slurry of carbohydrate origin which contains phospholipid, iv) in a process for the extraction of oil, v) in a process for the production of an animal feed product, vi) in a process for the production of a biofuel, e.g. a biodiesel, vii) in a process for the production of a detergent product, and/or viii) in a process for making a baked product, comprising adding the phospholipase to a dough, and baking the dough to make the baked product. Hence, another aspect of the present invention provides a composition as defined in any of the aspects and embodiments above in any of claim for use in the aforementioned applications.

**Processes**

The combinations of phospholipases set forth above and the compositions of the invention may be applied in a process comprising contacting a phospholipid or lysophospholipid with a combination of fungal and bacterial PLCs, such as the combinations provided in the compositions of the invention. The phospholipases in the compositions react with the phospholipids or lysophospholipid to form monoglyceride or diglyceride and a phosphate ester or phosphoric acid.

Hence, in another aspect, the invention provides a process for hydrolysing a phospholipid or lysophospholipid, comprising contacting said phospholipid or lysophospholipid with a plurality of polypeptides having phospholipase C activity, wherein

- at least one of said polypeptides having phospholipase C activity is a polypeptide derived from a fungus; and

- at least one of said polypeptides having phospholipase C activity is a polypeptide derived from a bacterium.

According to some embodiments, the process comprises contacting said phospholipid or lysophospholipid with a polypeptide, which has phospholipase C activity, is derived from a fungus and is selected from the group consisting of:

(a) a polypeptide having at least 60% sequence identity to the mature polypeptide of any one of SEQ ID NO: 2, SEQ ID NO: 4, and SEQ ID NO: 7;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 1, SEQ ID NO: 3 and SEQ ID NO: 5 (ii) the genomic DNA sequence comprising the mature polypeptide coding sequence of any one of SEQ ID NO: 1, SEQ ID NO: 3 and SEQ ID NO: 5, (iii) the cDNA sequence of SEQ ID NO: 5 (SEQ ID NO: 6) or (iv) the full-length complementary strand of (i), (ii) or (iii);

(c) a polypeptide encoded by a polynucleotide having at least 60% sequence identity to the mature polypeptide coding sequence of anyone of SEQ ID NO: 1, SEQ ID NO: 3 and SEQ ID NO: 5;

(d) a variant of the mature polypeptide of any one of SEQ ID NO: 2, SEQ ID NO: 4, and SEQ ID NO: 7 comprising a substitution, deletion, and/or insertion at one or more positions; and

(e) a fragment of the polypeptide of (a), (b), (c), or (d).

In other embodiments, the process comprises contacting said phospholipid or lysophospholipid with a polypeptide, which has phospholipase C activity and is selected from the group consisting of:

(a) a polypeptide having at least 60% sequence identity to the mature polypeptide of SEQ ID NO: 2,

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 1, (ii) the genomic DNA sequence comprising the mature polypeptide coding sequence of SEQ ID NO: 1, or (iii) the full-length complementary strand of (i) or (ii));

(c) a polypeptide encoded by a polynucleotide having at least 60% sequence identity to the mature polypeptide coding sequence of SEQ ID NO: 1,

(d) a variant of the mature polypeptide of SEQ ID NO: 2, comprising a substitution, deletion, and/or insertion at one or more positions; and

(e) a fragment of the polypeptide of (a), (b), (c), or (d).

The process may also comprise contacting said phospholipid or lysophospholipid with a polypeptide, which has phospholipase C activity and is selected from the group consisting of:

(a) a polypeptide having at least 60% sequence identity to the mature polypeptide of SEQ ID NO: 4;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 3 (ii) the genomic DNA sequence comprising the mature polypeptide coding sequence of SEQ ID NO: 3, or (iii) the full-length complementary strand of (i) or (ii);

(c) a polypeptide encoded by a polynucleotide having at least 60% sequence identity to the mature polypeptide coding sequence of SEQ ID NO: 3;

(d) a variant of the mature polypeptide of SEQ ID NO: 4, comprising a substitution, deletion, and/or insertion at one or more positions; and

(e) a fragment of the polypeptide of (a), (b), (c), or (d).

In other embodiments the process of the Invention comprises contacting said phospholipid or lysophospholipid with a polypeptide, which has phospholipase C activity and is selected from the group consisting of:

(a) a polypeptide having at least 60% sequence identity to the mature polypeptide of SEQ ID NO: 7;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 5 (ii) the genomic DNA sequence comprising the mature polypeptide coding sequence of SEQ ID NO: 5, (iii) the cDNA sequence of SEQ ID NO: 5 (SEQ ID NO: 6) or (iv) the full-length complementary strand of (i), (ii) or (iii);

(c) a polypeptide encoded by a polynucleotide having at least 60% sequence identity to the mature polypeptide coding sequence of SEQ ID NO: 5;

(d) a variant of the mature polypeptide of SEQ ID NO: 7 comprising a substitution, deletion, and/or insertion at one or more positions; and

(e) a fragment of the polypeptide of (a), (b), (c), or (d).

According to these embodiments as well as embodiments disclosed in the following, the said mature polypeptide may in particular consist of or comprise an amino acid sequence selected from the group consisting of amino acid residues 19-643 of SEQ ID NO.: 2, amino acid residues 17-610 of SEQ ID NO.: 4, amino acid residues 20-626 of SEQ ID NO.: 7, and amino acid residues 37-626 of SEQ ID NO: 7.

In other embodiments, the mature polypeptide of SEQ ID NO: 2 comprises, consists of or consists essentially of amino acids 43-618 of SEQ ID NO: 2. In further embodiments, the mature polypeptide of SEQ ID NO: 4 comprises, consists of or consists essentially of amino acids 20-576 of SEQ ID NO: 4. In still further embodiments of the invention, the mature polypeptide of SEQ ID NO: 7 comprises, consists of or consists essentially of amino acids 37-618 of SEQ ID NO: 7.

In further embodiments, the process according to the Invention comprises contacting said phospholipid or lysophospholipid with a polypeptide, which has phospholipase C activity, and is selected from the group consisting of:

(a) a polypeptide having at least 60% sequence identity to the mature polypeptide of any one of SEQ ID NO: 9, SEQ ID NO: 12, SEQ ID NO: 15, SEQ ID NO: 18, SEQ ID NO: 21, SEQ ID NO: 24, SEQ ID NO: 26, SEQ ID NO: 29, SEQ ID NO: 31, SEQ ID NO: 34, SEQ ID NO: 36, SEQ ID NO: 39, SEQ ID NO: 41, SEQ ID NO: 43 and SEQ ID NO: 45;

(b) a polypeptide encoded by a polynucleotide that hybridizes under medium high stringency conditions with (i) the mature polypeptide coding sequence of any one of SEQ ID NO: 8, SEQ ID NO: 11, SEQ ID NO: 14, SEQ ID NO: 17, SEQ ID NO: 20, SEQ ID NO: 23 SEQ ID NO: 25, SEQ ID NO: 28, SEQ ID NO: 30, SEQ ID NO: 33, SEQ ID NO: 35, SEQ ID NO: 38, SEQ ID NO: 40, SEQ ID NO: 42 and SEQ ID NO: 44 (ii) the genomic DNA sequence comprising the mature polypeptide coding sequence of any one of SEQ ID NO: 8, SEQ ID NO: 11, SEQ ID NO: 14, SEQ ID NO: 17, SEQ ID NO: 20, SEQ ID NO: 23, SEQ ID NO: 25, SEQ ID NO: 28, SEQ ID NO: 30, SEQ ID NO: 33, SEQ ID NO: 35, SEQ ID NO: 38, SEQ ID NO: 40, and SEQ ID NO: 42 and SEQ ID NO: 44 or (iii) the full-length complementary strand of (i) or (ii);

(c) a polypeptide encoded by a polynucleotide having at least 60% sequence identity to the mature polypeptide coding sequence of any one of SEQ ID NO: 8, SEQ ID NO: 11, SEQ ID NO: 14, SEQ ID NO: 17, SEQ ID NO: 20, SEQ ID NO: 23, SEQ ID NO: 25, SEQ ID NO: 28, SEQ ID NO: 30, SEQ ID NO: 33, SEQ ID NO: 35, SEQ ID NO: 38, SEQ ID NO: 40, SEQ ID NO: 42 and SEQ ID NO: 44;

(d) a variant of the mature polypeptide of any one of SEQ ID NO: 9, SEQ ID NO: 12, SEQ ID NO: 15, SEQ ID NO: 18, SEQ ID NO: 21, SEQ ID NO: 24, SEQ ID NO: 26, SEQ ID NO: 29, SEQ ID NO: 31, SEQ ID NO: 34, SEQ ID NO: 36, SEQ ID NO: 39, SEQ ID NO: 41, and SEQ ID NO: 43 and SEQ ID NO: 45 comprising a substitution, deletion, and/or insertion at one or more positions; and

(e) a fragment of the polypeptide of (a), (b), (c), or (d).

In still further embodiments the process according to the invention comprises contacting said phospholipid or lysophospholipid with a polypeptide, which has phospholipase C activity and is selected from the group consisting of:

(a) a polypeptide having at least 60% sequence identity to the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42 (ii) the genomic DNA sequence comprising the mature polypeptide coding sequence of any one of SEQ ID NO: 8, SEQ ID NO: 11, SEQ ID NO: 14, SEQ ID NO: 17, SEQ ID NO: 20, SEQ ID NO: 23, and SEQ ID NO: 42 or (iii) the full-length complementary strand of (i) or (ii);

(c) a polypeptide encoded by a polynucleotide having at least 60% sequence identity to the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42;

(d) a variant of the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43, comprising a substitution, deletion, and/or insertion at one or more positions; and

(e) a fragment of the polypeptide of (a), (b), (c), or (d).

The process may also, according to other embodiments, comprise contacting said phospholipid or lysophospholipid with a polypeptide, which has phospholipase C activity and is selected from the group consisting of:

(a) a polypeptide having at least 60% sequence identity to the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45;
(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44, (ii) the genomic DNA sequence comprising the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44, or (iii) the full-length complementary strand of (i) or (ii);
(c) a polypeptide encoded by a polynucleotide having at least 60% sequence identity to the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44;
(d) a variant of the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45, comprising a substitution, deletion, and/or insertion at one or more positions; and
(e) a fragment of the polypeptide of (a), (b), (c), or (d).

The process according to the invention may in further embodiments comprise contacting said phospholipid or lysophospholipid with a polypeptide, which has phospholipase C activity and is selected from the group consisting of:

(a) a polypeptide having at least 60% sequence identity to the mature polypeptide of SEQ ID NO: 41;
(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 40 (ii) the genomic DNA sequence comprising the mature polypeptide coding sequence of SEQ ID NO: 40, or (iii) the full-length complementary strand of (i) or (ii);
(c) a polypeptide encoded by a polynucleotide having at least 60% sequence identity to the mature polypeptide coding sequence of SEQ ID NO: 40;
(d) a variant of the mature polypeptide of SEQ ID NO: 41, comprising a substitution, deletion, and/or insertion at one or more positions; and
(e) a fragment of the polypeptide of (a), (b), (c), or (d).
(f) PURIFINE® PLC, which is commercially available from DSM.

Variants of the mature polypeptide of SEQ ID NO 41 and of PURIFINE® PLC are disclosed in international patent publication WO 2011/0146812. In particular, the variants of SEQ ID NO 41 may have one or more of the amino acid changes or substitutions described in Table 12 (page 210), Table 13 (pages 211-18), Table 14 (pages 220-21) and/or table 15 (pages 222-23) in the said WO-publication.

In relation to the embodiments disclosed above, the said mature polypeptide may consist of, consist essentially of or comprise an amino acid sequence selected from the group consisting of amino acid residues 23-322 of SEQ ID NO: 9, amino acid residues 24-322 of SEQ ID NO: 9, amino acid residues 25-322 of SEQ ID NO: 9, amino acid residues 26-322 of SEQ ID NO: 9, amino acid residues 27-322 of SEQ ID NO: 9, amino acid residues 28-322 of SEQ ID NO: 9, the amino acid sequence of SEQ ID NO: 10, amino acid residues 26-323 of SEQ ID NO: 12, the amino acid sequence of SEQ ID NO: 13, amino acid residues 26-323 of SEQ ID NO: 15, the amino acid sequence of SEQ ID NO: 16, amino acid residues 29-323 of SEQ ID NO: 18, the amino acid sequence of SEQ ID NO: 19, amino acid residues 26-322 of SEQ ID NO: 21, the amino acid sequence of SEQ ID NO: 22, amino acid residues 26-322 of SEQ ID NO: 24, amino acid residues 34-278 of SEQ ID NO: 26, the amino acid sequence of SEQ ID NO: 27, amino acid residues 29-283 of SEQ ID NO: 29, amino acid residues 25-283 of SEQ ID NO: 31, the amino acid sequence of SEQ ID NO: 32, amino acid residues 25-283 of SEQ ID NO: 34, amino acid residues 28-289 of SEQ ID NO: 36, the amino acid sequence of SEQ ID NO: 37, the amino acid sequence of SEQ ID NO: 39, amino acid residues 24-328 of SEQ ID NO: 41, amino acid residues 28-339 of SEQ ID NO: 43, and amino acid residues 25-280 of SEQ ID NO: 45.

In particular, the said plurality of polypeptides having phospholipase C activity may be selected from the group consisting of:

- one polypeptide which is as defined above and is derived from a fungus and one polypeptide which is as defined above and is derived from a bacterium;
- one polypeptide which is as defined above and is derived from a fungus and two polypeptides which are as defined above and are each derived from a bacterium;
- one polypeptide which is as defined above and is derived from a fungus and three polypeptides which are as defined above and are each derived from a bacterium;
- two polypeptides which are as defined above and are each derived from a fungus and one polypeptide which is as defined above and is derived from a bacterium;
- two polypeptides which are as defined above and are each derived from a fungus and two polypeptides which are as defined above and are each derived from a bacterium;
- two polypeptides which are as defined above and are each derived from a fungus and three polypeptides which are as defined above and are each derived from a bacterium;
- three polypeptides which are as defined above and are each derived from a fungus and one polypeptide which is as defined above and is derived from a bacterium;
- three polypeptides which are as defined above and are each derived from a fungus and two polypeptides which are as defined above and are each derived from a bacterium;
- three polypeptides which are as defined above and are each derived from a fungus and three polypeptides which are as defined above and are each derived from a bacterium.

In particular embodiments, the process according to the invention comprises contacting said phospholipid or lysophospholipid with a polypeptide, which has phospholipase C activity, and is selected from the group consisting of:

(a) a polypeptide having at least 60% sequence identity to the mature polypeptide of SEQ ID NO: 2,

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 1, (ii) the genomic DNA sequence comprising the mature polypeptide coding sequence of any one of SEQ ID NO: 1, or (iii) the full-length complementary strand of (i) or (ii);

(c) a polypeptide encoded by a polynucleotide having at least 60% sequence identity to the mature polypeptide coding sequence of SEQ ID NO: 1;

(d) a variant of the mature polypeptide of SEQ ID NO: 2, comprising a substitution, deletion, and/or insertion at one or more positions; and

(e) a fragment of the polypeptide of (a), (b), (c), or (d);

and
with a polypeptide, which has phospholipase C activity and is selected from the group consisting of:

(f) a polypeptide having at least 60% sequence identity to the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44 (ii) the genomic DNA sequence comprising the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44, or (iii) the full-length complementary strand of (i) or (ii);

(h) a polypeptide encoded by a polynucleotide having at least 60% sequence identity to the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 440;

(i) a variant of the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45, comprising a substitution, deletion, and/or insertion at one or more positions; and

(j) a fragment of the polypeptide of (f), (g), (h), or (i).

In further embodiments the process according to the invention comprises contacting said phospholipid or lysophospholipid with a polypeptide, which has phospholipase C activity, and is selected from the group consisting of:

(a) a polypeptide having at least 60% sequence identity to the mature polypeptide of SEQ ID NO: 2,

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 1, (ii) the genomic DNA sequence comprising the mature polypeptide coding sequence of any one of SEQ ID NO: 1, or (iii) the full-length complementary strand of (i) or (ii);

(c) a polypeptide encoded by a polynucleotide having at least 60% sequence identity to the mature polypeptide coding sequence of SEQ ID NO: 1;

(d) a variant of the mature polypeptide of SEQ ID NO: 2, comprising a substitution, deletion, and/or insertion at one or more positions; and

(e) a fragment of the polypeptide of (a), (b), (c), or (d);
and

with a polypeptide, which has phospholipase C activity, is derived from a bacterium and is selected from the group consisting of:

(f) a polypeptide having at least 60% sequence identity to the mature polypeptide of SEQ ID NO: 39;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 38 (ii) the genomic DNA sequence comprising the mature polypeptide coding sequence of SEQ ID NO: 38, or (iii) the full-length complementary strand of (i) or (ii);

(h) a polypeptide encoded by a polynucleotide having at least 60% sequence identity to the mature polypeptide coding sequence of SEQ ID NO: 38;

(i) a variant of the mature polypeptide of SEQ ID NO: 39, comprising a substitution, deletion, and/or insertion at one or more positions; and

(j) a fragment of the polypeptide of (f), (g), (h), or (i).

In other embodiments the process according to the invention comprises contacting said phospholipid or lysophospholipid with a polypeptide, which has phospholipase C activity, and is selected from the group consisting of:

(a) a polypeptide having at least 60% sequence identity to the mature polypeptide of SEQ ID NO: 4,

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 3, (ii) the genomic DNA sequence comprising the mature polypeptide coding sequence of any one of SEQ ID NO: 3, or (iii) the full-length complementary strand of (i) or (ii);

(c) a polypeptide encoded by a polynucleotide having at least 60% sequence identity to the mature polypeptide coding sequence of SEQ ID NO: 3;

(d) a variant of the mature polypeptide of SEQ ID NO: 4, comprising a substitution, deletion, and/or insertion at one or more positions; and

(e) a fragment of the polypeptide of (a), (b), (c), or (d);
and

with a polypeptide, which has phospholipase C activity, is derived from a bacterium and is selected from the group consisting of:

(f) a polypeptide having at least 60% sequence identity to the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44 (ii) the genomic DNA sequence comprising the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44, or (iii) the full-length complementary strand of (i) or (ii);

(h) a polypeptide encoded by a polynucleotide having at least 60% sequence identity to the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44;

(i) a variant of the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45, comprising a substitution, deletion, and/or insertion at one or more positions; and

(j) a fragment of the polypeptide of (f), (g), (h), or (i).

In other embodiments the process according to the invention comprises contacting said phospholipid or lysophospholipid with a polypeptide, which has phospholipase C activity, and is selected from the group consisting of:

(a) a polypeptide having at least 60% sequence identity to the mature polypeptide of SEQ ID NO: 4,

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 3, (ii) the genomic DNA sequence comprising the mature polypeptide coding sequence of any one of SEQ ID NO: 3, or (iii) the full-length complementary strand of (i) or (ii);

(c) a polypeptide encoded by a polynucleotide having at least 60% sequence identity to the mature polypeptide coding sequence of SEQ ID NO: 3;

(d) a variant of the mature polypeptide of SEQ ID NO: 4, comprising a substitution, deletion, and/or insertion at one or more positions; and

(e) a fragment of the polypeptide of (a), (b), (c), or (d);
and

with a polypeptide, which has phospholipase C activity, is derived from a bacterium and is selected from the group consisting of:

(f) a polypeptide having at least 60% sequence identity to the mature polypeptide of SEQ ID NO: 39;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 38 (ii) the genomic DNA sequence comprising the mature polypeptide coding sequence of SEQ ID NO: 38, or (iii) the full-length complementary strand of (i) or (ii);

(h) a polypeptide encoded by a polynucleotide having at least 60% sequence identity to the mature polypeptide coding sequence of SEQ ID NO: 38;

(i) a variant of the mature polypeptide of SEQ ID NO: 39, comprising a substitution, deletion, and/or insertion at one or more positions; and

(j) a fragment of the polypeptide of (f), (g), (h), or (i).

In still other embodiments, the process according to the invention comprises contacting said phospholipid or lysophospholipid with a polypeptide, which has phospholipase C activity and is selected from the group consisting of:

(a) a polypeptide having at least 60% sequence identity to the mature polypeptide of SEQ ID NO: 7;

(b) a polypeptide encoded by a polynucleotide that hybridizes very low stringency conditions, low stringency conditions, medium stringency conditions, medium-high stringency conditions, high stringency conditions, or very high stringency conditions with (i) the mature polypeptide coding sequence of SEQ ID NO: 5 (ii) the genomic DNA sequence comprising the mature polypeptide coding sequence of SEQ ID NO: 5, (iii) the cDNA sequence of SEQ ID NO: 5 (SEQ ID NO: 6) or (iv) the full-length complementary strand of (i), (ii) or (iii);

(c) a polypeptide encoded by a polynucleotide having at least 60% sequence identity to the mature polypeptide coding sequence of anyone of SEQ ID NO: 5;

(d) a variant of the mature polypeptide of SEQ ID NO: 7 comprising a substitution, deletion, and/or insertion at one or more positions; and

(e) a fragment of the polypeptide of (a), (b), (c), or (d);
and

with a polypeptide, which has phospholipase C activity and is selected from the group consisting of:

(f) a polypeptide having at least 60% sequence identity to the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44 (ii) the genomic DNA sequence comprising the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44, or (iii) the full-length complementary strand of (i) or (ii);

(h) a polypeptide encoded by a polynucleotide having at least 60% sequence identity to the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44;

(i) a variant of the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45, comprising a substitution, deletion, and/or insertion at one or more positions; and

(j) a fragment of the polypeptide of (f), (g), (h), or (i).

In other embodiments the process according to the invention comprises contacting said phospholipid or lysophospholipid with a polypeptide, which has phospholipase C activity and is selected from the group consisting of:

(a) a polypeptide having at least 60% sequence identity to the mature polypeptide of SEQ ID NO: 7;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 5 (ii) the genomic DNA sequence comprising the mature polypeptide coding sequence of SEQ ID NO: 5, (iii) the cDNA sequence of SEQ ID NO: 5 (SEQ ID NO: 6) or (iv) the full-length complementary strand of (i), (ii) or (iii);

(c) a polypeptide encoded by a polynucleotide having at least 60% sequence identity to the mature polypeptide coding sequence of anyone of SEQ ID NO: 5;

(d) a variant of the mature polypeptide of SEQ ID NO: 7 comprising a substitution, deletion, and/or insertion at one or more positions; and

(e) a fragment of the polypeptide of (a), (b), (c), or (d);
and

with a polypeptide, which has phospholipase C activity, is derived from a bacterium and is selected from the group consisting of:

(f) a polypeptide having at least 60% sequence identity to the mature polypeptide of SEQ ID NO: 39;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 38 (ii) the genomic DNA sequence comprising the mature polypeptide coding sequence of SEQ ID NO: 38, or (iii) the full-length complementary strand of (i) or (ii);

(h) a polypeptide encoded by a polynucleotide having at least 60% sequence identity to the mature polypeptide coding sequence of SEQ ID NO: 38;

(i) a variant of the mature polypeptide of SEQ ID NO: 39, comprising a substitution, deletion, and/or insertion at one or more positions; and

(j) a fragment of the polypeptide of (f), (g), (h), or (i).

In even further embodiments the process according to the invention comprises contacting said phospholipid or lysophospholipid with a polypeptide, which has phospholipase C activity and is selected from the group consisting of:

(a) a polypeptide having at least 60% sequence identity to the mature polypeptide of SEQ ID NO: 7;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 5 (ii) the genomic DNA sequence comprising the mature polypeptide coding sequence of SEQ ID NO: 5, (iii) the cDNA sequence of SEQ ID NO: 5 (SEQ ID NO: 6) or (iv) the full-length complementary strand of (i), (ii) or (iii);

(c) a polypeptide encoded by a polynucleotide having at least 60% sequence identity to the mature polypeptide coding sequence of SEQ ID NO: 5;

(d) a variant of the mature polypeptide of SEQ ID NO: 7 comprising a substitution, deletion, and/or insertion at one or more positions; and

(e) a fragment of the polypeptide of (a), (b), (c), or (d);
and

with a polypeptide, which has phospholipase C activity and is selected from the group consisting of:

(f) a polypeptide having at least 60% sequence identity to the mature polypeptide of any one of SEQ ID NO: 9, SEQ ID NO: 12, SEQ ID NO: 15, SEQ ID NO: 18, SEQ ID NO: 21 SEQ ID NO: 24 and SEQ ID NO: 43;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42, (ii) the genomic DNA sequence comprising the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42, or (iii) the full-length complementary strand of (i) or (ii);

(h) a polypeptide encoded by a polynucleotide having at least 60% sequence identity to the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 44;

(i) a variant of the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 45, comprising a substitution, deletion, and/or insertion at one or more positions; and

(j) a fragment of the polypeptide of (f), (g), (h), or (i);
and

with a polypeptide, which has phospholipase C activity and is selected from the group consisting of:

(k) a polypeptide having at least 60% sequence identity to the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45;

(l) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44 (ii) the genomic DNA sequence comprising the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44, or (iii) the full-length complementary strand of (i) or (ii);

(m) a polypeptide encoded by a polynucleotide having at least 60% sequence identity to the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44;

(n) a variant of the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45, comprising a substitution, deletion, and/or insertion at one or more positions; and

(o) a fragment of the polypeptide of (k), (l), (m), or (n).

In still other embodiments the process according to the invention comprises contacting said phospholipid or lysophospholipid with a polypeptide, which has phospholipase C activity and is selected from the group consisting of:

(a) a polypeptide having at least 60% sequence identity to the mature polypeptide of SEQ ID NO: 7;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 5 (ii) the genomic DNA sequence comprising the mature polypeptide coding sequence of SEQ ID NO: 5, (iii) the cDNA sequence of SEQ ID NO: 5 (SEQ ID NO: 6) or (iv) the full-length complementary strand of (i), (ii) or (iii);

(c) a polypeptide encoded by a polynucleotide having at least 60% sequence identity to the mature polypeptide coding sequence of anyone of SEQ ID NO: 5;

(d) a variant of the mature polypeptide of SEQ ID NO: 7 comprising a substitution, deletion, and/or insertion at one or more positions; and

(e) a fragment of the polypeptide of (a), (b), (c), or (d);
and

a polypeptide, which has phospholipase C activity, is derived from a bacterium and is selected from the group consisting of:

(f) a polypeptide having at least 60% sequence identity to the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42, (ii) the genomic DNA sequence comprising the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42, or (iii) the full-length complementary strand of (i) or (ii);

(h) a polypeptide encoded by a polynucleotide having at least 60% sequence identity to the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42;

(i) a variant of the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 45, comprising a substitution, deletion, and/or insertion at one or more positions; and

(j) a fragment of the polypeptide of (f), (g), (h), or (i);
and

a polypeptide, which has phospholipase C activity, is derived from a bacterium and is selected from the group consisting of:

(k) a polypeptide having at least 60% sequence identity to the mature polypeptide of SEQ ID NO: 39;

(l) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 38 (ii) the genomic DNA sequence comprising the mature polypeptide coding sequence of SEQ ID NO: 38, or (iii) the full-length complementary strand of (i) or (ii);

(m) a polypeptide encoded by a polynucleotide having at least 60% sequence identity to the mature polypeptide coding sequence of SEQ ID NO: 40;

(n) a variant of the mature polypeptide of SEQ ID NO: 39, comprising a substitution, deletion, and/or insertion at one or more positions; and

(o) a fragment of the polypeptide of (k), (l), (m), or (n).

According to some embodiments the invention also provides a process comprising contacting said phospholipid or lysophospholipid with a polypeptide, which has phospholipase C activity and is selected from the group consisting of:

(a) a polypeptide having at least 60% sequence identity to the mature polypeptide of any one of SEQ ID NO: 2,

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 1, (ii) the genomic DNA sequence comprising the mature polypeptide coding sequence of any one of SEQ ID NO: 1, or (iii) the full-length complementary strand of (i) or (ii);

(c) a polypeptide encoded by a polynucleotide having at least 60% sequence identity to the mature polypeptide coding sequence of SEQ ID NO: 1;

(d) a variant of the mature polypeptide of SEQ ID NO: 2, comprising a substitution, deletion, and/or insertion at one or more positions; and

(e) a fragment of the polypeptide of (a), (b), (c), or (d);

and
with a polypeptide, which has phosplipase C activity and is derived from a bacterium, said polypeptide being selected from the group consisting of:

(f) a polypeptide having at least 60% sequence identity to the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42, (ii) the genomic DNA sequence comprising the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42, or (iii) the full-length complementary strand of (i) or (ii);

(h) a polypeptide encoded by a polynucleotide having at least 60% sequence identity to the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42;

(i) a variant of the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43, comprising a substitution, deletion, and/or insertion at one or more positions; and

(j) a fragment of the polypeptide of (f), (g), (h), or (i);
and

with a polypeptide, which has phospholipase C activity and is derived from a bacterium is selected from the group consisting of:

(k) a polypeptide having at least 60% sequence identity to the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45;

(l) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44 (ii) the genomic DNA sequence comprising the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44, or (iii) the full-length complementary strand of (i) or (ii);

(m) a polypeptide encoded by a polynucleotide having at least 60% sequence identity to the mature polypeptide coding sequence of any one of SEQ ID NO: 25, SEQ ID NO: 28, SEQ ID NO: 30, SEQ ID NO: 33, SEQ ID NO: 35, SEQ ID NO: 38 and SEQ ID NO: 44;

(n) a variant of the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45, comprising a substitution, deletion, and/or insertion at one or more positions; and

(o) a fragment of the polypeptide of (k), (l), (m), or (n).

In even further embodiments the invention provides a process comprising contacting said phospholipid or lysophospholipid with a polypeptide, which has phospholipase C activity and is selected from the group consisting of

(a) a polypeptide having at least 60% sequence identity to the mature polypeptide of any one of SEQ ID NO: 2,

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 1, (ii) the genomic DNA sequence comprising the mature polypeptide coding sequence of any one of SEQ ID NO: 1, or (iii) the full-length complementary strand of (i) or (ii);

(c) a polypeptide encoded by a polynucleotide having at least 60% sequence identity to the mature polypeptide coding sequence of SEQ ID NO: 1;

(d) a variant of the mature polypeptide of SEQ ID NO: 2, comprising a substitution, deletion, and/or insertion at one or more positions; and

(e) a fragment of the polypeptide of (a), (b), (c), or (d);
and

a polypeptide, which has phospholipase C activity, is derived from a bacterium, and is selected from the group consisting of:

(f) a polypeptide having at least 60% sequence identity to the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43;

(g) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42, (ii) the genomic DNA sequence comprising the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42, or (iii) the full-length complementary strand of (i) or (ii);

(h) a polypeptide encoded by a polynucleotide having at least 60% sequence identity to the mature polypeptide coding sequence of any one of SEQ ID NO: 8, SEQ ID NO: 11, SEQ ID NO: 14, SEQ ID NO: 17, SEQ ID NO: 20, SEQ ID NO: 23 and SEQ ID NO: 42;

(i) a variant of the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43, comprising a substitution, deletion, and/or insertion at one or more positions; and

(j) a fragment of the polypeptide of (f), (g), (h), or (i);

and
a polypeptide, which has phospholipase C activity, is derived from a bacterium is selected from the group consisting of:

(k) a polypeptide having at least 60% sequence identity to the mature polypeptide of SEQ ID NO: 39;

(l) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 38 (ii) the genomic DNA sequence comprising the mature polypeptide coding sequence of SEQ ID NO: 38, or (iii) the full-length complementary strand of (i) or (ii);

(m) a polypeptide encoded by a polynucleotide having at least 60% sequence identity to the mature polypeptide coding sequence of SEQ ID NO: 38;

(n) a variant of the mature polypeptide of SEQ ID NO: 39, comprising a substitution, deletion, and/or insertion at one or more positions; and

(o) a fragment of the polypeptide of (k), (l), (m), or (n).

A further aspect of the invention provides a process for hydrolysing a phospholipid or lysophospholipid, comprising contacting said phospholipid or lysophospholipid with at least one polypeptide, which has phospholipase C activity and is derived from a fungus; and at least one polypeptide, which has phospholipase C activity, and is selected from the group consisting of:

(a) a polypeptide having at least 60% sequence identity to the mature polypeptide of any one of SEQ ID NO: 41;

(b) a polypeptide encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 40 (ii) the genomic DNA sequence comprising the mature polypeptide coding sequence of any one of SEQ ID NO: 40, (iii) the cDNA sequence of SEQ ID NO: 40 or (iv) the full-length complementary strand of (i), (ii) or (iii);

(c) a polypeptide encoded by a polynucleotide having at least 60% sequence identity to the mature polypeptide coding sequence of SEQ ID NO: 40;

(d) a variant of the mature polypeptide of SEQ ID NO: 41 comprising a substitution, deletion, and/or insertion at one or more positions; and

(e) a fragment of the polypeptide of (a), (b), (c), or (d).

(f) PURIFINE® PLC, which is commercially available from DSM

As mentioned above, variants of the mature polypeptide of SEQ ID NO 41 and of PURIFINE® PLC are disclosed in international patent publication WO 2011/0146812. In particular, the variants of SEQ ID NO 41 may have one or more of the amino acid changes or substitutions described in Table 12 (page 210), Table 13 (pages 211-18), Table 14 (pages 220-21) and/or table 15 (pages 222-23) in the said WO-publication.

In particular embodiments of the process, the said mature polypeptide comprises or consists of amino acid residues 24-328 of SEQ ID NO: 41 and/or the said variant in (d) comprises amino acid residues 56-195 of SEQ IN NO: 41 and/or the said fragment in (e) comprises amino acid residues 56-195 of SEQ ID NO: 41.

Also, in the embodiments of the invention provided above, the polypeptide having at least 60% sequence identity to the mature polypeptide of any one of SEQ ID NO: 9, SEQ ID NO: 12, SEQ ID NO: 15, SEQ ID NO: 18, SEQ ID NO: 21, SEQ ID NO: 24 and SEQ ID NO: 43 preferably has a length of 280-320 amino acid residues, such as a length of 280-310 amino acid residues, 280-305 amino acid residues, 280-300 amino acid residues, 280-298 amino acid residues 280-297 amino acid residues, 280-296 amino acid residues, 285-320 amino acid residues, 285-315 amino acid residues, 285-310 amino acid residues, 285-305 amino acid residues, 285-300 amino acid residues, 285-298 amino acid residues, 285-297 amino acid residues, 285-296 amino acid residues, 290-320 amino acid residues, 290-315 amino acid residues, 290-310 amino acid residues, 290-305 amino acid residues, 290-300 amino acid residues, 290-298 amino acid residues, 290-297 amino acid residues, 290-296 amino acid residues, 295-320 amino acid residues, 295-315 amino acid residues, 295-310 amino acid residues, 295-305 amino acid residues, 295-300 amino acid residues, 295-298 amino acid residues, 255-297 amino acid residues, or a length of 295-296 amino acid residues.

Similarly, the polypeptide having at least 60% sequence identity to the mature polypeptide of any one of SEQ ID NO: 26, SEQ ID NO: 29, SEQ ID NO: 31, SEQ ID NO: 34, SEQ ID NO: 36, SEQ ID NO: 39 and SEQ ID NO: 45 preferably has a length of 220-280 amino acid residues, such as a length of 220-270 amino acid residues, 220-260 amino acid residues, 220-250 amino acid residues, 220-248 amino acid residues 220-246 amino acid residues, 220-244 amino acid residues, 225-280 amino acid residues, 225-270 amino acid residues, 225-260 amino acid residues, 225-250 amino acid residues, 225-248 amino acid residues 225-246 amino acid residues, 225-244 amino acid residues, 230-280 amino acid residues, 230-270 amino acid residues, 230-260 amino acid residues, 230-250 amino acid residues, 230-248 amino acid residues, 230-246 amino acid residues, 230-244 amino acid residues, 235-280 amino acid residues, 235-270 amino acid residues, 235-260 amino acid residues, 235-250 amino acid residues, 235-248 amino acid residues 235-246 amino acid residues, 235-244 amino acid residues, 240-280 amino acid residues, 240-270 amino acid residues, 240-260 amino acid residues, 240-250 amino acid residues, 240-248 amino acid residues 240-246 amino acid residues, 240-244 amino acid residues, 242-280 amino acid residues, 242-270 amino acid residues, 242-260 amino acid residues, 242-250 amino acid residues, 242-248 amino acid residues 242-246 amino acid residues, 242-244 amino acid residues, 243-280 amino acid residues, 243-270 amino acid residues, 243-260 amino acid residues, 243-250 amino acid residues, 243-248 amino acid residues 243-246 amino acid residues, 243-244 amino acid residues,

It will be understood that the process according to this aspect of the invention may have any of the characteristics and features set forth in relation to the processes of the other aspects of the invention provided above. Likewise, it will be understood that the plurality of polypeptides having Phospholipase C activity used in the process according to this aspect of the invention may be as described for the pluralities of polypeptides having Phospholipase C activity the processes of the other aspects of the invention provided above.As the skilled person will also realize the process of the invention may comprise contacting a phospholipid or lysophospholipid with at least one of the polypeptides which has phospholipase C activity and is derived from a fungus; and at least one polypeptide which has phospholipase C activity and is derived from a bacterium, either simultaneous or in sequence.

When the contacting is done simultaneously, the polypeptides may be in a composition as described above, or may be added individually at the same time. When the polypeptides are added in sequence, the at least one of the polypeptides which has phospholipase C activity and is derived from a fungus may be added first and the addition of said at least one polypeptide which has phospholipase C activity and is derived from a bacterium be delayed, such as by 5 minutes - 3 hours, 10 minutes - 3 hours, 20 minutes - 3 hours, 30 minutes - 3 hours, 1 - 3 hours, 2 - 3 hours, 5 minutes - 2 hours, 10 minutes - 2 hours, 20 minutes - 2 hours, 30 minutes - 2 hours, 1 - 2 hours, 5 minutes - 1.5 hours, 10 minutes - 1.5 hours, 20 minutes - 1.5 hours, 30 minutes - 1.5 hours, 1 - 1.5 hours, 5 minutes - 1 hour, 10 minutes - 1 hour, 20 minutes - 1 hour, 30 minutes - 1 hour, 5 - 30 minutes, 10 - 30 minutes or 20 - 30 minutes.

Alternatively the at least one of the polypeptides which has phospholipase C activity and is derived from a bacterium may be added first and the addition of said at least one polypeptide which has phospholipase C activity and is derived from a fungus be delayed, such as by 5 minutes - 3 hours, 10 minutes - 3 hours, 20 minutes - 3 hours, 30 minutes - 3 hours, 1 - 3 hours, 2 - 3 hours, 5 minutes - 2 hours, 10 minutes - 2 hours, 20 minutes - 2 hours, 30 minutes - 2 hours, 1 - 2 hours, 5 minutes - 1.5 hours, 10 minutes - 1.5 hours, 20 minutes - 1.5 hours, 30 minutes - 1.5 hours, 1 - 1.5 hours, 5 minutes - 1 hour, 10 minutes - 1 hour, 20 minutes - 1 hour, 30 minutes - 1 hour, 5 - 30 minutes, 10 - 30 minutes or 20 - 30 minutes.

In further embodiments of the invention the composition comprises one or more enzyme activities in addition to the said plurality of polypeptides having phospholipase C activity. In particular, the composition may comprise one or more polypeptides having phopolipase A activity. The one or more polypeptides having phospholipase A activity may in particular be an isolated polypeptide having phospholipase A activity, selected from the group consisting of:

(a) a polypeptide having at least 80% sequence identity, such as at least 85%, at least 90%, at least 95% or at least 98% sequence identity, to the mature polypeptide of SEQ ID NO: 46 or to the polypeptide of SEQ ID NO: 47;

(b) a fragment of the polypeptide of (a) that has phospholipase A activity.

The said phospholipid may be in a composition comprising triglycerides. The composition may be an oil, such as a vegetable oil; e.g. an edible vegetable oil. The process may further comprise contacting said phospholipid or lysophospholipid or said composition comprising triglycerides, such as said oil, with the plurality of polypeptides having Phospholipase C activity under conditions sufficient for said polypeptides having phospholipase C activity to react with said phospholipid or lysophospholipid to create diglyceride and phosphate ester.

Additionally, the process according to the invention may comprise separating the phosphate ester from the triglycerides or the oil.

According to particular embodiments of the invention said phospholipid is in a composition comprising triglycerides. The composition may in particular be an oil, such as a vegetable oil; e.g. an edible vegetable oil.

PI-specific PLC converts phosphatidyl inositol (PI) to diglyceride and phosphoinositol. PC-specific PLC converts phosphatidylcholine (PC) to diglyceride and phosphocholine. PE-specific PLC converts phosphatidylethanolamine (PE) to diglyceride and phophoethanolamine. The diglyceride stays in the oil phase (improving oil yield) and the phosphorous-containing moieties separates into the aqueous phase where it is removed as a component of the heavy phase during centrifugation. The gum phase (heavy phase) may be treated further with a phospholipase or composition of the present invention to increase hydrolysis of phospholipids in the gum fraction from water degumming to release entrapped triglyceride oil. This is particular useful when the degumming process has not already applied phospholipases. Compositions or phospholipases according to the invention can be incorporated into either a water degumming or a chemical or physical oil refining process with preferably less than 10%, 9%, 8%, 7%, 6% or 5% water, even more preferably less than 4%, 3% or 2% water, preferably at 50°C or above, even more preferably at 60°C or above. In a preferred embodiment the compositions of the invention are incorporated into a water degumming process, caustic refining process or acid degumming process.

In another preferred embodiment the phospholipases of the invention are incorporated into a physical refining process applying citric acid or phosphoric acid and sodium hydroxide to facilitate hydratability of insoluble phospholipids and ensure an environment suitable for the enzyme with preferably less than 0.15% citric acid or phosphoric acid, even more preferably less than 0.1%, 0.09%, 0.08%, 0.07%, 0.06% or 0.05%; and less than 4%, 3% or 2% water, preferably at 50°C or above, even more preferably at 60°C or above.

In other embodiments the degumming process is a caustic refining process or acid degumming process

Phospholipids are commonly measured in oil as "phosphorous content" in parts per million. Table 1 sets forth the typical amounts of phospholipids present in the major oilseed crops, and the distribution of the various functional groups as a percentage of the phospholipids present in the crude oil.

Table 1: Typical levels and phospholipid distributions in the crude oil from common oilseeds

| | Soy Oil | Canola Oil | Sunflower Oil |
|---|---|---|---|
| Phosphorous (ppm) | 400-1500 | 200-900 | 300-700 |
| PC% | 12-46 | 25-40 | 29-52 |
| PE% | 8-34 | 15-25 | 17-26 |
| PA% | 17-26 | 10-20 | 15-30 |
| PI% | 2-15 | 2-25 | 11-22 |

The compositions and processes of the invention can be used to achieve a more complete degumming of high phosphorous oils, e.g. an oil with more than 200 ppm of phosphorous, preferably more than 300 ppm, 400ppm, 500 ppm, 600 ppm, 700 ppm, 800 ppm, 900 ppm, even more preferred the oil contains more than 1000 ppm phosphorous.

Preferably the oil comprises phosphatidic acid (PA), phosphatidylcholine (PC), phosphatidylethanolamine (PE) and phosphatidyl inositol (PI). Preferably the oil contains more than 50 ppm phosphorous originating from phosphatidyl inositol (PI), more preferably it contains more than 75 ppm, 100 ppm, 125 ppm PI, even more preferably it contains more than 150 ppm, most preferably it contains more than 175 ppm phosphorous originating from PI. Preferably the oil contains more than 100 ppm phosphorous originating from phosphatidylcholine (PC), more preferably it contains more than 150 ppm, 200 ppm, 250 ppm PC, even more preferably it contains more than 300 ppm, most preferably it contains more than 400 ppm phosphorous originating from PC. Preferably the oil contains more than 75 ppm phosphorus originating from phosphatidylethanolamine (PE), more preferably it contains more than 100 ppm, 125 ppm, 150 ppm PE, even more preferably it contains more than 200 ppm, most preferably it contains more than 300 ppm phosphorous originating from PE. The oil may also contain more than 5 ppm phosphorus originating from phosphatidic acid (PA), such as more than 10 ppm phosphorus originating from phosphatidic acid (PA), more than 15 ppm, 20 ppm, 25 ppm, 40 ppm, 50 ppm PE, or 75 ppm phosphorous originating from PA.

In a preferred embodiment the oil is an edible oil. More preferred, the edible oil is selected from rice bran, rapeseeds, palm, peanuts and other nuts, soybean, corn, canola, and sunflower oils. The plurality of polypeptides having Phospholipase C activity and the compositions of the invention can be used in any "degumming" procedure, including water degumming, ALCON oil degumming (e.g., for soybeans), safinco degumming, "super degumming," UF degumming, TOP degumming, uni-degumming, dry degumming and ENZYMAX™ degumming. See, for example, WO 2007/103005, US 2008/0182322, US 6,355,693, US 6,162,623, US 6,103,505, US 6,001,640, US 5,558,781 and US 5,264,367 for description of degumming processes where phospholipases of the present invention can be applied. Various "degumming" procedures incorporated by the methods of the invention are described in Bockisch, M. (1998) In Fats and Oils Handbook, The extraction of Vegetable Oils (Chapter 5), 345-5 445, AOCS Press, Champaign, Illinois. The plurality of polypeptides having Phospholipase C activity and compositions of the invention can be used in the industrial application of enzymatic degumming of triglyceride oils as described, e.g., in EP 513 709. In a further embodiment the oil is selected from crude oil, water degummed oil, caustic refined oil and acid degummed oil. The water-degumming of a crude oil or fat may be achieved by thoroughly mixing hot water and warm oil or fat having a temperature of between 50°C to 90°C for 30 to 60 minutes. This process serves to partially remove the hydratable phospholipids. Also, an acid treatment may be performed before the enzymatic degumming, where the acid used is selected from the group consisting of phosphoric acid, acetic acid, citric acid, tartaric acid, succinic acid, and mixtures thereof, in particular a treatment using citric acid or phosphoric acid are preferred. The acid treatment is preferably followed by a neutralization step to adjust the pH between about 4.0 to 7.0, more preferably from 4.5 to 6.5, preferably using NaOH or KOH. The acid treatment serves to chelate metals bound to the phospholipids hereby making a more hydratable form. Preferably, the phospholipases as described herein is added after water degumming or acid treatment of the oil. It is also possible to perform the degumming step using the plurality of polypeptides having Phospholipase C activity and/or the compositions as described herein on a crude oil or fat, i.e. an oil or fat not previously water degummed or acid treated.

In one aspect, the invention provides methods for enzymatic degumming under conditions of low water, e.g., in the range of between about 0.1 % to 20 % water or 0.5% to 10% water. In one aspect, this results in the improved separation of a heavy phase from the oil phase during centrifugation. The improved separation of these phases can result in more efficient removal of phospholipids from the oil, including both hydratable and nonhydratable phospholipids. In one aspect, this can produce a gum fraction that contains less entrained neutral oil (triglycerides), thereby improving the overall yield of oil during the degumming process. In one aspect, the plurality of polypeptides having Phospholipase C activity and the compositions of the invention are used to treat oils to reduce gum mass and increase neutral oil gain through reduced oil entrapment. In one aspect, the plurality of polypeptides having Phospholipase C activity and the compositions of the invention are used for diacylglycerol (DAG) production and to contribute to the oil phase.

In particular embodiments, the oil is contacted with 0.5-200 mg enzyme protein (EP)/Kg oil of said fungal and bacterial phospholipases; such as with 0.5-100 mg enzyme protein (EP)/Kg oil of said fungal and bacterial phospholipases, with 0.5-25 mg enzyme protein (EP)/Kg oil of said fungal and bacterial phospholipases, with 0.5-15 mg enzyme protein (EP)/Kg oil of said fungal and bacterial phospholipase. with 0.5-10 mg enzyme protein (EP)/Kg oil of said fungal and bacterial phospholipase, with 0.5-5 mg enzyme protein (EP)/Kg oil of said fungal and bacterial phospholipase, with 1-200 mg enzyme protein (EP)/Kg oil of said fungal and bacterial phospholipase, with 1-100 mg enzyme protein (EP)/Kg oil of said fungal and bacterial phospholipase, with 1-25 mg enzyme protein (EP)/Kg oil of said fungal and bacterial phospholipase, with 1-15 mg enzyme protein (EP)/Kg oil of said fungal and bacterial phospholipase, with 1-10 mg enzyme protein (EP)/Kg oil of said fungal and bacterial phospholipase, with 1-5 mg enzyme protein (EP)/Kg oil of said fungal and bacterial phospholipase, with 2-200 mg enzyme protein (EP)/Kg oil of said fungal and bacterial phospholipase, with 2-100 mg enzyme protein (EP)/Kg oil of said fungal and bacterial phospholipase, with 2-50 mg enzyme protein (EP)/Kg oil of said fungal and bacterial phospholipase, with 2-25 mg enzyme protein (EP)/Kg oil of said fungal and bacterial phospholipase, with 2-15 mg enzyme protein (EP)/Kg oil of said fungal and bacterial phospholipase, with 2-10 mg enzyme protein (EP)/Kg oil of said fungal and bacterial phospholipase, with 2-7 mg enzyme protein (EP)/Kg oil of said fungal and bacterial phospholipase, or with 2-5 mg enzyme protein (EP)/Kg oil of said fungal and bacterial phospholipase.

In preferred embodiments the oil is contacted with 5-50 mg enzyme protein (EP)/Kg oil of said fungal phospholipase; and 0.5-20 mg enzyme protein (EP)/Kg oil of said bacterial phospholipase. In further embodiments, the oil is contacted with 5-40 mg enzyme protein (EP)/Kg oil of said fungal phospholipase; and 1-15 mg enzyme protein (EP)/Kg oil of said bacterial phospholipase. In still further embodiments, the oil is contacted with 5-30 mg enzyme protein (EP)/Kg oil of said fungal phospholipase; and 1-15 mg enzyme protein (EP)/Kg oil of said bacterial phospholipase. In other embodiments, the oil is contacted with 10-30 mg enzyme protein (EP)/Kg oil of said fungal phospholipase; and 1-5 mg enzyme protein (EP)/Kg oil of said bacterial phospholipase.

The phospholipase treatment can be conducted by dispersing an aqueous solution of the phospholipase, preferably as droplets with an average diameter below 10 microM. The amount of water is preferably 0.5-5% by weight in relation to the oil. An emulsifier may optionally be added. Mechanical agitation may be applied to maintain the emulsion. Agitation may be done with a high shear mixer with a tip speed above 1400 cm/s.

In certain embodiments, a suitable oil degumming method comprises a) mixing an aqueous acid with an oil to obtain an acidic mixture having pH of about 1 to 4, b) mixing a base with the acidic mixture to obtain a reacted mixture having pH of about 6-9, and c) degumming the reacted mixture with a plurality of polypeptides having Phospholipase C activity and/or a composition of the present invention to obtain a degummed oil. In certain embodiments, mixing in steps a) and/or b) creates an emulsion that comprises an aqueous phase in average droplet size between about 15 microM to about 45 microM. In certain embodiments, mixing in steps a) and/or b) creates an emulsion that comprises at least about 60% of an aqueous phase by volume in droplet size between about 15 microM to about 45 microM in size, wherein percentage of the aqueous phase is based on the total volume of the aqueous phase. Any acid deemed suitable by one of skill in the art can be used in the methods provided herein. In certain embodiments, the acid is selected from the group consisting of phosphoric acid, acetic acid, citric acid, tartaric acid, succinic acid, and a mixture thereof. Any acid deemed suitable by one of skill in the art can be used in the methods provided herein. In certain embodiments, the base is selected from the group consisting of sodium hydroxide, potassium hydroxide, sodium silicate, sodium carbonate, calcium carbonate, and a combination thereof.

In a preferred embodiment the phospholipase treatment can be conducted at a pH in the range of about 4.0 to 7.0, more preferably from 4.5 to 6.5. The pH is measured in the emulsion or in the interphase between the oil and aqueous solution. A suitable temperature is generally 30-80°C. In a preferred embodiment the temperature of the oil is between 50 and 70°C, more preferred between 55 and 65 °C and most preferred between 50 and 60°C. In other preferred embodiments the temperature of the oil is between 60 and 80°C, more preferred between 65 and 75 °C and most preferred between 67 and 72°C

The reaction time will typically be 1-12 hours (e.g., 1-6 hours, or 1-3 hours, most preferred the reaction time is between 1.5 and 4 hours, even more preferred between 1.5 and 2 hours). A suitable enzyme dosage will usually be 0.1-10 mg per liter (e.g., 0.5-5 mg per liter). The phospholipase treatment may be conducted batch wise, e.g., in a tank with stirring, or it may be continuous, e.g., a series of stirred tank reactors. The phospholipase treatment may be followed by separation of an aqueous phase and an oil phase. The separation may be performed by conventional means, e.g., centrifugation. When a liquid lipase is used, the aqueous phase will contain phospholipase, and the enzyme may be re-used to improve the process economy.

In a preferred embodiment of the present invention the treatment reduces the total phosphorous content of the oil to 200 ppm, preferably below 100 ppm, below 50 ppm, preferably below 40 ppm, 30 ppm, 20 ppm, 15 ppm, more preferably below 10 ppm, below 9 ppm, below 8 ppm, below 7 ppm, below 6 ppm, most preferably below 5 ppm.

The compositions and processes of the invention may be used for partial hydrolysis of phospholipids, preferably lecithin, to obtain improved phospholipid emulsifiers. This application is further described in Ullmann's Encyclopedia of Industrial Chemistry (Publisher: VCH Weinheim (1996)), JP patent 2794574, and JP-B 6-087751.

The compositions and processes of the invention can be used to improve the filterability of an aqueous solution or slurry of carbohydrate origin by treating it with the phospholipase. This is particularly applicable to a solution of slurry containing a starch hydrolyzate, especially a wheat starch hydrolyzate, since this tends to be difficult to filter and to give cloudy filtrates. The treatment can be done in analogy with EP 219,269 (CPC International).

The compositions and processes of the invention may be used in a process for the production of an animal feed which comprises mixing the phospholipase with feed substances comprising at least one phospholipid. This can be done in analogy with EP 743 017.

The plurality of polypeptides having Phospholipase C activity and the compositions of the present invention may be used in combination with one or more lipolytic enzymes to convert fats and oils to fatty acid alkyl esters while achieving degumming in the same process. Such a process is for example described in US 8,012,724.

The plurality of polypeptides having Phospholipase C activity and the compositions the invention may be added to and thus be used as a component of a detergent composition. The detergent composition may for example be formulated as a hand or machine laundry detergent composition including a laundry additive composition suitable for pre-treatment of stained fabrics and a rinse added fabric softener composition, or be formulated as a detergent composition for use in general household hard surface cleaning operations, or be formulated for hand or machine dishwashing operations.

The plurality of polypeptides having Phospholipase C activity and the compositions of the invention may be used for production of dough and baked products from dough, as well as for production of baking compositions and baking additives. The dough generally comprises wheat meal or wheat flour and/or other types of meal, flour or starch such as corn flour, corn starch, rye meal, rye flour, oat flour, oat meal, soy flour, sorghum meal, sorghum flour, potato meal, potato flour or potato starch. The dough may be fresh, frozen or par-baked. The dough is normally leavened dough or dough to be subjected to leavening. The dough may be leavened in various ways, such as by adding chemical leavening agents, e.g., sodium bicarbonate or by adding a leaven (fermenting dough), but it is preferred to leaven the dough by adding a suitable yeast culture, such as a culture of *Saccharomyces cerevisiae* (baker's yeast), e.g. a commercially available strain of *S. cerevisiae.* The dough may also comprise other conventional dough ingredients, e.g.: proteins, such as milk powder, gluten, and soy; eggs (either whole eggs, egg yolks or egg whites); an oxidant such as ascorbic acid, potassium bromate, potassium iodate, azodicarbonamide (ADA) or ammonium persulfate; an amino acid such as L-cysteine; a sugar; a salt such as sodium chloride, calcium acetate, sodium sulfate or calcium sulfate. The dough may comprise fat (triglyceride) such as granulated fat or shortening, but the invention is particularly applicable to a dough where less than 1 % by weight of fat is added, and particularly to a dough which is made without addition of fat. The dough may further comprise an emulsifier such as mono- or diglycerides, diacetyl tartaric acid esters of mono- or diglycerides, sugar esters of fatty acids, polyglycerol esters of fatty acids, lactic acid esters of monoglycerides, acetic acid esters of monoglycerides, polyoxyethylene stearates, or lysolecithin. The dough may be used for any kind of baked product prepared from dough, either of a soft or a crisp character, either of a white, light or dark type. Examples are bread (in particular white, whole-meal or rye bread), typically in the form of loaves or rolls, French baguette-type bread, pita bread, tortillas, cakes, pancakes, biscuits, wafers, cookies, pie crusts, crisp bread, steamed bread, pizza and the like.

The present invention is further described by the following examples that should not be construed as limiting the scope of the invention.

## Examples

### Example 1:

### P-NMR assay of purified PLC enzymes

Concept

The assay was conducted by incubating the PLC with a 10:1 mixture of a crude vegetable oil and aqueous citrate buffer, pH 4.0, 5.5 or 7.0. Enzyme concentration was 10 mg/kg and 100 mg/kg (mg enzyme protein (EP) per kg oil). The mixture was incubated with vigorous shaking at 50 C for 2 h. The reaction mixture was then analyzed by P-NMR. This involved an aqueous extraction step during which the phosphor species liberated by the PLC are removed from the oil phase. Hence, only lipophilic P-species were detected, i.e. unreacted phospholipid.

Assay procedure

The purified enzyme was diluted to 0.9 mg/mL and 0.09 mg/mL in 100 mM citrate buffer, pH 4.0, 5.5 or 7.0. The assay was initiated by adding 25 uL diluted enzyme to 250 uL crude vegetable oil in a 2 mL Eppendorf tube and incubating the mixture in a thermoshaker at 50 C for 2h. The oil used was a crude soybean oil containing a significant amount of both PA (128 ppm P), PE (141 ppm P), PI (103 ppm P) and PC (157 ppm P). For assay of mature polypeptide of SEQ ID NO: 4 a fully refined soybean oil spiked with a mixture of PA (180 ppm P), PE (349 ppm P), PI (595 ppm P) and PC (431 ppm P) was used.

NMR analysis

To the oil sample was then added 0.500 mL internal standard (IS) solution, followed by 0.5 mL $CDCl_3$ and 0.5 mL Cs-EDTA buffer. The sample was shaken for 30 s, and then centrifuged (tabletop centrifuge, 3 min, 13,400 rpm) to get phase separation. The lower phase was transferred to a NMR-tube. P-NMR was performed with 128 scans and a delay time of 5 s. Set scale reference according to IS signal (-17.75 ppm). All signals were integrated. Assignments (approx. ppm @ 25 C): 1.7 (PA), -0.1 (PE), -0.5 (PI), -0.8 (PC). The concentration of each species was calculated as "ppm P", i.e. mg elemental P per kg oil sample. Hence, ppm P = I/I(IS) * n(IS) * M(P) / m(oil). Finally, residual phospholipid content was calculated as the ratio of enzyme treated sample vs. blank.

The internal standard solution is 2 mg/mL triphenylphosphate in methanol.

The Cs-EDTA buffer was prepared as: EDTA (5.85 g) is dispersed in water (approx. 50 mL). The pH was adjusted to 7.5 using 50% w/w CsOH. This gave a clear solution. Water was added up to 100 mL to give a concentration of 0.2 M EDTA.

Results

The tables below show residual phospholipid content in percent.

**PURIFINE® PLC, 100 mg/kg**

| | PA | PE | PI | PC |
|---|---|---|---|---|
| pH 4.0 | 107 | 107 | 91 | 114 |
| pH 5.5 | 107 | 38 | 106 | 20 |
| pH 7.0 | 89 | 0 | 94 | 0 |
| 100 mg/kg | | | | |
| | PA | PE | PI | PC |
| pH 4.0 | 106 | 106 | 98 | 105 |
| pH 5.5 | 101 | 114 | 106 | 98 |
| pH 7.0 | 112 | 18 | 102 | 0 |

**Bt-PLC/mature polypeptide of SEQ ID NO: 29 (batch A)**

| 100 mg/kg | | | | |
|---|---|---|---|---|
| | PA | PE | PI | PC |
| pH 4.0 | 94 | 102 | 91 | 94 |
| pH 5.5 | 92 | 90 | 92 | 70 |
| pH 7.0 | 98 | 19 | 100 | 7 |
| 10 mg/kg | | | | |
| | PA | PE | PI | PC |
| pH 4.0 | 100 | 101 | 92 | 92 |
| pH 5.5 | 103 | 100 | 93 | 96 |
| pH 7.0 | 112 | 54 | 103 | 13 |

**Bt-PLC/mature polypeptide of SEQ ID NO: 29 (batch B)**

| 100 mg/kg | | | | |
|---|---|---|---|---|
| | PA | PE | PI | PC |
| pH 4.0 | n.d. | n.d. | n.d. | n.d. |
| pH 5.5 | n.d. | n.d. | n.d. | n.d. |
| pH 7.0 | 61 | 0 | 90 | 0 |
| 10 mg/kg | | | | |
| | PA | PE | PI | PC |
| pH 4.0 | 105 | 103 | 96 | 98 |
| pH 5.5 | 95 | 90 | 99 | 61 |
| pH 7.0 | 103 | 23 | 96 | 6 |

**Mature polypeptide of SEQ ID NO: 7 (batch A)**

| 100 mg/kg | | | | |
|---|---|---|---|---|
| | PA | PE | PI | PC |
| pH 4.0 | 68 | 72 | 77 | 68 |
| pH 5.5 | 26 | 19 | 28 | 33 |
| pH 7.0 | 10 | 19 | 9 | 30 |
| 10 mg/kg | | | | |
| | PA | PE | PI | PC |
| pH 4.0 | 90 | 90 | 90 | 91 |
| pH 5.5 | 66 | 77 | 60 | 77 |
| pH 7.0 | n.d. | n.d. | n.d. | n.d. |

**Mature polypeptide of SEQ ID NO: 7 (batch B)**

| 100 mg/kg | | | | |
|---|---|---|---|---|
| | PA | PE | PI | PC |
| pH 4.0 | n.d. | n.d. | n.d. | n.d. |
| pH 5.5 | 17 | 18 | 10 | 16 |
| pH 7.0 | 7 | 0 | 0 | 11 |
| 10 mg/kg | | | | |
| | PA | PE | PI | PC |
| pH 4.0 | n.d. | n.d. | n.d. | n.d. |
| pH 5.5 | 45 | 65 | 41 | 29 |
| pH 7.0 | 31 | 65 | 23 | 63 |

**Mature polypeptide of SEQ ID NO: 2**

| 100 mg/kg | | | | |
|---|---|---|---|---|
| | PA | PE | PI | PC |
| pH 4.0 | 54 | 54 | 83 | 51 |
| pH 5.5 | 21 | 67 | 51 | 30 |
| pH 7.0 | 23 | 31 | 13 | 17 |
| 10 mg/kg | | | | |
| | PA | PE | PI | PC |
| pH 4.0 | 85 | 91 | 90 | 80 |
| pH 5.5 | 63 | 83 | 51 | 61 |
| pH 7.0 | 61 | 78 | 61 | 67 |

**Mature polypeptide of SEQ ID NO: 4**

| 100 mg/kg | | | | |
|---|---|---|---|---|
| | PA | PE | PI | PC |
| pH 4.0 | 51 | 49 | 0 | 10 |
| pH 5.5 | 38 | 47 | 0 | 12 |
| pH 7.0 | 0 | 58 | 0 | 16 |
| 10 mg/kg | | | | |
| | PA | PE | PI | PC |
| pH 4.0 | 79 | 80 | 43 | 72 |
| pH 5.5 | 94 | 93 | 55 | 64 |
| pH 7.0 | 108 | 82 | 69 | 73 |

**Example 2: Degumming assay /metal composition of crude oil**

Performance of the phospholipase C enzyme compositions of the present invention:

- Bacterial: a) Bt-PLC(mature polypeptide of SEQ ID NO: 29) and b) Mature polypeptide of SEQ ID NO: 9
- Fungal: a) Mature polypeptide of SEQ ID NO:4 and B) Mature polypeptide of SEQ ID NO: 2 and C) Mature polypeptide of SEQ ID NO: 9
- PURIFINE® PLC,

was tested in a degumming assay that mimics industrial scale degumming. The assay measured the following parameters in the oil phase after the degumming:

a) Diglyceride content by High-performance liquid chromatography (HPLC) coupled to Evaporative Light Scattering Detector (ELSD) or Charged Aerosol Detector (Corona Veo).

b) Quantification of the individual phospholipids species: Phosphatidylcholine (PC); Phosphatidylinositol (PI); Phosphatidylethanolamine (PE); Phosphatidic acid (PA); by Liquid Chromatography quadrupole mass spectrometer time of flight (LC/TOF/MS)

c) Total phosphorus reduction by Inductively coupled plasma optical emission spectrometry (ICP-OES).

The phosphorous, calcium, magnesium and zink composition as well as the phospholipid composition in the crude soybean oil, used in the experiments, is indicated in table 1A and 1B. The metal composition was measured by ICP-OES and individual phospholipids by LCMS.

| Table 1A: Metal composition of crude oil measured by ICP-OES (mg/kg oil) | | | | |
|---|---|---|---|---|
| | P | Ca | Mg | Zn |
| Crude oil 1 | 718 | 85 | 66 | 1 |
| Crude oil 2 | 649 | 60 | 57 | 1.2 |
| Crude oil 3 | 1030 | 110 | 93 | - |
| Crude oil 4 | 985 | 74 | 79 | 1.2 |
| Crude oil 5 | 684 | 82 | 62 | 1.0 |

| Table 1B: Phospholipid composition of crude oil (mg/kg phosphorus). | | | |
|---|---|---|---|
| | Crude oil 1 | Crude oil 2 | Crude oil 5 |
| PA | 295 | 172 | 98 |
| PE | 125 | 225 | 191 |
| PI | 84 | 210 | 89 |
| PC | 229 | 283 | 185 |
| Total | 732 | 890 | 24 |

**Degumming assay**

Crude soybean oil (75 g) was initially acid/base pretreated (or not) to facilitate conversion of insoluble phospholipis salts into more hydratable forms and ensure an environment suitable for the enzyme. Acid/base pretreatment was done by acid addition of Ortho Phosphoric acid (85% solution) applied in amounts equal to 0.05% (100% pure Ortho Phosphoric acid) based on oil amount and mixing in ultrasonic bath (BRANSON 3510) for 5 min and incubation in rotator for 15 min followed by base neutralization with 4 M NaOH applied in equivalents (from 0.5 to 1.5) to pure Ortho Phosphoric acid in ultrasonic bath for 5 min. The enzyme reaction was conducted in low aqueous system (3% water total based on oil amount) in 100 ml centrifuge tubes, cylindrical, conical bottom. Samples were ultrasonic treated for 5 min, followed by incubation in a heated cabinet at selected temperature (from 50 to 60°C) with stirring at 20 rpm for a selected incubation time (from 1 to 5 hours). To separate the mixture into an oil phase and a heavy water/gum phase the samples were centrifuged at 700 g at 85°C for 15 min (Koehler Instruments, K600X2 oil centrifuge).

**a) Diglyceride measurement**

The HPLC- method (using DIONEX equipment and Lichrocart Si-60, 5 μm, Lichrosphere 250-4mm, MERCK column) was based on the principle of the AOCS Official Method Cd 11 d-96 and quantifies the diglyceride content down to 0.1 wt%.

**b) Phosphorus/phospholipid measurement**

The ICP-OES quantifies the phosphorus (P) content and other metals such as Ca, Mg, Zn down to 4 ppm with an accuracy of approximately ± 1 ppm P.

**c) Quantitative analysis of phospholipids by LCMS/MS**

Liquid Chromatography coupled to triple quadrupole mass spectrometer (LC/MS/MS) or coupled to quadrupole mass spectrometer time of flight (LC/TOF/MS) was used to quantify the individual phospholipids species: phosphatidylcholine (PC); Phosphatidylinositol (PI); Phosphatidylethanolamine (PE) and Phosphatidic acid (phosphatidate) (PA). The sensitivity of the assay goes down to less than 1 mg Phosphorus/kg oil for PC, PE and PI (ppm) and less than 10 mg Phosphorus/kg for PA. The oil sample was dissolved in chloroform. The extract was then analysed on LC-TOF-MS (or on LC-MS/MS if lower detection limits are needed) using following settings:

<u>LC-settings</u>

| | |
|---|---|
| Eluent A: | 50% Acetonitril, 50% Water, 0.15% formic acid |
| Eluent B: | 100% Isopropionic acid, 0.15% formic acid |
| Run time: | 26.9 min |
| Flow: | 0.50 mL/min |
| Column temperature: | 50°C |
| Autosampler temp: | 15-25°C |
| Injection volume: | 1 μL |
| Column type Material: | Charged Surface Hybrid, length: 50mm, size:1.7μm, ID: 2.1 mm |

MS-settings

| TOF/MS | MS/MS (Xevo) |
|---|---|
| Capillary: 3.50 kV<br>• Cone: 28<br>• Extractor: 2 V<br>• RF-lens: 0.5 V<br>• Source temp: 125° C<br>• Desolvation temp: 500°C<br>• Cone gas flow: 30 L/hour<br>• Desolvation gas flow: 850 L/hour | Capillary: +3.50 / -2.0 kV<br>• Cone: Component specific<br>• Extractor: 2.5 V<br>• RF-lens:<br>• Source temp: 150°C<br>• Desolvation temp: 500°C<br>• Cone gas flow: 30 L/hour<br>• Desolvation gas flow: 850 L/hour |

The data was processed using MassLynx version 4.1 Software. In the below examples the method is just termed LCMS.

Example 3 to 6 below describes results obtained using the degumming assay of this example.

**Example 3: Mature polypeptide of SEQ ID NO: 2 and PURIFINE® PLC blend.**

The mature polypeptide of SEQ ID NO: 2 applied in degumming assay at 60°C in combination with PURIFINE® PLC at various enzyme dosages (enzyme protein per kg oil) applying oil 1. The diglyceride content after enzymatic degumming for 2, 3, 4 and 5 hrs were measured (oil pretreated with 0.05% phosphoric acid/1.5 eqv. NaOH) as well as the composition of individual phospholipids after 4 hours incubation measured by LCMS. The results are presented in table 2A and 2B.

| Table 2A: Diglyceride increase (% w/w) after enzyme incubation in oil 1 (hours) | | | | | |
|---|---|---|---|---|---|
| Purifine dosage (mg EP/kg oil) | Mature p.p. of SEQ ID NO: 2 dosage (mg EP/kg oil) | 2 | 3 | 4 | 5 |
| 10 | 20 | 0.58 | 0.83 | 0.95 | 1.18 |
| 10 | 10 | 0.55 | 0.69 | 0.74 | 0.91 |
| 4 | 20 | 0.47 | 0.67 | 0.80 | 0.98 |
| 4 | 10 | 0.46 | 0.65 | 0.79 | 0.92 |
| 4 | - | 0.33 | 0.59 | 0.61 | 0.63 |

| Table 2B: Phospholipid composition measured by LCMS after 4 hours enzyme incubation (mg/kg oil) | | | | | |
|---|---|---|---|---|---|
| Purifine dosage (mg EP/kg oil) | Mature p.p. of SEQ ID NO: 2 dosage (mg EP/kg oil) | PA | PE | PI | PC |
| 4 | 10 | ≤10* | 1.6 | 0 | 0 |
| * Below PA detection limit | | | | | |

Degumming with the mature polypeptide of SEQ ID NO: 2 combined with PURIFINE® PLC results in significant diglyceride formation at 60°C and converts up to 80% of the phospholipids at conditions tested (60°C, 5 hours). Conversion calculation is based on the assumption that 732 ppm P total measured by LC/MS is equal to 1.83 wt% phospholipid (Average PL Mw ~772 g/mol, Mw P~31 g/mol) equal to max 1.46% DG increase obtainable (80% of phospholipid molecule).

Results shows less than 10 mg P/kg oil originating from PI, PC, PE, PA in the degummed oil sample and confirms that the enzyme blend attacks all phospholipid species.

**Example 4: Blend of mature polypeptide of SEQ ID NO: 2 and mature polypeptide of SEQ ID NO: 29.**

**C rude oil 2**

The mature polypeptide of SEQ ID NO: 29 applied in degumming assay at 60C in combination with the mature polypeptide of SEQ ID NO: 2 applying crude soybean oil 2 pretreated with 0.05% phosphoric acid/1.5 eqv. NaOH. The diglyceride content after enzymatic degumming for 2, 3, 4 and 5 hrs, were measured as well as phosphorous content after 5 hours degumming. The results are presented in table 3.

| Table 3: Diglyceride increase (% w/w) after enzyme reaction (hours) | | | | | | | |
|---|---|---|---|---|---|---|---|
| Purifine dosage (mg EP/kg oil) | Bacterial dosage mature p.p. of SEQ ID NO: 29 (mg EP/kg oil) | Fungal mature p.p. of SEQ ID NO: 2 dosage (mg EP/kg oil) | 2 | 3 | 4 | 5 | Phosphorous after 5 hours measured by ICP(mg/kg) |
| 10 | | | 0.90 | 0.81 | 0.89 | 0.93 | 13 |
| | | 30 | 0.15 | 0.44 | 0.49 | 0.69 | 15 |
| | 10 | | 0.73 | 0.79 | 0.90 | 0.89 | 12 |
| | 10 | 30 | 0.87 | 1.22 | 1.32 | 1.41 | 10 |

Degumming with the PE; PC specific mature polypeptide of SEQ ID NO: 29 combined with mature polypeptide of SEQ ID NO: 2 result in a combined effect at 60°C compared to individual solutions.

Full conversion is achieved after 4 hours based on the assumption that that 649 ppm P total measured by ICP is equal to 1.62 wt% phospholipid (Average PL Mw ~772 g/mol, Mw P~31 g/mol) equal to max 1.3% DG increase obtainable (80% of phospholipid molecule).

Phosphorous content in degummed oil was reduced to less than 10 mg/kg P total originating from PI, PC, PE, PA and confirms that the blend attack all phospholipid species.

**Example 5: Mature polypeptide of SEQ ID NO: 4 and mature polypeptide of SEQ ID NO: 29.**

The mature polypeptide of SEQ ID NO: 29 applied in degumming assay in combination with the mature polypeptide of SEQ ID NO: 4 applying crude soybean oil 3 without any acid /base pretreatment. The enzyme incubation is done at 60°C (for 1st hour) followed by incubation at 80°C (for another 23 hours). The diglyceride content after enzymatic degumming for 1, 2, 4 and 24 hrs, were measured.
The results are presented in table 3.

| Table 4: DG increase after enzyme incubation (hours) | | | | |
|---|---|---|---|---|
| | 1 | 2 | 4 | 24 |
| Mature p.p. of SEQ ID NO: 29 in B. subtilis (U1DW7) 4 mg EP | 0.47 | 0.63 | 0.67 | 1.43 |
| Mature p.p. of SEQ ID NO: 4 in T. reseii (U1DW6) 10 mg EP | 0.61 | 0.83 | 0.68 | 0.89 |
| Mature p.p. of SEQ ID NO: 29+ Mature p.p. of SEQ ID NO: 4 + 10 mg EP | 0.82 | 0.93 | 1.02 | 1.90 |

Degumming with the PE; PC specific mature polypeptide of SEQ ID NO: 29 combined with mature polypeptide of SEQ ID NO: 4 result in a combined effect compared to individual solutions. Almost full conversion (92.3%) is achieved after 24 hours based on the assumption that that 1030 ppm P total measured by ICP is equal to 2.58 wt% phospholipid (Average PL Mw ~772 g/mol, Mw P~31 g/mol) equal to max 2.06% DG increase obtainable (80% of phospholipid molecule).

**Example 6: Triple of mature polypeptide of SEQ ID NO: 9 + mature polypeptide of SEQ ID NO: 29 + mature polypeptide of SEQ ID NO: 2**

Mature polypeptide of SEQ ID NO: 2 applied in degumming assay at 60°C in combination with mature polypeptide of SEQ ID NO: 9 and mature polypeptide of SEQ ID NO: 29 applying crude oil 4. The diglyceride content after enzymatic degumming for 2, 3, 5 and 24 hrs were measured (oil pretreated with 0.05% phosphoric acid/1.5 eqv. NaOH) as well as the total phosphorous content after 24 hours.
The results are presented in table 5A and 5B.

| Enzyme and dosing (mg EP/kg oil) | Table 5A: DG increase after enzyme incubation (hours) based on double determinations with a coefficient of variation of max. 6.7%. | | | |
|---|---|---|---|---|
| | 2 | 3 | 5 | 24 |
| Mature p.p. of SEQ ID NO: 2 + mature p.p. of SEQ ID NO:29 | 0.66 | 0.68 | 0.74 | 1.04 |
| Mature p.p. of SEQ ID NO: 2 + Mature p.p. of SEQ ID NO: 29 + Mature p.p. of SEQ ID NO: 9 (10 + 4 + 2) | 0.69 | 0.76 | 0.84 | 1.17 |

Degumming with a triple PLC solution result in an improved effect compared to a double PLC solutions. Phosphorous content in degummed oil (after 24 hours incubation) was reduced to less than 3 mg/kg P total originating from PI, PC, PE, PA and confirms that the enzyme blend used in the described assay result in full removal/conversion of all phospholipid species.

**Example 7: Triple of mature polypeptide of SEQ ID NO: 9 + mature polypeptide of SEQ ID NO: 29 + mature polypeptide of SEQ ID NO: 7**

Mature polypeptide of SEQ ID NO: 7 applied in degumming assay at 60°C in combination with mature polypeptide of SEQ ID NO: 9 and mature polypeptide of SEQ ID NO: 29 applying crude oil 5. The diglyceride content after enzymatic degumming for 2, 3, and 5 hrs were measured (oil pretreated with 0.05% phosphoric acid/1.5 eqv. NaOH) as well as the total phosphorous content after 5 hours.

Table 6: Increase of diglyceride after enzyme treatment measured by HPLC-Corona Veo and phosphorous content measured by ICP after oil pre-treatment with of 0.05% phosphoric acid/1.5 eqv. NaOH.

| **Enzyme** | **Enz dosing (mg EP/kg oil)** | **Water dosing** | **DG increase after enzyme reaction (hours)** | | | **Total P after 5 h enz reaction mg/kg oil=ppm** |
|---|---|---|---|---|---|---|
| | | | 2 | 3 | 5 | |
| No enz. | - | 3% water | 0.00 | 0.00 | 0.02 | 43 |
| Seq 2+29+9 | 10 + 4 + 2 | | 0.35 | 0.40 | 0.56 | 35 |
| Seq 5+29+9 | 10 + 4 + 2 | | 0.29 | 0.34 | 0.51 | 30 |
| Seq 2+29+9 | 30 + 10 + 4 | | 0.36 | 0.44 | 0.65 | 26 |
| Seq 5+29+9 | 30 + 10 + 4 | | 0.22 | 0.28 | 0.44 | 31 |
| No enz. | | 5% water | 0.00 | 0.00 | 0.03 | 18 |
| Seq 5+29+9 | 10 + 4 + 2 | | 0.50 | 0.55 | 0.73 | 27 |
| Seq 5+29+9 | 30 + 10 + 4 | | 0.35 | 0.46 | 0.68 | 19 |

Degumming with SEQ ID NO: 5+29+9 triple PLC solution result in significant diglyceride formation (up to 0.76 wt% DG) after 2-5 hours reaction. Improved performance of SEQ ID NO: 5+29+9 in terms of extra diglyceride formation was observed when water content was increased from 3 to 5% based on oil amount.

**Example 8: Mature polypeptide of SEQ ID NO: 5 and mature polypeptide of SEQ ID NO: 29.**

The mature polypeptide of SEQ ID NO: 29 applied in degumming assay in combination with the mature polypeptide of SEQ ID NO: 5 applying crude soybean oil 5 after oil pre-treatment with 355 ppm phosphoric acid + 1.5 eqv NaOH. The enzyme incubation is done at 60°C for 5 hours.

| Enzyme | Enz dosing (mg EP/kg oil) | Water dosing | DG increase after enzyme reaction (hours) | | | Total P after 5 h enz reaction mg/kg oil=ppm |
|---|---|---|---|---|---|---|
| | | | 2 | 3 | 5 | |
| no enz-blank control | | 3% | - | - | - | 70 |
| Seq ID 5 | 10 | | 0.12 | 0.07 | 0.11 | 76 |
| Seq ID 5 + 29 | 10 + 4 | | 0.19 | 0.17 | 0.18 | 65 |
| Seq ID 5 + 29 | 30 + 10 | | 0.27 | 0.24 | 0.33 | 63 |
| no enz-blank control | - | 5% | - | - | - | 62 |
| Seq ID 5 | 10 | | 0.10 | 0.13 | 0.25 | 65 |
| Seq ID 5 + 29 | 10 + 4 | | 0.42 | 0.51 | 0.59 | 63 |
| Seq ID 5 + 29 | 30 + 10 | | 0.55 | 0.59 | 0.79 | 63 |

Degumming with a blend PLC solution of SEQ ID 5 + SEQ ID 29 results in an improved performance in terms of diglyceride formation when compared to single component PLC solution SEQ ID 5.

**Example 9: Mature polypeptide of SEQ ID NO: 4 and mature polypeptide of SEQ ID NO: 29.**

The mature polypeptide of SEQ ID NO: 29 applied in degumming assay in combination with the mature polypeptide of SEQ ID NO: 4 applying crude soybean oil after oil pre-treatment with 650 ppm citric acid + 1.5 eqv NaOH. Total water content is 3% w/w. The enzyme incubation is done at 60°C or 70°C for 22 hours.

| Enzyme | Enz dosing (mg EP/kg oil) | Incubation temperature °C | DG increase after enzyme reaction (hours) | | |
|---|---|---|---|---|---|
| | | | 2 | 5 | 22 |
| Seq ID 29 | 10 | 60 | 0.24 | 0.25 | 0.28 |
| Seq ID X + 29 | 30 + 10 | 60 | 0.44 | 0.61 | 0.99 |
| Seq ID 29 | 10 | 70 | 0.12 | 0.19 | 0.19 |
| Seq ID X + 29 | 30 + 10 | 70 | 0.42 | 0.54 | 0.64 |

Degumming with a blend PLC solution of SEQ ID NO: 4 + SEQ ID NO: 29 results in an improved performance in terms of diglyceride formation when compared to single component PLC solution SEQ ID NO: 29.

**Example 10: Mature polypeptide of SEQ ID NO: 2 and mature polypeptide of SEQ ID NO: 29.**

The mature polypeptide of SEQ ID NO: 29 applied in degumming assay in combination with the mature polypeptide of SEQ ID NO: 2 applying crude soybean oil after oil pre-treatment with 650 ppm citric acid + 1.5 eqv NaOH. Total water content is 3% w/w. The enzyme incubation is done at 60°C for 5 hours.

| Enzyme | Enz dosing (mg EP/kg oil) | DG increase after enzyme reaction (hours) | | |
|---|---|---|---|---|
| | | 2 | 3 | 5 |
| Seq ID 29 | 10 | 0.17 | 0.17 | 0.19 |
| Seq ID 2 | 30 | 0.26 | 0.31 | 0.41 |
| Seq ID 2 + 29 | 10 + 30 | 0.45 | 0.48 | 0.63 |
| Blank | 0 | 0.02 | 0.03 | 0.00 |

Degumming with a blend PLC solution of SEQ ID NO: 2 + SEQ ID 29 results in an improved performance in terms of diglyceride formation when compared to single component PLC solutions SEQ ID NO: 29 or SEQ ID NO: 2.

The invention described and claimed herein is not to be limited in scope by the specific aspects herein disclosed, since these aspects are intended as illustrations of several aspects of the invention. Any equivalent aspects are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

## Claims

**1.** A composition comprising a plurality of polypeptides having phospholipase C activity, wherein

- at least one of said polypeptides having phospholipase C activity is a polypeptide selected from the group consisting of:

(a) a polypeptide having at least 60% sequence identity to the mature polypeptide of any one of SEQ ID NO: 2, SEQ ID NO: 4, and SEQ ID NO: 7;
(b) a polypeptide encoded by a polynucleotide having at least 60% sequence identity to the mature polypeptide coding sequence of anyone of SEQ ID NO: 1, SEQ ID NO: 3 and SEQ ID NO: 5.; and

- at least one of said polypeptides having phospholipase C activity is a polypeptide selected from the group consisting of:

(a) a polypeptide having at least 60% sequence identity to the mature polypeptide of any one of SEQ ID NO: 9, SEQ ID NO: 12, SEQ ID NO: 15, SEQ ID NO: 18, SEQ ID NO: 21, SEQ ID NO: 24, SEQ ID NO: 26, SEQ ID NO: 29, SEQ ID NO: 31, SEQ ID NO: 34, SEQ ID NO: 36, SEQ ID NO: 39, SEQ ID NO: 41, SEQ ID NO: 43 and SEQ ID NO: 45;
(b) a polypeptide encoded by a polynucleotide having at least 60% sequence identity to the mature polypeptide coding sequence of any one of SEQ ID NO: 8, SEQ ID NO: 11, SEQ ID NO: 14, SEQ ID NO: 17, SEQ ID NO: 20, SEQ ID NO: 23, SEQ ID NO: 25, SEQ ID NO: 28, SEQ ID NO: 30, SEQ ID NO: 33, SEQ ID NO: 35, SEQ ID NO: 38, SEQ ID NO: 40, SEQ ID NO: 42 and SEQ ID NO: 44.

**2.** The composition according to claim 1, comprising a polypeptide, which has phospholipase C activity and is selected from the group consisting of:

(a) a polypeptide having at least 60% sequence identity to the mature polypeptide of SEQ ID NO: 41;
(b) a polypeptide encoded by a polynucleotide having at least 60% sequence identity to the mature polypeptide coding sequence of SEQ ID NO: 40.

**3.** Use of a composition as defined in any of the preceding claims for

i) degumming of an aqueous carbohydrate solution or slurry, in degumming of oil, e.g. vegetable oil, or an edible vegetable oil, or in a process comprising hydrolysis of phospholipids in the gum fraction from water degumming to release entrapped triglyceride oil,
ii) a process comprising hydrolysis of phospholipids to obtain improved phospholipid emulsifiers, in particular wherein said phospholipid is lecithin,
iii) a process for improving the filterability of an aqueous solution or slurry of carbohydrate origin which contains phospholipid,
iv) a process for the extraction of oil,
v) a process for the production of an animal feed product,
vi) a process for the production of a biofuel, e.g. a biodiesel,
vii) a process for the production of a detergent product, and/or
viii) a process for making a baked product, comprising adding the phospholipase to a dough, and baking the dough to make the baked product.

**4.** A process for hydrolysing a phospholipid or lysophospholipid, comprising contacting said phospholipid or lysophospholipid with a plurality of polypeptides having phospholipase C activity, wherein

- at least one of said polypeptides having phospholipase C activity is selected from the group consisting of:

(a) a polypeptide having at least 60% sequence identity to the mature polypeptide of any one of SEQ ID NO: 2, SEQ ID NO: 4, and SEQ ID NO: 7;
(b) a polypeptide encoded by a polynucleotide having at least 60% sequence identity to the mature polypeptide coding sequence of anyone of SEQ ID NO: 1, SEQ ID NO: 3 and SEQ ID NO: 5.; and

- at least one of said polypeptides having phospholipase C activity is is selected from the group consisting of:

(a) a polypeptide having at least 60% sequence identity to the mature polypeptide of SEQ ID NO: 41;
(b) a polypeptide encoded by a polynucleotide having at least 60% sequence identity to the mature polypeptide coding sequence of SEQ ID NO: 40.

**5.** The process according to claim 4, comprising contacting said phospholipid or lysophospholipid with a polypeptide, which has phospholipase C activity, and is selected from the group consisting of:

(a) a polypeptide having at least 60% sequence identity to the mature polypeptide of any one of SEQ ID NO: 9, SEQ ID NO: 12, SEQ ID NO: 15, SEQ ID NO: 18, SEQ ID NO: 21, SEQ ID NO: 24, SEQ ID NO: 26, SEQ ID NO: 29, SEQ ID NO: 31, SEQ ID NO: 34, SEQ ID NO: 36, SEQ ID NO: 39, SEQ ID NO: 41, and SEQ ID NO: 43 and SEQ ID NO: 45;
(b) a polypeptide encoded by a polynucleotide having at least 60% sequence identity to the mature polypeptide coding sequence of any one of SEQ ID NO: 8, SEQ ID NO: 11, SEQ ID NO: 14, SEQ ID NO: 17, SEQ ID NO: 20, SEQ ID NO: 23, SEQ ID NO: 25, SEQ ID NO: 28, SEQ ID NO: 30, SEQ ID NO: 33, SEQ ID NO: 35, SEQ ID NO: 38, SEQ ID NO: 40, SEQ ID NO: 42 and SEQ ID NO: 44.

## Patentansprüche

1. Zusammensetzung, umfassend mehrere Polypeptide mit Phospholipase-C-Aktivität, wobei

    - es sich bei wenigstens einem der Polypeptide mit Phospholipase-C-Aktivität um ein Polypeptid handelt, das ausgewählt ist aus der Gruppe bestehend aus:

        (a) einem Polypeptid mit wenigstens 60% Sequenzidentität mit dem reifen Polypeptid unter einer von SEQ ID NO: 2, SEQ ID NO: 4 und SEQ ID NO: 7;
        (b) einem Polypeptid, das durch ein Polynukleotid mit wenigstens 60% Sequenzidentität mit der Codiersequenz des reifen Polypeptids unter einer von SEQ ID NO: 1, SEQ ID NO: 3 und SEQ ID NO: 5 codiert ist; und

    - es sich bei wenigstens einem der Polypeptide mit Phospholipase-C-Aktivität um ein Polypeptid handelt, das ausgewählt ist aus der Gruppe bestehend aus:

        (a) einem Polypeptid mit wenigstens 60% Sequenzidentität mit dem reifen Polypeptid unter einer von SEQ ID NO: 9, SEQ ID NO: 12, SEQ ID NO: 15, SEQ ID NO: 18, SEQ ID NO: 21, SEQ ID NO: 24, SEQ ID NO: 26, SEQ ID NO: 29, SEQ ID NO: 31, SEQ ID NO: 34, SEQ ID NO: 36, SEQ ID NO: 39, SEQ ID NO: 41, SEQ ID NO: 43 und SEQ ID NO: 45;
        (b) einem Polypeptid, das durch ein Polynukleotid mit wenigstens 60% Sequenzidentität mit der Codiersequenz des reifen Polypeptids unter einer von SEQ ID NO: 8, SEQ ID NO: 11, SEQ ID NO: 14, SEQ ID NO: 17, SEQ ID NO: 20, SEQ ID NO: 23 SEQ ID NO: 25, SEQ ID NO: 28, SEQ ID NO: 30, SEQ ID NO: 33, SEQ ID NO: 35, SEQ ID NO: 38, SEQ ID NO: 40, SEQ ID NO: 42 und SEQ ID NO: 44 codiert ist.

2. Zusammensetzung nach Anspruch 1, umfassend ein Polypeptid, das Phospholipase-C-Aktivität aufweist und ausgewählt ist aus der Gruppe bestehend aus:

    (a) einem Polypeptid mit wenigstens 60% Sequenzidentität mit dem reifen Polypeptid unter SEQ ID NO: 41;
    (b) einem Polypeptid, das durch ein Polynukleotid mit wenigstens 60% Sequenzidentität mit der Codiersequenz des reifen Polypeptids unter SEQ ID NO: 40 codiert ist.

3. Verwendung einer Zusammensetzung gemäß einem der vorhergehenden Ansprüche für

    i) Entschleimen einer wässrigen Kohlenhydratlösung oder Aufschlämmung, bei der Entschleimung von Öl, z. B. Pflanzenöl oder einem essbaren Pflanzenöl, oder bei einem Verfahren, das Hydrolyse von Phospholipiden in der Schleimstofffraktion vom Wasserentschleimen zur Freisetzung von eingeschlossenem Triglyceridöl umfasst,
    ii) ein Verfahren, das Hydrolyse von Phospholipiden zur Gewinnung verbesserter Phospholipidemulgatoren umfasst, insbesondere wobei es sich bei dem Phospholipid um Lecithin handelt,
    iii) ein Verfahren zur Verbesserung der Filtrierbarkeit einer wässrigen Lösung oder Aufschlämmung, die von Kohlenhydraten stammt und Phospholipid enthält,
    iv) ein Verfahren zur Extraktion von Öl,
    v) ein Verfahren zur Herstellung eines Tierfutterprodukts,
    vi) ein Verfahren zur Herstellung eines Biokraftstoffs, z. B. eines Biodiesels,
    vii) ein Verfahren zur Herstellung eines Detergenzienprodukts und/oder
    viii) ein Verfahren zum Herstellen eines Backprodukts, umfassend Zugeben der Phospholipase zu einem Teig und Backen des Teigs, um das Backprodukt herzustellen.

4. Verfahren zur Hydrolyse eines Phospholipids oder Lysophospholipids, umfassend Inkontaktbringen des Phospholipids oder Lysophospholipids mit mehreren Polypeptiden mit Phospholipase-C-Aktivität, wobei

    - wenigstens eines der Polypeptide mit Phospholipase-C-Aktivität ausgewählt ist aus der Gruppe bestehend aus:

        (a) einem Polypeptid mit wenigstens 60% Sequenzidentität mit dem reifen Polypeptid unter einer von SEQ ID NO: 2, SEQ ID NO: 4 und SEQ ID NO: 7;
        (b) einem Polypeptid, das durch ein Polynukleotid mit wenigstens 60% Sequenzidentität mit der Codiersequenz des reifen Polypeptids unter einer von SEQ ID NO: 1, SEQ ID NO: 3 und SEQ ID NO: 5 codiert ist; und

- wenigstens eines der Polypeptide mit Phospholipase-C-Aktivität ausgewählt ist aus der Gruppe bestehend aus:

(a) einem Polypeptid mit wenigstens 60% Sequenzidentität mit dem reifen Polypeptid unter SEQ ID NO: 41;
(b) einem Polypeptid, das durch ein Polynukleotid mit wenigstens 60% Sequenzidentität mit der Codiersequenz des reifen Polypeptids unter SEQ ID NO: 40 codiert ist.

**5.** Verfahren nach Anspruch 4, umfassend Inkontaktbringen des Phospholipids oder Lysophospholipids mit einem Polypeptid, das Phospholipase-C-Aktivität aufweist und ausgewählt ist aus der Gruppe bestehend aus:

(a) einem Polypeptid mit wenigstens 60% Sequenzidentität mit dem reifen Polypeptid unter einer von SEQ ID NO: 9, SEQ ID NO: 12, SEQ ID NO: 15, SEQ ID NO: 18, SEQ ID NO: 21, SEQ ID NO: 24, SEQ ID NO: 26, SEQ ID NO: 29, SEQ ID NO: 31, SEQ ID NO: 34, SEQ ID NO: 36, SEQ ID NO: 39, SEQ ID NO: 41, SEQ ID NO: 43 und SEQ ID NO: 45;
(b) einem Polypeptid, das durch ein Polynukleotid mit wenigstens 60% Sequenzidentität mit der Codiersequenz des reifen Polypeptids unter einer von SEQ ID NO: 8, SEQ ID NO: 11, SEQ ID NO: 14, SEQ ID NO: 17, SEQ ID NO: 20, SEQ ID NO: 23 SEQ ID NO: 25, SEQ ID NO: 28, SEQ ID NO: 30, SEQ ID NO: 33, SEQ ID NO: 35, SEQ ID NO: 38, SEQ ID NO: 40, SEQ ID NO: 42 und SEQ ID NO: 44 codiert ist.

## Revendications

**1.** Composition comprenant une pluralité de polypeptides ayant une activité de phospholipase C, dans laquelle

- au moins l'un desdits polypeptides ayant une activité de phospholipase C est un polypeptide choisi dans le groupe constitué par :

(a) un polypeptide ayant une identité de séquence d'au moins 60% avec le polypeptide mature de l'une quelconque des SEQ ID n° : 2, SEQ ID n° : 4 et SEQ ID n° : 7 ;
(b) un polypeptide codé par un polynucléotide ayant une identité de séquence d'au moins 60% avec la séquence codant pour le polypeptide mature de l'une quelconque des SEQ ID n° : 1, SEQ ID n° : 3 et SEQ ID n° : 5 ; et

- au moins l'un desdits polypeptides ayant une activité de phospholipase C est un polypeptide choisi dans le groupe constitué par :

(a) un polypeptide ayant une identité de séquence d'au moins 60% avec le polypeptide mature de l'une quelconque des SEQ ID n° : 9, SEQ ID n° : 12, SEQ ID n° : 15, SEQ ID n° : 18, SEQ ID n° : 21, SEQ ID n° : 24, SEQ ID n° : 26, SEQ ID n° : 29, SEQ ID n° : 31, SEQ ID n° : 34, SEQ ID n° : 36, SEQ ID n° : 39, SEQ ID n° : 41, SEQ ID n° : 43 et SEQ ID n° : 45 ;
(b) un polypeptide codé par un polynucléotide ayant une identité de séquence d'au moins 60% avec la séquence codant pour le polypeptide mature de l'une quelconque des SEQ ID n° : 8, SEQ ID n° : 11, SEQ ID n° : 14, SEQ ID n° : 17, SEQ ID n° : 20, SEQ ID n° : 23, SEQ ID n° : 25, SEQ ID n° : 28, SEQ ID n° : 30, SEQ ID n° : 33, SEQ ID n° : 35, SEQ ID n° : 38, SEQ ID n° : 40, SEQ ID n° : 42 et SEQ ID n° : 44.

**2.** Composition, selon la revendication 1, comprenant un polypeptide, qui a une activité de phospholipase C et qui est choisi dans le groupe constitué par :

(a) un polypeptide ayant une identité de séquence d'au moins 60% avec le polypeptide mature de SEQ ID n° : 41 ;
(b) un polypeptide codé par un polynucléotide ayant une identité de séquence d'au moins 60% avec la séquence codant pour le polypeptide mature de SEQ ID n° : 40.

**3.** Utilisation d'une composition, telle que définie dans l'une quelconque des revendications précédentes, pour

i) démucilaginer une solution aqueuse ou une boue de glucides, dans la démucilagination d'une huile, à titre d'exemple une huile végétale ou une huile végétale comestible, ou bien dans un processus comprenant une hydrolyse de phospholipides dans la fraction de gomme provenant d'une démucilagination à l'eau pour libérer une huile de triglycérides piégée,
ii) un processus comprenant une hydrolyse de phospholipides pour obtenir des émulsifiants phospholipidiques améliorés, en particulier dans lequel ledit phospholipide est la lécithine,
iii) un processus destiné à améliorer la filtrabilité d'une solution aqueuse ou d'une boue d'origine glucidique qui contient un phospholipide,
iv) un processus destiné à l'extraction d'une huile,
v) un processus destiné à la production d'un produit alimentaire pour animaux,
vi) un processus destiné à la production d'un bio-carburant, à titre d'exemple un bio-gasoil,
vii) un processus destiné à la production d'un produit détergent et/ou
viii)un processus destiné à la fabrication d'un produit boulanger, comprenant un ajout de la phospholipase à une pâte et une cuisson de la pâte pour fabriquer le produit boulanger.

**4.** Processus destiné à l'hydrolyse d'un phospholipide ou d'un lysophospholipide, comprenant une mise en contact dudit phospholipide ou lysophospholipide avec une pluralité de polypeptides ayant une activité de phospholipase C, dans lequel

- au moins l'un desdits polypeptides ayant une activité de phospholipase C est choisi dans le groupe constitué par :

  (a) un polypeptide ayant une identité de séquence d'au moins 60% avec le polypeptide mature de l'une quelconque des SEQ ID n° : 2, SEQ ID n° : 4 et SEQ ID n° : 7 ;
  (b) un polypeptide codé par un polynucléotide ayant une identité de séquence d'au moins 60% avec la séquence codant pour le polypeptide mature de l'une quelconque des SEQ ID n° : 1, SEQ ID n° : 3 et SEQ ID n° : 5 ; et

- au moins l'un desdits polypeptides ayant une activité de phospholipase C est choisi dans le groupe constitué par :

  (a) un polypeptide ayant une identité de séquence d'au moins 60% avec le polypeptide mature de SEQ ID n° : 41 ;
  (b) un polypeptide codé par un polynucléotide ayant une identité de séquence d'au moins 60% avec la séquence codant pour le polypeptide mature de SEQ ID n° : 40.

**5.** Processus, selon la revendication 4, comprenant une mise en contact dudit phospholipide ou lysophospholipide avec un polypeptide, qui a une activité de phospholipase C et qui est choisi dans le groupe constitué par :

(a) un polypeptide ayant une identité de séquence d'au moins 60% avec le polypeptide mature de l'une quelconque des SEQ ID n° : 9, SEQ ID n° : 12, SEQ ID n° : 15, SEQ ID n° : 18, SEQ ID n° : 21, SEQ ID n° : 24, SEQ ID n° : 26, SEQ ID n° : 29, SEQ ID n° : 31, SEQ ID n° : 34, SEQ ID n° : 36, SEQ ID n° : 39, SEQ ID n° : 41 et SEQ ID n° : 43 et SEQ ID n° : 45 ;
(b) un polypeptide codé par un polynucléotide ayant une identité de séquence d'au moins 60% avec la séquence codant pour le polypeptide mature de l'une quelconque des SEQ ID n° : 8, SEQ ID n° : 11, SEQ ID n° : 14, SEQ ID n° : 17, SEQ ID n° : 20, SEQ ID n° : 23, SEQ ID n° : 25, SEQ ID n° : 28, SEQ ID n° : 30, SEQ ID n° : 33, SEQ ID n° : 35, SEQ ID n° : 38, SEQ ID n° : 40, SEQ ID n° : 42 et SEQ ID n° : 44.

**Figure 1**

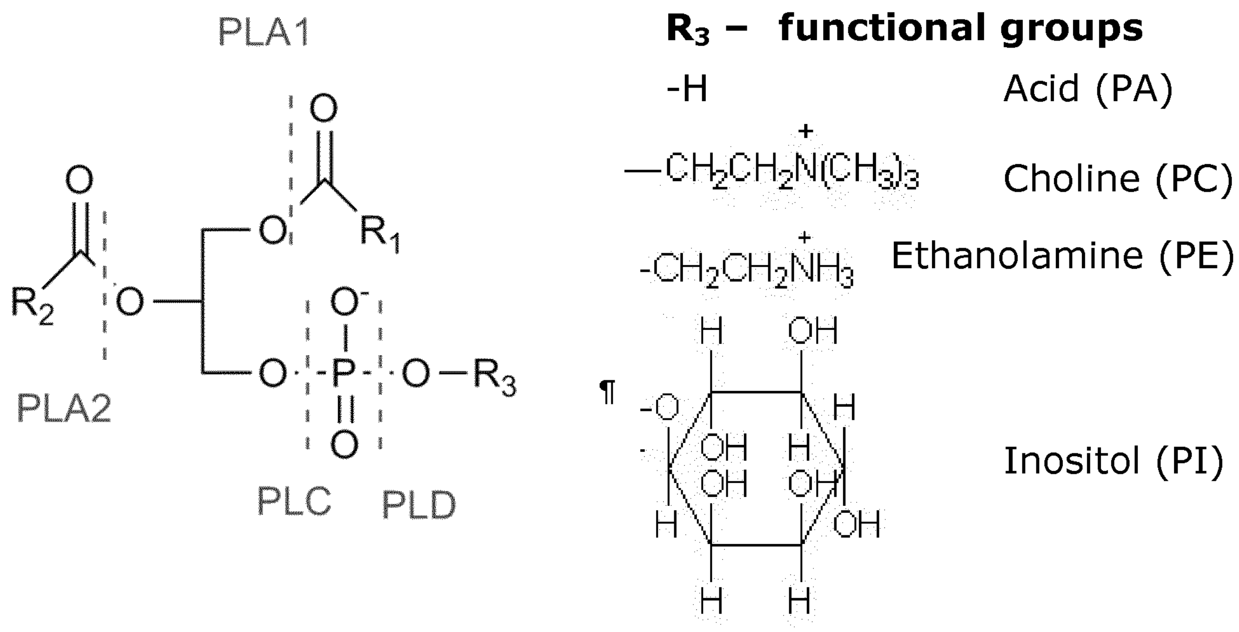


**Figure 2**

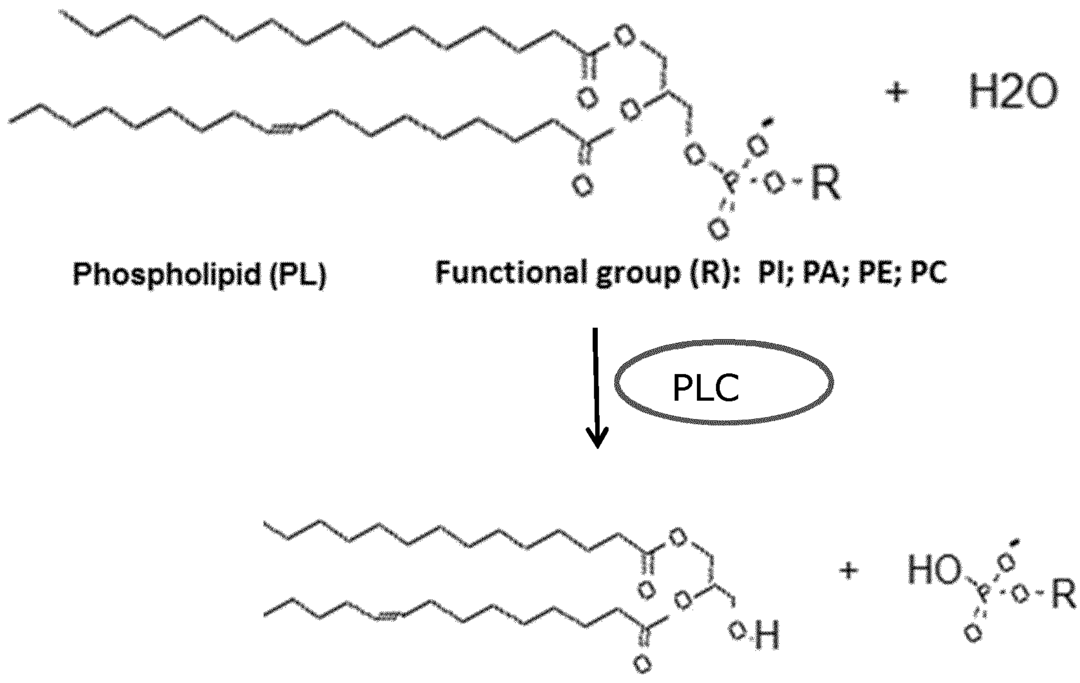


Phospholipid (PL)

Functional group (R): PI; PA; PE; PC

Diglyceride (DG)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003089620 A
- WO 2008094847 A
- WO 07059927 A
- WO 2012062817 A
- WO 2011046815 A
- WO 199826057 A
- WO 9517413 A
- WO 9522625 A
- US 5223409 A
- WO 9206204 A
- WO 20110146812 A
- WO 2007103005 A
- US 20080182322 A
- US 6355693 B
- US 6162623 A
- US 6103505 A
- US 6001640 A
- US 5558781 A
- US 5264367 A
- EP 513709 A
- JP 2794574 B
- JP 6087751 B
- EP 219269 A
- EP 743017 A
- US 8012724 B


### Non-patent literature cited in the description


- **DIJKSTRA.** *Eur. J. Lipid Sci. Technol.,* 2010, vol. 112, 1178
- **CLAUSEN.** *Eur J Lipid Sci Techno,* 2001, vol. 103, 333-340
- **DIJKSTRA.** *101st AOCS Annual Meeting,* 10 May 2010
- **KAUFFMANN.** Conversion of Bacillus thermocatenulatus lipase into an efficient phospholipase with increased activity towards long-chain fatty acyl substrates by directed evolution and rational design. *Protein Engineering,* 2001, vol. 14, 919-928
- **IBRAHIM.** Evidence implicating phospholipase as a virulence factor of Candida albicans. *Infect. Immun.,* 1995, vol. 63, 1993-1998
- **GOODE.** Evidence for cell surface internal phospholipase activity in ascidian eggs. *Develop. Growth Differ.,* 1997, vol. 39, 655-660
- **DIAZ.** Direct fluorescence-based lipase activity assay. *BioTechniques,* 1999, vol. 27, 696-700
- **REYNOLDS.** *Methods in Enzymol.,* 1991, vol. 197, 3-13
- **TAGUCHI.** Phospholipase from Clostridium novyi type A.I. *Biochim. Biophys. Acta,* 1975, vol. 409, 75-85
- **TAGUCHI.** *Biochim. Biophys. Acta,* 1975, vol. 409, 75-85
- **KORBSRISATE.** *J. Clin. Microbiol.,* 1999, vol. 37, 3742-3745
- **BERKA.** *Infect. Immun.,* 1981, vol. 34, 1071-1074
- **TITBALL.** *Microbiol. Rev.,* 1993, vol. 57, 347-366
- **NIELSEN et al.** *Protein Engineering,* 1997, vol. 10, 1-6
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **RICE et al.** *EMBOSS: The European Molecular Biology Open Software Suite,* 2000
- **H. NEURATH ; R.L. HILL.** The Proteins. Academic Press, 1979
- **CUNNINGHAM ; WELLS.** *Science,* 1989, vol. 244, 1081-1085
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS Lett.,* 1992, vol. 309, 59-64
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896

- **COOPER et al.** *EMBO J.,* 1993, vol. 12, 2575-2583
- **DAWSON et al.** *Science,* 1994, vol. 266, 776-779
- **MARTIN et al.** *J. Ind. Microbiol. Biotechnol.,* 2003, vol. 3, 568-576
- **SVETINA et al.** *J. Biotechnol.,* 2000, vol. 76, 245-251
- **RASMUSSEN-WILSON et al.** *Appl. Environ. Microbiol.,* 1997, vol. 63, 3488-3493
- **WARD et al.** *Biotechnology,* 1995, vol. 13, 498-503
- **CONTRERAS et al.** *Biotechnology,* 1991, vol. 9, 378-381
- **EATON et al.** *Biochemistry,* 1986, vol. 25, 505-512
- **COLLINS-RACIE et al.** *Biotechnology,* 1995, vol. 13, 982-987
- **CARTER et al.** *Proteins: Structure, Function, and Genetics,* 1989, vol. 6, 240-248
- **STEVENS.** *Drug Discovery World,* 2003, vol. 4, 35-48
- Ullmann's Encyclopedia of Industrial Chemistry. VCH Weinheim, 1996